# NEW EUROPEAN PATENT SPECIFICATION

(11) **EP 0 399 731 B2**
(45) Date of publication and mention of the opposition decision: **11.07.2001**
(45) Mention of the grant of the patent: 08.12.1993
(21) Application number: 90305389.0
(22) Date of filing: 18.05.1990
(51) Int. Cl.: C07D 471/04, A61K 31/33, C07D 487/04, C07D 473/00

(54) **Azaindenes**
Azaindene
Azaindènes

(30) Priority: 23.05.1989 GB 8911855; 15.03.1990 GB 9005843
(43) Date of publication of application: 28.11.1990
(73) Proprietor: AstraZeneca AB, 151 85 Södertälje (SE)
(72) Inventor: Roberts, David Anthony, Congleton, Cheshire, CW12 2HH (GB); Russell, Simon Thomas, Macclesfield, Cheshire, SK10 3BT (GB); Ratcliffe, Arnold Harry, Poynton, Cheshire SK10 1NN (GB); Gibson, Keith Hopkinson, Macclesfield, Cheshire SK10 4AD (GB); Wood, Robin, Stockport, Cheshire SK2 5HR (GB)
(74) Representative: Tait, Brian Steele

(56) References cited:
- EP-A- 0 132 606
- EP-A- 0 186 190
- EP-A- 0 253 310
- EP-A- 0 400 974
- EP-A- 0 420 237
- EP-A- 0 426 021
- WO-A-89/08653
- Pharmazie 43, 1988, pp. 315-317
- Drug Development Research 8, 1988, pp. 95-102
- Yakugaku Zasshi 94 (6), 1974, pp. 708-716

## Description

This invention concerns novel azaindenes and, more particularly, novel imidazopyridine and imidazodiazine derivatives which possess pharmacologically useful properties in antagonising at least in part one or more of the actions of the substances known as angiotensins, and in particular of that known as angiotensin II (hereafter referred to as All). The invention also concerns pharmaceutical compositions of the novel compounds for use in treating diseases or medical conditions such as hypertension, congestive heart failure and/or hyperaldosteronism in warm-blooded animals (including man), as well as in other diseases or medical conditions in which the renin-angiotensin-aldosterone system plays a significant causative role. The invention also includes processes for the manufacture of the novel compounds which can be used in treating one of the afore-mentioned diseases or medical conditions and for the production of novel pharmaceuticals for use in such medical treatments.

The angiotensins are key mediators of the renin-angiotensin-aldosterone system, which is involved in the control of homeostasis and fluid/electrolyte balance in many warm-blooded animals, including man. The angiotensin known as All is produced by the action of angiotensin converting enzyme (ACE) from angiotensin I, itself produced by the action of the enzyme renin from the blood plasma protein angiotensinogen. All is a potent spasmogen especially in the vasculature and is known to increase vascular resistance and blood pressure. In addition, the angiotensins are known to stimulate the release of aldosterone and hence result in vascular congestion and hypertension via sodium and fluid retention mechanisms. Hitherto there have been a number of different approaches to pharmacological intervention in the renin-angiotensin-aldosterone system for therapeutic control of blood pressure and/or fluid/electrolyte balance, including, for example, inhibiting the actions of renin or ACE. Bowever, there remains a continuing need for an alternative approach because of the side-effects and/or idiosyncratic reactions associated with any particular therapeutic approach.

Certain substituted imidazoles described in European Patent Application, publication no. 253310 A2 are known to inhibit the action of angiotensin II.

We have now discovered that the compounds of the invention (set out below) surprisingly antagonise one or more of the actions of the substances known as angiotensins (and in particular of All) and thus minimise the physiological effects associated with their presence in warm-blooded animals (including man) and this is the basis of the invention.

According to the invention there is provided an azaindene derivative of the formula I (set out hereinafter, together with the other chemical formulae identified by Roman numerals) wherein A, together with the adjacent vinylene group of the imidazole moiety completes an azene ring selected from pyridine, pyrimidine, pyridazine or pyrazine ring; R¹ is (1-8C)alkyl, (3-8C)cycloalkyl, (3-8C)cycloalkyl-(1-4C)alkyl, phenyl or phenyl(1-4C)alkyl; R² is hydrogen, (1-4C)alkyl, (1-4C)alkoxy, halogeno, trifluoromethyl, cyano or nitro; R³ and R⁴ are optional substituents on the said azene ring, independently selected from hydrogen, (1-4C)alkyl, (3-8C)cycloalkyl, (1-4C)alkoxy, halogeno, trifluoromethyl, cyano, hydroxy, hydroxymethyl, formyl, and nitro; or when A together with the imidazole moiety to which it is attached is an imidazo[4,5-b]pyridine or imidazo[4,5-c]pyridine group, R³ and R⁴ when they are on adjacent carbon atoms of A form a trimethylene or tetramethylene group, or together with the adjacent vinylene group of A complete a benzene ring, the latter optionally bearing a halogeno, (1-4C)alkyl or (1-4C)alkoxy substituent; or when A together with the imidazole moiety to which it is attached is other than a 1H-imidazo[4,5-c]pyridine ring, one of R³ or R⁴ is a carboxy or (1-6C)alkoxycarbonyl group and the other is as defined above; X is phenylene optionally bearing a substituent selected from (1-4C)alkyl, (1-4C)alkoxy and halogeno, or X is a direct bond between the adjacent phenyl and methylene moieties; and Z is 1H-tetrazol-5-yl or a group of the formula **-CO.OR**^{**5**} or **-CO.NH.SO**_{**2**}**.R**^{**6**} in which R⁵ is hydrogen or a non-toxic, biodegradable residue of a physiologically acceptable alcohol or phenol, and R⁶ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or a physiologically acceptable salt thereof except when R5 is other than hydrogen and R³ or R⁴ is other than carboxy.

It will appreciated that ten different groups of imidazopyridines (formulae la - Id) and imidazodiazines (formulae le - Ij) are encompassed within the above formula I definition and are included as separate parts of the invention. Of these groups, the compounds of formula la are of particular interest. Also, depending on the nature of the substituents, certain of the formula I compounds may possess one or more chiral centres and may be isolated in one or more racemic or optically active forms. It is to be understood that this invention concerns any form of such a compound of formula I which possesses the afore-mentioned useful pharmacological properties, it being well known how to make optically active forms, for example by synthesis from suitable chiral intermediates, and how to determine their pharmacological properties, for example by use of the standard tests described hereinafter.

It is to be understood that generic terms such as "alkyl" include both straight and branched chain variants when the carbon numbers permit. Bowever, when a particular radical such as "propyl" is given, it is specific to the straight chain variant, branched chain variants such as "isopropyl", being specifically named where intended. The same convention applies to other radicals.

A particular value for R¹ when it is alkyl is, for example, methyl, ethyl, propyl, butyl, isobutyl, sec-butyl, pentyl or hexyl; when it is cycloalkyl is, for example, cyclopropyl, cyclopentyl or cyclohexyl; when it is cycloalkylalkyl is, for example, cyclopropylmethyl, cyclopentylmethyl, cyclohexylmethyl or 2-cyclopentyl-ethyl; and when it is phenylalkyl is, for example, benzyl, 1-phenylethyl or 2-phenylethyl.

Particular values for R², R³, R⁴ or an optional substituent which may be present when X is phenylene or when R³ and R⁴ together with the adjacent vinylene group of A complete a benzene ring, include, by way of example:-
when it is alkyl, methyl and ethyl;
when it is alkoxy, methoxy and ethoxy; and
when it is halogeno, fluoro, chloro, bromo and iodo.

A particular value for R³ or R⁴ when it is cycloalkyl is, for example, cyclopropyl, cyclopentyl or cyclohexyl.

A particular value for R³ or R⁴ when it is (1-6C)alkoxycarbonyl is, for example, methoxycarbonyl, ethoxycarbonyl or propoxycarbonyl.

A particular value for R⁵ when it is a non-toxic, biodegradable residue of a physiologically acceptable alcohol or phenol is, for example, a residue derived from a (1-6C)alkanol such as methanol or ethanol, or phenol, glycerol or the like.

A particular value for R⁶ when it is alkyl is, for example, methyl, ethyl, propyl, isopropyl, butyl or pentyl; and when it is cycloalkyl is, for example, cyclobutyl, cyclopentyl or cyclohexyl.

Particular values for optional substituents which may be present on phenyl moieties include, by way of example, for halogeno, fluoro, chloro and bromo; for alkyl, methyl or ethyl; and for alkoxy, methoxy and ethoxy.

Specific values for X which are of particular interest include, for example, when it is a direct bond or p-phenylene.

A preferred value for R⁵ is, for example, hydrogen, for R¹ is, for example, butyl, and for Z is, for example, 1H-tetrazol-5-yl.

Particular groups of compounds of the invention comprise:-
(1) imidazopyridines of the formula la, Ib, Ic or Id;
(2) imidazopyrazines of the formula le;
(3) imidazopyrimidines (that is purines) of the formula If or Ig; and
(4) imidazopyridazines of the formula Ih, Ii or Ij; in any of which R¹, R², R³, R⁴, X and Z have any of the meanings defined above; together with the pharmaceutically acceptable salts thereof.

A particularly preferred group of compounds of the formula I comprises:-
(a) imidazopyridines of the formula la, Ib or Id;
(b) imidazopyrazines of the formula le;
(c) imidazopyrimidines of the formula If or Ig; and
(d) imidazopyridazines of the formula Ij;
wherein in any of the above groups R¹ is (1-6C)alkyl; R² is hydrogen or halogeno; R³ and R⁴ are selected from hydrogen, halogeno, (1-6C)alkyl, (1-6C)alkoxy, hydroxymethyl and hydroxy; X is a direct bond or p-phenylene; and Z is selected from 1H-tetrazol-5-yl, carboxy and a group of the formula -CO.NH.SO₂.R⁶ in which R⁶ has any of the meanings defined above, and wherein Z is attached at the 2- or 4-position relative to X.

Compounds of the invention which are of particular interest include, for example, the specific embodiments set out hereinafter in the accompanying Examples, and in particular those in Examples 1, 3, 4, 5, 11, 12, 15 and 17.

Particularly suitable salts of compounds of formula I include, for example, salts with bases affording physiologically acceptable cations, for example, alkali metal (such as sodium and potassium), alkaline earth metal (such as magnesium and calcium), aluminium and ammonium salts, as well as salts with suitable organic bases, such as with ethanolamine, methylamine, diethylamine or triethylamine. In addition, for those compounds in which the ring containing A is sufficiently basic (for example, where it is pyrazine or pyridine), particularly suitable salts of formula I compounds also include, salts with acids affording physiologically acceptable anions, for example salts with hydrohalides (such as hydrochloride or hydrobromide salts), sulphuric and phosphoric acid.

The compounds of formula I may be obtained by standard procedures of organic chemistry well known in the art for the production of structurally analogous heterocyclic compounds. Such procedures are provided as a further feature of the invention and include, by way of example, the following illustrative procedures in which the generic radicals have any of the values given above, unless stated otherwise.
a) For those compounds in which Z is carboxy (that is in which Z is a group of the formula **-CO.OR**^{**5**} in which R⁵ is hydrogen), a carboxylic acid derivative of the formula II, in which Q is a protected carboxy group selected from (1-6C)alkoxycarbonyl (especially methoxy-, ethoxy-, propoxy- or t-butoxy-carbonyl), phenoxycarbonyl, benzyloxycarbonyl and carbamoyl, is converted to carboxy.
   The conversion may be carried out, for example by hydrolysis, conveniently in the presence of a suitable base such as an alkali metal hydroxide, for example, lithium, sodium or potassium hydroxide. The hydrolysis is generally carried out in the presence of a suitable aqueous solvent or diluent, for example in an aqueous (1-4C)alkanol, such as aqueous methanol or ethanol. However, it may also be performed in a mixture of an aqueous and non-aqueous solvent such as water and toluene using a conventional quaternary ammonium phase tranfer catalyst. The hydrolysis is generally performed at a temperature in the range, for example, 0 - 120°C, depending on the reactivity of the group Q. In general, when Q is carbamoyl, temperatures in the range, for example, 40 - 120°C are required to effect the hydrolysis.
   Alternatively, when Q is benzyloxycarbonyl, the conversion may also be performed by hydrogenolysis, for example using hydrogen at 1-3 bar in the presence of a suitable catalyst, such as palladium on charcoal or on calcium sulphate, in a suitable solvent or diluent such as a (1-4C)alkanol (typically ethanol or 2-propanol) and at a temperature in the range, for example, 0 - 40°C.
   Further, when Q is t-butoxycarbonyl, the conversion may also be carried out by hydrolysis at a temperature in the range, for example, 0 - 100°C, in the presence of a strong acid catalyst, such as trifluoroacetic acid. The hydrolysis may either be performed in an excess of the acid or in the presence of a suitable diluent such as tetrahydrofuran, t-butyl methyl ether or 1,2-dimethoxyethane.
b) For those compounds of formula I wherein Z is tetrazolyl, a compound of the formula III in which L is a suitable protecting group, such as trityl, benzhydryl, trialkyltin (for example trimethyltin) or triphenyltin, affixed to a nitrogen of the tetrazolyl moiety, is deprotected.
   The reaction conditions used to carry out the deprotection necessarily depend on the nature of the group L. As an illustration, when it is trityl, benzhydryl, trialkyltin or triphenyltin, the decomposition conditions include, for example, acid catalysed hydrolysis in a mineral acid (such as aqueous hydrochloric or hydrobromic acid), conveniently in an aqueous solvent (such as aqueous dioxan or 2-propanol). The compound of formula I may then be conveniently isolated as the corresponding acid addition salt. Alternatively, a trityl or benzhydryl group may be removed by hydrogenolysis, for example as described in (a) above for conversion of benzyloxycarbonyl to carboxy.
   Compounds of the formula III wherein L is trialkyltin or triphenyltin may be obtained, for example, by reaction of a nitrite of the formula IX with a trialkyltin, such as trimethyltin, or triphenyltin respectively. The reaction is conveniently carried out in a suitable solvent or diluent, such as toluene or xylene, and at a temperature in the range 50-150°C. It will be appreciated that the use of suitable work-up conditions, for example acidic work-up conditions, may enable compounds of the formula I wherein Z is tetrazolyl to be obtained directly without prior isolation of the protected tetrazole. The nitriles of formula IX may be obtained, for example, by alkylation of an imidazole derivative of the formula IV with a nitrile of the formula X wherein **Hal.** stands for a suitable leaving group such as chloro, bromo, iodo, methanesulphonyloxy or p-toluenesulphonyloxy, using similar conditions to those used in process (c) described hereinafter. The necessary compounds of formula IX may be made by standard procedures such as that illustrated in Scheme 1 for compounds in which X is phenylene.
c) Reacting an imidazole derivative of the formula IV with a compound of the formula V wherein **Hal.** stands for a suitable leaving group such as chloro, bromo, iodo, methanesulphonyloxy or p-toluenesulphonyloxy.
   The reaction is generally carried out in the presence of a suitable base, for example, an alkali metal carbonate such as potassium carbonate or an organic base such as diisopropylethylamine and in a suitable solvent or diluent, for example in a polar solvent such as dimethylformamide and at a temperature in the range, for example, 10 - 80°C.
   Procedure (c) is particularly suitable for the production of those compounds of the formula I in which Z is a group of the formula **-CO.OR**^{**5**} in which R⁵ is other than hydrogen, for example wherein R⁵ is (1-6C)alkyl, benzyl or phenyl, which compounds are also starting materials of formula II for the reaction described in (a) above. Similarly, using an analogous procedure, but starting with the appropriate compound of the formula VI, the starting materials of the formula III may be obtained for procedure (b). In this context, it will be appreciated that when the ring containing A contains the nitrogen atoms asymmetrically disposed (that is when it is [c]pyridazine, [d]pyrimidine or pyridine), various positional isomeric forms of formula II or III will generally be obtained during alkylation of the imidazole derivative of formula IV with the compound of formula V and VI, respectively. Such positional isomeric forms may be separated by conventional procedures such as fractional crystallisation or chromatography.
   The majority of the imidazole derivatives of formula IV are already known and the remainder can be made by analogy therewith using standard procedures of organic chemistry well known in the art, for example as described in standard works of heterocyclic chemistry such as that edited by Elderfield. The necessary compounds of the formula V (and also of formula VI) may be made by standard procedures such as those which are illustrated in Scheme 1 for compounds in which X is phenylene.
(d) Reacting a diamino derivative of the formula VII with a carboxylic acid of the formula **R**^{**1**}**.CO**_{**2**}**H** or a (1-4C)alkyl orthoester (for example the methyl or ethyl orthoester) thereof.

When the acid **R**^{**1**}**.CO**_{**2**}**H** is used, the reaction is generally performed in the presence of a dehydrating agent. Particularly suitable dehydrating agents include, for example, polyphosphoric acid and lower alkyl esters thereof, for example, the ethyl ester thereof.

The reaction may be performed in the absence of solvent or conveniently in the presence of an excess of the carboxylic acid of formula **R**^{**1**}**.CO**_{**2**}**H** or of an orthoester thereof. The reaction is usually performed at an elevated temperature, for example in the range 40-150°C.

It will be appreciated that an intermediate frequently obtained in the reaction is an alkanoylamino compound of the formula VIII, formed by initial acylation of the primary amino group. This compound of formula VIII may be separately formed, for example, by reaction of the compound of formula VII with the acid chloride, bromide or anhydride of those alkanoic acids of formula **R**^{**1**}**.CO**_{**2**}**H** in which R¹ is other than hydrogen, generally in the presence of a suitable base such as triethylamine or pyridine at a temperature in the range, for example, 0 - 50°C. Those compounds of formula VIII wherein R¹ is hydrogen may be obtained, for example, by heating the diamino compound of formula VII with formic acid or triethylorthoformate at a temperature in the range, for example, 40 - 100°C. The alkanoylamino compound of formula VIII may then be cyclised by heating with a suitable dehydrating agent as described for process (d) above. The diamine derivatives of formula VII may be obtained by standard procedures of heterocyclic chemistry, for example as are described in the standard textbooks such as those edited by Elderfield et alia.

Whereafter, those compounds of the formula I wherein Z is a group of the formula **-CO.NH.SO**_{**2**}**R**^{**6**} or a group of the formula **-CO.OR**^{**5**} in which R⁵ is other than hydrogen, may be obtained, for example, by reacting a carboxylic acid of the formula I in which Z is carboxy (or a reactive derivative of said acid) with a sulphonamide of the formula **NH**_{**2**}**.SO**_{**2**}**R**^{**6**} or a hydroxy compound of the formula **HO.R**^{**5**}**,** or with a salt thereof (for example, an alkali metal thereof). Suitable reactive derivatives include, for example the chloride, bromide, azide, anhydride and mixed anhydride with formic or acetic acid of the carboxylic acid of formula I as defined above. When the free acid form is used, the reaction is generally carried out in the presence of a suitable dehydrating agent such as dicyclohexycarbodiimide or 3-(3-dimethylaminopropyl)-1-ethylcarbodiimide in the presence of a base such as triethylamine or pyridine. When a reactive derivative is used, either the reaction is carried out in the presence of a base such as mentioned above, or the sulphonamide or hydroxy compound is used in the form of a salt, such as its alkali metal salt (in particular the lithium, sodium or potassium salt thereof). The reaction is generally performed in the presence of a suitable diluent or solvent such as dioxan, t-butyl methyl ether or tetrahydrofuran and at a temperature in the range, for example, 0 - 60°C.

Whereafter, when a salt of a compound of formula I is required, it may be obtained, for example, by reaction with the appropriate base or acid affording a physiologically acceptable ion, or by any other conventional salt formation procedure.

Further, when an optically active form of a compound of formula I is required, one of the aforesaid processes may be carried out using an optically active starting material. Alternatively, the racemic form of a compound of formula I may be resolved, for example when Z is an acidic group by reaction with an optically active form of a suitable organic base, for example, ephedrine, N,N,N-trimethyl-(1-phenylethyl)ammonium hydroxide or 1-phenylethylamine, followed by conventional separation of the diastereoisomeric mixture of salts thus obtained, for example by fractional crystallisation from a suitable solvent, for example a (1-4C)alkanol, whereafter the optically active form of said compound of formula I may be liberated by treatment with acid using a conventional procedure, for example using an aqueous mineral acid such as dilute hydrochloric acid. In an analogous manner, when the compound of formula I is sufficiently basic, the resolution may also be performed, for example by reacting the racemic form with the optically active form of a suitable organic acid such as camphorsulphonic acid.

Certain of the intermediates defined herein are novel, for example the compounds of the formula II, III and VII, and are provided as a further feature of the invention.

As stated above, the compounds offormula I will have beneficial pharmacological effects in warm-blooded animals (including man) in diseases and medical conditions where amelioration of the vasoconstrictor and fluid retaining properties of the renin-angiotensin-aldosterone system is desirable, at least in part by antagonism of one or more of the physiological actions of All. The compounds of the invention will thus be useful in the treatment of diseases or medical conditions such as hypertension, congestive heart failure and/or hyperaldosteronism in warm-blooded animals (including man), as well as in other diseases or medical conditions in which the renin-angiotensin-aldosterone system plays a significant causative role.

The antagonism of one or more of the physiological actions of All and, in particular, the antagonism of the interaction of All with the receptors which mediate its effects on a target tissue, may be assessed using one or more of the following, routine laboratory procedures:-

### Test A:

This in vitro procedure involves the incubation of the test compound initially at a concentration of 100 micromolar (or less) in a buffered mixture containing fixed concentrations of radiolabelled All and a cell surface membrane fraction prepared from a suitable angiotensin target tissue. In this test, the source of cell surface membranes is the guinea pig adrenal gland which is well known to respond to All. Interaction of the radiolabelled All with its receptors (assessed as radiolabel bound to the particulate membrane fraction following removal of unbound radiolabel by a rapid filtration procedure such as is standard in such studies) is antagonized by compounds which also bind to the membrane receptor sites and the degree of antagonism (observed in the test as displacement of membrane-bound radioactivity) is determined readily by comparing the receptor-bound radioactivity in the presence of the test compound at the specified test concentration with a control value determined in the absence of the test compound. Using this procedure compounds showing at least 50% displacement of radiolabelled All binding at a concentration of 10⁻⁴ M are retested at lower concentrations to determine their potency. For determination of the IC₅₀ (concentration for 50% displacement of radiolabelled All binding), concentrations of the test compound are ordinarily chosen to allow testing over at least four orders of magnitude centred about the predicted approximate IC₅₀, which latter is subsequently determined from a plot of percentage displacement against concentration of the test compound.

In general, acidic compounds of formula I as defined above show significant inhibition in **Test A** at a concentration of 50 micromolar or much less.

### Test B:

This in vitro test involves the measurement of the antagonistic effects of the test compound against All-induced contractions of isolated rabbit aorta, maintained in a physiological salt solution at 37°C. In order to ensure that the effect of the compound is specific to antagonism of All, the effect of the test compound on noradrenaline-induced contractions may also be determined in the same preparation.

In general, acidic compounds of formula I as defined above show significant inhibition in **Test B** at a final concentration of 50 micromolar or much less.
[Note: Compounds of formula I wherein Z is a group of the formula **-CO.OR**^{**5**} in which R⁵ is other than hydrogen in general show only weak activity in the in vitro **Tests A or B.]**

### Test C:

This in vivo test involves using terminally-anaesthetised or conscious rats in which an arterial catheter has been implanted under anaesthesia for the measurement of changes in blood pressure. The All antagonistic effects of the test compound following oral or parenteral administration, are assessed against angiotensin II-induced pressor responses. To ensure that the effect is specific the effect of the test compound on vasopressin-induced pressor responses may also be determined in the same preparation.

The compounds of formula I generally show specific All-antagonist properties in **Test C** at a dose of 50 mg/kg body weight or much less, without any overt toxicological or other untoward pharmacological effect.

### Test D:

This in vivo involves the stimulation of endogenous All biosynthesis in a variety of species including rat, marmoset and dog by introducing a diet of low sodium content and giving appropriate daily doses of a saluretic known as frusemide. The test compound is then administered orally or parenterally to the animal in which an arterial catheter has been implanted under anaesthesia for the measurement of changes in blood pressure.

In general compounds of formula I will show All-antagonist properties in **Test D** as demonstrated by a significant reduction in blood pressure at a dose of 50 mg/kg body weight or much less, without any overt toxicological or other untoward pharmacological effect.

The compounds of formula I will generally be administered for therapeutic or prophylactic purposes to warm-blooded animals (including man) requiring such treatment in the form of a pharmaceutical composition in conjunction with a pharmaceutically acceptable diluent or carrier, as is well known in the pharmaceutical art. Such a pharmaceutical composition is provided as a furtherfaeture of the invention and will conveniently be in a form suitable for oral administration (e.g. as a tablet, capsule, solution, suspension or emulsion) or parenteral administration (e.g. as an injectable aqueous or oily solution, or injectable emulsion).

The compounds of formula I may also be advantageously administered for therapeutic or prophylactic purposes together with another pharmacological agent known in the general art to be of value in treating one or more of the diseases or medical conditions referred to hereinabove.

In general a compound of formula I (or a pharmaceutically acceptable salt thereof as appropriate) will generally be administered to man so that, for example, a daily oral dose of up to 50 mg/kg body weight (and preferably of up to 10 mg/kg) or a daily parenteral dose of up to 5 mg/kg body weight (and preferably of up to 1 mg/kg) is received, given in divided doses as necessary, the precise amount of compound (or salt) administered and the route and form of administration depending on size, age and sex of the person being treated and on the particular disease or medical condition being treated according to principles well known in the medical arts.

By way of illustration of the angiotensin II inhibitory properties of compounds of formula I, the compound of Example 3 gave the following results in tests A, B and C described above:-
In test A: IC₅₀ of 1.49x10⁻⁸M;
In test B: pA₂ of 9.2;
In test C: ED₅₀ of 1.07mg/kg (parenteral administration); 54% inhibition after one hour at a dose of 20mg/kg (oral administration).

In addition to their aforesaid use in therapeutic medicine in humans, the compounds of formula I are also useful in the veterinary treatment of similar conditions affecting commercially valuable warm-blooded animals, such as dogs, cats, horses and cattle. In general for such treatment, the compounds of the formula I will generally be administered in an analogous amount and manner to those described above for administration to humans. The compounds of formula I are also of value as pharmacological tools in the development and standardisation of test systems for the evaluation of the effects of All in laboratory animals such as cats, dogs, rabbits, monkeys, rats and mice, as part of the continuing search for new and improved therapeutic agents.

The invention will now be illustrated by the following non-limiting Examples in which, unless otherwise stated:-
(i) concentrations and evaporations were carried out by rotary evaporation in vacuo;
(ii) operations were carried out at room temperature, that is in the range 18-26°C;
(iii) flash column chromatography was performed on Merck Kieselgel 60 (Art. no. 9385) obtained from E Merck, Darmstadt, Germany;
(iv) yields, where given, are intended for the assistance of the reader only and are not necessarily the maximum attainable by diligent process development;
(v) proton NMR spectra were normally determined at 200 MHz in CDCl₃ using tetramethylsilane (TMS) as an internal standard, and are expressed as chemical shifts (delta values) in parts per million relative to TMS using conventional abbreviations for designation of major peaks: s, singlet; m, multiplet; t, triplet; br, broad; d,doublet; and
(vi) all end-products had satisfactory microanalyses.

### Example 1

4M Aqueous sodium hydroxide solution (0.4 ml) was added to a solution of methyl 4'-[(2butyl-3H-imidazo[4,5-b]pyrid-3-yl)methyl]-biphenyl-2-carboxylate (A) (210 mg) in methanol (3 ml). The solution was heated under reflux for 2 hours and then volatile material was removed by evaporation. The residue was dissolved in water (5 ml) and the solution acidified to pH 4 with 20% w/v aqueous citric acid. The precipitated solid was filtered off and triturated with ether to give 4'-[(2-butyl-3H-imidazo[4, 5-b]pyrid-3-yl)-methyl]-biphenyl-2-carboxylic acid (153 mg), as a cream powder, m.p. 185-187°C; NMR (d₆-DMSO): 0.9(t,3H), 1.35(m,2H), 1.7(m,2H), 2.85(t,2H), 5.6(s,2H), 7.1-7.6 (complex m,8H), 7.7(dd,1H), 8.0(dd,1H), 8.3(dd,1H), 12.6(br, 1H); mass spectrum (negative fast atom bombardment, hereinafter referred to as "-ve FAB"), 199, 174, 168; microanalysis found: C,74.3; H,5.9; N,10.7 C₂₄H₂₃N₃O₂ requires: C,74.8; H,6.0; N,10.9%.

The starting material (A) was obtained as follows:-
(i) A 1.6M solution of butyllithium in hexane (24.0 ml) was added dropwise to a stirred solution of 4-bromotoluene (6.0 g) in dry tetrahydrofuran (THF) (50 ml) at -78°C under an atomosphere of argon. The temperature was maintained at -78°C for 20 minutes and then a 1M solution of anhydrous zinc chloride in ether (38.6 ml) was added. The solution was kept at -78°C for 15 minutes and then tetrakis(triphenylphosphine)palladium (60 mg) in THF (5 ml) was added, followed by methyl-2-idodobenzoate (6.1 g) in THF (10 ml). The solution was allowed to reach ambient temperature over 1 hour and then heated under reflux for 5 hours. The solvent was removed by evaporation and the residue was dissolved in chloroform (150 ml). The solution was washed with a solution of ethylenediaminetetraacetic acid (10 g) in water (100 ml) and the aqueous layer was re-extracted with chloroform (100 ml). The combined organic extracts were dried (MgSO₄) and the solvent removed by evaporation. The residue was purified by flash chromatography, eluting with ethyl/acetate hexane (1:9 v/v to give methyl 4'-methylbiphenyl-2-carboxylate (B) as a colourless oil (4.4 g); NMR(CDCl₃): 2.4(s,3H), 3.65(s,3H), 7.2(s,4H), 7.35(m,2H), 7.5(m,1H), 7.8(d,1H).
(ii) N-Bromosuccinimide (8.1 g) and azo(bisisobutyronitrile) (130 mg) were added to a solution of the compound (B) (9.3 g) in carbon tetrachloride (300 ml). The mixture was heated under reflux for 4 hours and then cooled to ambient temperature. Insoluble material was removed by filtration and the filtrate concentrated in vacuo. The residue was purified by flash chromatography, eluting with ethyl acetate/hexane (1:9 v/v) to give methyl 4'-(bromomethyl)biphenyl-2-carboxylate (C) as a solid (10.9 g), m.p. 48°C; NMR: 3.65(s,3H), 4.55(s,2H), 7.25-7.60 (complex m,7H), 7.85(d,1H).
(iii) A mixture of 2-butylimidazo[4,5-b]pyridine (400 mg), [Indian J. Chem., Sec. B, 1978, 16B, 531], the bromomethyl compound (C) (700 mg) and potassium carbonate (630 mg) in N,N-dimethylformamide (DMF) (7 ml) was stirred for 4 hours. The solvent was removed by evaporation and the residue partitioned between water (20 ml) and ethyl acetate (20 ml). The organic phase was separated, washed with water (20 ml) and then with saturated brine (20 ml), dried (MgSO₄) and the solvent evaporated. The residue was purified by flash chromatography, eluting with methanol/dichloromethane (3:97 v/v, gradually changing to 5:95 v/v), to give initially methyl 4'-[(2-butyl-3H-imidazo [4,5-b]pyrid-3-yl)methyl]biphenyl-2-carboxylate (A) (260 mg), as an orange gum; NMR (CDCl₃): 0.9(t,3H), 1.4(m,2H), 1.8(m,2H), 2.85(t,2H), 3.6(s,3H), 5.55(s,2H), 7.1-7.6(complex m,8H), 7.8(dd,1H), 8.0(dd,1H), 8.35(d,1H); and then methyl 4'-[(2butyl-1H-imidazo[4,5-b]pyrid-1-yl)methyl]biphenyl-2-carboxylate (80 mg), as a yellow gum; NMR(CDCl₃): 0.9(t,3H), 1.45(m,2H), 1.85(m,2H), 2.9(t,2H), 3.6(s,3H), 5.4(s,2H), 7.0-7.6(complex m,9H), 7.8(dd,1H), 8.5(dd,1H); and finally methyl 4'-[(2-butyl-4H-imidazo[4,5-b]pyrid-4-yl)methyl]biphenyl-2-carboxylate (220 mg), as a yellow gum; NMR(CDCl₃): 1.0(t,3H), 1.5(m,2H), 1.9(m,2H), 3.05(t,2H), 3.6(s,3H), 5.9(s,2H), 7.0(t,1H), 7.2-7.6(complex m,3H), 7.85(d,1H), 8.1(d,1H).

### Example 2

Using an analogous procedure to that described in Example 1, methyl 4'-[(2-butyl-1H-imidazo[4,5-b]pyrid-1-yl)methyl]biphenyl-2-carboxylate (50 mg) was hydrolysed to give 4'-[(2-butyl-1H-imidazo[4,5-b]pyrid-1-yl)methyl]biphenyl-2-carboxylic acid (26 mg), as a non-crystalline solid; NMR (d₆-DMSO): 0.9(t,3H), 1.4(m,2H), 1.75(m,2H), 2.9(t,2H), 5.6(s,2H), 7.1-7.6(complex m,8H), 7.7(dd,1H), 7.9(dd,1H), 8.35(dd,1H), 12.7(br,1H); mass spectrum (-ve FAB, DMSO/nitrobenzyl alcohol): 384(M-H)⁻; microanalysis found: C,71.6; H.5.6; N,9.7; C₂₄H₂₃N₃O₂.H₂O requires C,71.8; H,6.2; N,10.4%.

### Example 3

2-Butyl-3[(2'-(2-triphenymethyl-2H-tetrazol-5-yl)biphenyl-4-yl)methyl]-3H-imidazo[4,5-b]pyridine (D) (600 mg) was dissolved in dioxan (20 ml) and concentrated hydrochloric acid (20 ml) was added. The solution was allowed to stand for 6 hours and then volatile material was removed by evaporation. The residue was stirred vigorously with dichloromethane (5 ml) and ether (20 ml) was added. The mixture was stirred for 15 minutes. The solid material was collected by filtration and washed with ether (10 ml) to give 2-butyl-3-[(2'-(1H-tetrazol-5-yl)biphenyl-4-yl)methyl]-3H-imidazo[4,5-b]pyridine hydrochloride (300 mg), as a cream powder, m.p. 224°C (decomposition); NMR (d₆-DMSO): 0.9(t,3H), 1.4(m,2H),1.1 (m,2H), 3.05(t,2H), 5.6(s,2H), 7.1(d,2H), 7.2(d,2H), 7.4-7.7(complex, 5H), 8.2(dd,1H), 8.5(dd,1H); mass spectrum (-ve FAB, DMSO/nitrobenzyl alcohol): 408(M-H)⁻; microanalysis found: C,64.2; H,5.6; N,219; Cl,8.0; C₂₄H₂₃N₇,HCl requires: C,64.6; H,5.4; N,22.0; Cl 8.5%.

The starting material (D) was obtained as follows:-
A mixture of 2-butylimidazo[4,5-b]pyridine (1.0 g), 5-[2-(4'-bromomethyl biphenylyl)]-2-triphenylmethyl-2H-tetrazole (obtained as described in European patent application, publication no. 291969; 3.17 g) and potassium carbonate (1.16 g) in DMF (20 ml) was heated at 95°C overnight. The reaction mixture was then treated using the work-up procedure described in Example 1, part (iii). The material obtained was purified by flash chromatography, eluting with ethyl acetate/ hexane (17.3 v/v), gradually changing to ethyl acetate/methanol (9:1 v/v). There was thus obtained initially 2-butyl-3-[(2'-(2-triphenylmethyl-2H-tetrazol-5-yl)biphenyl-4-yl)methyl]-3H-imidazo[4,5-b]pyridine (D) (0.72 g), as a yellow foam; NMR(CDCl₃): 1.0(t,3H), 1.45(m,2H), 1.9(m,2H), 2.8(t,2H), 5.5(s,2H), 7.0(m,8H), 7.2(d,2H), 7.3-7.5 (complex, 12H) 7.55(m,2H), 8.0(m,1H), 8.1(dd,1H), 8.45(dd,1H); and then 2-butyl-4-[(2'-(2-triphenylmethyl-2H-tetrazol-5-yl)biphenyl-4-yl)methyl]-4H-imidazo[4,5-b]pyridine (1.10 g), as a reddish foam; NMR: 1.0(t,3H), 1.5(m,2H), 1.9(m,2H), 3.1(t,2H), 5.7(s,2H), 6.7(dd,1H), 6.9(m,6H), 7.1-7.4 (complex m,16H), 7.5(m,1H), 8.0(m,1H), 8.1(d,1H).

### Example 4

Using an analogous procedure to that described in Example 1, methyl 4-[(2-butyl-3H-imidazo[4,5-b]pyrid-3-yl)methyl]benzoate (E) (267 mg) was hydrolysed to give 4-[2-butyl-3H-imidazo[4,5-b]pyrid-3-yl)methyl]benzoic acid (239 mg), as a light brown solid, m.p. 174-176°C; NMR (d₆-DMSO): 0.85(t,3H), 1.3(m,2H), 1.7(m,2H), 2.8(t,2H), 5.6(s,2H), 7.25(m,3H), 7.9(d,2H), 8.0(dd,1H), 8.3(dd,1H), 12.85(br,1H); mass spectrum (+ve chemical ionisation): 310(M+H)⁺; microanalysis found: C,69.6; H,6.3; N,13.4%; C₁₈H₁₉N₃O₂ requires: C,69.9; H,6.15; N,13.6%.

The starting material (E) was obtained as follows:-
(i) A solution of 2-chloro-3-nitropyridine (1.5 g) in chloroform (30 ml) was added to a solution of methyl 4-(aminomethyl)benzoate (3.12 g) and triethylamine (2.0 ml) in toluene (50 ml). The solution was heated under reflux for 5 hours and then volatile material was removed by evaporation. The residue was purified by flash chromatography, eluting with ethyl acetate/hexane (1:1 v/v), to give methyl 4-N-(3-nitropyrid-2-yl)aminomethyl benzoate (F) (2.58 g), as a bright yellow solid, m.p. 135-136°C; NMR(CDCl₃): 3.9(s,3H), 4.9(d,2H), 6.7(dd,1H), 7.4(d,2H), 8.0(d,2H), 8.4(m,2H), 8.55(br s, 1H).
(ii) A solution of the nitro compound (F) (530 mg) in methanol (150 ml) was catalytically hydrogenated over platinum oxide catalyst (100 mg) at 1 bar pressure. After uptake of hydrogen had ceased (approximately 15 minutes), the catalyst was removed by filtration through diatomaceous earth and the filtrate evaporated to give methyl 4-N-(3-aminopyrid-2-yl)aminomethylbenzoate (G) (470 mg), as a red gum which was used without purification in the next stage; NMR(CDCl₃): 3.2(br,2H), 3.9(s,3H), 4.5(br,1H), 4.7(br s,2H), 6.55(dd,1H), 6.9(dd,1H), 7.45(d,2H), 7.75(dd,1H), 8.0(d,2H).
(iii) A solution of the diamine (G) (530 mg), valeric anhydride (0.38 g), valeric acid (0.21 g) and p-toluenesulphonic acid (0.39 g) in toluene (20 ml) was heated under reflux for 14 hours. The solution was diluted with ethyl acetate (50 ml) and washed successively with sodium carbonate solution (30 ml), water (30 ml) and saturated brine (30 ml), and then dried (MgSO₄). Volatile material was removed by evaporation and the residue was purified by flash chromatography, eluting with ethyl/hexane (1:1 v/v), to give methyl 4-[(2-butyl-3H-imidazo[4,5-b]pyrid-3-yl)methyl]benzoate (E) (300 mg), as a beige solid, m.p. 77-80°C; NMR-(CDCl₃): 0.9(t,3H), 1.4(m,2H), 1.8(m,2H), 2.75(t,2H), 3.9(s,3H), 5.5(s,2H), 7.2(m,3H), 7.95(d,2H), 8.0(dd,1H), 8.3(dd,1H).

### Example 5

4-[(2-Butyl-3H-imidazo[4,5-b]pyrid-3-yl)methyl]benzoic acid (150 mg) was added to a solution of benzenesulphonamide (84 mg), dimethylaminopyridine (59 mg) and 1-[3-(dimethylamino)propyl]-3-ethylcarbodiimide (93 mg) in dry dichloromethane (5 ml). The mixture was stirred for 6 hours and then diluted with dichloromethane (20 ml). The solution was washed successively with sodium carbonate solution (20 ml), water (20 ml) and saturated brine (20 ml), and then dried (MgSO₄). The solvent was removed by evaporation and the residue was purified by flash chromatography, eluting with methanol/dichloromethane (1:9v/v) to give 4-[(2-butyl-3H-imidazo[4,5-b]pyrid-3-yl)methyl]-N-(phenylsulphonyl)benzamide (110 mg), as a cream powder, m.p. >270°C; NMR (d₆-DMSO): 0.85(t,3H), 1.3(m,2H), 1.65(m,2h), 2.8(t,2H),5.5(s,2H), 7.1(d,2H), 7.25(dd,1H), 7.4(m,3H), 7.89(m,4H), 8.0(dd,1H), 8.3(dd,1H); mass spectrum (positive fast atom bombardment, hereinafter referred to as "+ve FAB", DMSO/nitrobenzyl alcohol): 449(M+H)⁺; microanalysis found: C,59.4; H,5.1; N,11.3%; C₂₄H₂₃N₄O₃S.2H₂O requires: C,59.5; H,5.6; N,11.6%.

### Examples 6-9

Using an analogous procedure to that described in Example 1, but starting from the appropriate ester instead of compound A, the following compounds were prepared in yields of 54-74%:-

### (Example 6):

4'-[(2-butyl-5-bromo-1H-imidazo[4,5-b]pyrazin-1-yl)methyl]biphenyl-2-carboxylic acid was obtained as a foam; NMR (d₆-DMSO): 0.9(t,3H), 1.4(m,2H), 1.7(m,2H), 3.0(t,2H), 5.6(s,2H), 7.1-7.3(m,5H), 7.5(m,2H), 7.7(dd,1H), 8.5(s,1H), 12.3(bs,1H); mass spectrum (chemical ionisation, ammonia): 465(M+H)⁺; microanalysis, found: C,59.3; H,5.0; N,11.6%; C₂₃H₂₁N₄O₂Br requires: C,59.4; H,5.0; N,12.0%;

### (Example 7):

4'-[(8-butyl-9H-purin-9-yl)methyl]biphenyl-2-carboxylic acid was obtained as a solid, m.p. 153-155°C; NMR(d₆-DMSO): 0.9(t,3H), 1.4(m,2H), 1.7(m,2H), 2.9(t,2H), 5.6(s,2H), 7.1-7.35(m,5H), 7.5(m,2H), 7.7(dd,1H), 8.9(s,1H), 9.1(s,1H), 12.5(bs,1H); mass spectrum (-ve FAB, DMSO/glycerol): 385(M-H)⁻; microanalysis, found: C,71.4; H,6.1; N,14.4%; C₂₃H₂₂N₄O₂ requires: C,71.5; H,5.7; N,14.5%;

### (Example 8):

4'-[(8-butyl-7H-purin-7-yl)methyl]biphenyl-2-carboxylic acid was obtained as a solid, m.p. 150-153°C; NMR (d₆-DMSO): 0.9(t,3H), 1.4(m,2H), 1.7(m,2H), 3.0(t,2H), 5.6(s,2H), 7.2-7.4(m,5H), 7.5(m,2H), 7.7(dd,1H), 8.9(s,1H), 9.1(s,1H); mass spectrum (chemical ionisation, ammonia): 387(M+H)⁺; microanalysis, found: C,69.6; H,5.5; N,13.8%; C₂₃O₂₂N₄O₂,0.5H₂O requires C,69.9; H,5.8; N,14.2%; and

### (Example 9):

4'-[(2-butyl-4-hydroxy-3H-imidazo[4,5-d]pyridazin-3-yl)methyl]biphenyl-2-carboxylic acid was obtained as a solid, m.p. 232-233°C; NMR (d₆-DMSO): 0.8(t,3H); 1.3(m,2H), 1.6(m,2H), 2.75(t,2H), 5.8(s,2H), 7.18-7.35(m,5H), 7.45(m,20), 7.7(dd,1H), 8.35(d,1H), 12.75(s,1H); mass spectrum (-ve FAB, DMSO/glycerol): 401(M-H)⁻; microanalysis, found: C,68.3; H,5.4; N,13.7%; C₂₃H₂₂N₂O₄ requires C,68.7; H,5.5; N,13.9%.

The necessary starting materials for Examples 6-9, corresponding to starting material A in Example 1, were prepared using an analogous procedure to that described in Example 1, part (iii) by alkylation of the appropriate imidazole derivative of formula IV with 4'-(bromomethyl)biphenyl-2-carboxylate. The compounds were obtained in yields of 12-74% as follows:-

### (Example 6A):

Alkylation of 2-butyl-5-bromoimidazo[4,5-b]pyrazine (0.30 g) gave a mixture of regioisomers which was separated by flash chromatography, eluting with ethyl acetate/hexane (initially 1:4v/v gradually changing to 2:3v/v), to give methyl[(2-butyl-5-bromo-1H-imidazo[4,5-b]pyrazin-1-yl)methyl]biphenyl-2-carboxylate, as a gum; NMR (CDCl₃): 0.9(t,3H), 1.4(m,2H), 1.8(m,2H), 2.9(t,2H), 3.6(s,3H), 5.5(s,2H), 7.18-7.35(m,5H), 7.5(m,2H), 7.85(dd,1H), 8.4(s,1H); mass spectrum (chemical ionisation, ammonia): 479(M+H)⁺; the starting material 2-butyl-5-bromoimidazo[4,5-b]pyrazine was obtained by cyclisation of 5-bromo-2,3-diaminopyrimidine as follows:-

A mixture of 5-bromo-2,3-diaminopyrimidine (obtained as described in Gazz. Chim. Ital., 1960, 90, 1809) (0.4 g), valeric acid (0.6 ml) and polyphosphoric acid (10 g) was heated at 160°C for 1.5 hours. The mixture was cooled to ambient temperature, poured into water and the solution basified with sodium carbonate solution. The solution was extracted with ethyl acetate and the organic extract worked up using the procedure described in Example 1, part (iii). The resultant product was recrystallised from ethyl acetate to give 2-butyl-5-bromoimidazo[4,5-b]pyrazine (0.33 g) as a solid, m.p. 211-213°C; NMR (CDCl₃/d₆-DMSO): 1.0(t,3H), 1.4(m,2H), 1.9(m,2H), 3.0(t,2H), 8.3(s,1H); mass spectrum (chemical ionisation, ammonia) 255(M+H)⁺;

### (Examples 7A and 8A):

Alkylation of 8-butylpurine (0.68 g) gave a mixture of regioisomers, which was separated by flash chromatography, eluting with dichloromethane/methanol (95:5 v/v). The less polar isomerwas repurified by flash chromatography, eluting with ethyl acetate, to give methyl 4'-[(8-butyl-9H-purin-9-yl)methyl]biphenyl-2-carboxylate **(Example 7A)** (0.40 g) as a solid, m.p. 83-84°C; NMR (CDCl₃): 0.9(t,3H), 1.4(m,2H), 1.8(m,2H), 2.9(t,2H), 3.6(s,3H), 5.5(s,2H), 7.1-7.35(m,5H), 7.5(m,2H), 7.8(dd,1H), 8.9(s,1H), 9.1(s,1H); mass spectrum (chemical ionisation, ammonia): 401 (M+H)⁺; microanalysis, found: C,71.9; H,6.1; N,13.9%; C₂₄H₂₄N₄O₂ requires C,72.0; H,6.0; N,14.0%. The more polar isomer was similarly repurified to give methyl 4'-[(8-butyl-7H-purin-7-yl)methyl]biphenyl-2-carboxylate **(Example 8A)** (0.19 g); NMR (CDCl₃): 0.9(t,3H), 1.5(m,2H), 1.9(m,2H), 3.0(t,2H), 3.7(s,3H), 5.4(s,2H), 7.2(m,5H), 7.5(m,2H), 7.9(dd,1H), 8.6(s,1H), 9.1(s,1H); mass spectrum (chemical ionisation, ammonia): 401 (M+H)⁺; the starting material 8-butylpurine was obtained by cyclisation of 2,3-diaminopyrimidine as follows:-
A mixture of 2,3-diaminopyrimidine (0.55 9) and valeric anhydride (4.9 ml) was heated at 210°C for 1.5 hours under an atmosphere of argon. The mixture was cooled to ambient temperature, added to methanol (100 ml) and the resultant solution heated at reflux for 1.5 hours. Solvent and volatile material was removed by evaporation (60°C/0.1 mm of mercury) and the residue purified by flash chromatography eluting with ethyl acetate/methanol (5:1 v/v). The product was triturated with ether to give 8-butylpurine, m.p.164-165°C; NMR (CDCl₃): 1.0(t,3H), 1.5(m,2H),2.0(m,2H), 3.1(t,2H), 8.95(s,1H), 9.1(s,1H); mass spectrum (chemical ionisation, ammonia): 177(M+H)⁺; and

### (Example 9A):

Alkylation of 2-butyl-4-hydroxyimidazo[4,5-d]pyridazine gave methyl 4'-[(2-butyl-4-hydroxy-3H-imidazo[4,5-d]pyridazin-3-yl)methyl]biphenyl-2-carboxylate as a solid, m.p. 166-168°C; NMR (d₆-DMSO): 0.8(t,3H), 1.3(m,2H), 1.6(m,2H), 2.75(t,2H), 3.55(s,3H), 5.78(s,2H), 7.2-7.3(m,4H), 7.4(d,1H), 7.5(dt,1H), 7.6(dt,1H), 7.7(dd,1H), 8.4(s,1H), 12.8(s,1H); mass spectrum (chemical ionisation, ammonia): 417(M+H)⁺; the starting material 2-butyl-4-hydroxyimidazo[4,5-d]pyridazine was obtained as follows:-
4,5-diamino-3-hydroxypyridazine (obtained as described in Belgian patent No. 660637; Chem. Abs., 1966, 64, 5108) (5 g) was cyclised in an analogous manner to that described in Examples 7A and 8A for the preparation of 8-butylpurine to give 2-butyl-4-hydroxyimidazo[4,5-d]pyridazine (2.4 g) as a solid, m.p. 241-243°C; NMR(d₆-DMSO): 0.9(t,3H), 1.3(m,2H), 1.7(m,2H), 2.8(t,2H), 8.28(s,1H), 12.6(s,1H), 13.5(bs,1H); mass spectrum (chemical ionisation, ammonia): 193(M+H)⁺; microanalysis, found: C,55.9; H,6.2; N,29.0%; C₉H₁₂N₄O requires C,56.3; H,6.3; N,29.2%.

### Examples 10-16

Using an analogous procedure to that described in Example 3, but starting from the appropriate compound of formula III wherein L is triphenylmethyl, the following compounds were prepared in yields of 36-95%:-

### (Example 10):

2-butyl-4-hydroxy-3[(2'(1H-tetrazol-5-yl)biphenyl-4-yl)methyl]-3H-imidazo[4,5-d]pyridazine was obtained as a solid, m.p. 227-230°C; NMR (d₆-DMSO): 0.8(t,3H), 1.25(m,2H), 1.6(m,2H), 2.75(t,2H), 5.75(s,2H), 6.4(bs,3H), 7.05-7.2(m,4H), 7.45-7.7(m,4H), 8.35(s,1H); mass spectrum (-ve FAB, DMSO/glycerol): 425(M-H)⁻; microanalysis, found: C,59.4; H,4.9; N,23.6; Cl,7.3%; C₂₃H₂₂N₈O.HCl requires C,59.7; H,5.0; N,24.2; Cl,7.7%;

### (Example 11):

2-butyl-7-methyl-3-[(2'-(1H-tetrazol-5-yl)biphenyl-4-yl)methyl]-3H-imidazo[4,5-b]pyridine was obtained as a solid, m.p. 183-190°C (decomposition); NMR (d₆-DMSO): 0.9(t,3H), 1.4(m,2H), 1.7(m,2H), 2.7(s,3H), 3.1(t,2H), 5.7(s,2H), 7.1(dd,4H), 7.4(d,1H), 7.6(m,4H), 8.4(d,1H); mass spectrum (-ve FAB, DMSO/glycerol): 422(M-H)⁻; microanalysis, found: C,61.1; H,5.4; N,21.0%; C₂₅H₂₅N₇.HCl requires: C,65.3; H,5.7; N,21.3%;

### (Example 12):

2-butyl-5-methyl-3-[(2'-(1H-tetrazol-5-yl)biphenyl-4-yl)methyl]-3H-imidazo[4,5-b]pyridine was obtained as a solid; NMR (d₆-DMSO): 0.9(t,3H), 1.3(m,2H), 1.7(m,2H), 2.6(s,3H), 2.75(t,2H), 5.5(s,2H), 7.1(m,5H), 7.6(m,4H); mass spectrum (+ve FAB, DMSO/glycerol): 424(M+H)⁺, accurate mass determination 424.2272; C₂₅H₂₆N₇ requires 424.2250;

### (Example 13):

2-butyl-5-chloro-3-[(2'-(1H-tetrazol-5-yl)biphenyl-4-yl)methyl]-3H-imidazo[4,5-b]pyridine was obtained as a solid, m.p. 222-223°C (decomposition); NMR (d₆-DMSO): 0.8(t,3H), 1.3(m,2H), 1.7(m,2H), 3.0(t,2H), 5.6(s,2H), 7.3(dd,4H), 7.6(m,5H), 8.2(d,1H); mass spectrum (-ve FAB, DMSO/glycerol): 444, 442(M-H)⁻; microanalysis, found: C,59.3; H,5.6; N,17.5, Cl,13.1%; C₂₄H₂₂N₇Cl,HCl,CH₃CO₂C₂H₅ requires: C,59.2; H,5.3; N,17.3; Cl,12.7%;

### (Example 14):

2-butyl-5-chloro-1-[(2'-(1H-tetrazol-5-yl)biphenyl-4-yl)methyl]-1H-imidazo-[4,5-b]pyridine was obtained as a solid, m.p. 156°C (decomposition); NMR (CDCl₃): 0.9(t,3H), 1.45(m,2H), 1.7(m,2H), 3.0(t,2H), 3.0(t,2H), 5.6(s,2H), 7.1(dd,4H), 7.5-7.7(m,5H), 8.1(d,1H); mass spectrum (-ve FAB, DMSO/glycerol): 444, 442(M-H)⁻; microanalysis, found: C,58.9; H,5.3; N,19.2; H₂-0,1.8%; C₂₄H₂₂N₇Cl,0.75CH₃OH,0.5H₂O requires: C,58.3; H,5.3; N,18.9%.

### (Example 15):

2-butyl-7-chloro-3-[(2'-(1H-tetrazol-5-yl)biphenyl-4-ylmethyl]-3H-imidazo[4,5-b]pyridine was obtained as a solid, m.p. 170-180°C (decomposition); NMR (d₆-DMSO): 0.9(t,3H), 1.4(m,2H), 1.7(m,2H), 2.9(t,2H), 5.5(s,2H), 7.1(dd,4H), 7.4-7.7(m,5H), 8.3(d,1H); mass spectrum (-ve FAB, DMSO/glycerol): 444, 442(M-H)⁻; microanalysis, found: C,60.4; H,4.6; N,20.3%; C₂₄H₂₂N₇Cl,HCl requires: C,60.0; H,4.8; N,20.4%; and

### (Example 16):

2-butyl-5,7-dimethyl-3-[(2'-(1H-tetrazol-5-yl)biphenyl-4-yl)methyl]-3H-imidazo[4,5-b]pyridine was obtained as a solid; NMR (d₆-DMSO): 0.83(t,3H), 1.30(m,2H), 1.65(m,2H), 2.60(s,3H), 2.65(s,3H), 3.1(t,3H), 5.7(s,2H), 7.1(d,2H), 7.2(d,2H), 7.33(s,1H), 7.4-7.8 (complex m,4H); mass spectrum (-ve FAB, DMSO/glycerol): 436(M-H)⁻; microanalysis, found: C,65.4; H,5.8; N,20.1%; C₂₆O₃₇N₇,1HCl requires: C,65.85; H,6.07; N,20.37%.

The necessary starting materials for Examples 10-16, corresponding to starting material D in Example 3, were prepared using an analogous procedure to that described in Example 3, part (i) by alkylation of the appropriate imidazole derivative of formula IV. The compounds were obtained in yields of 19-67% as follows:-

### (Example 10D):

Alkylation of 2-butyl-4-hydroxyimidazo[4,5-d]pyridine (obtained as described in Example 9A) gave 2-butyl-4-hydroxy-3-[(2'-(2-triphenylmethyl-2H-tetrazol-5-yl)biphenyl-4-yl)methyl]-3H-imidazo[4,5-d]pyridazine as a solid, m.p. 169-170°C; NMR(CDCl₃): 0.9(t,3h), 1.3(m,2H), 1.7(m,2H), 2.67(t,2H), 5.65(s,2H), 6.8-6.95(complex m,6H), 7.0(d,2H), 7.1(d,2H), 7.2-7.35(complex m,10H), 7.45(m,2H), 7.9(m,1H), 8.35(s,1H), 11.16(s,1H); mass spectrum (-ve FAB, DMSO/glycerol): 667(M-H)⁻;

### (Example 11D):

Alkylation of 2-butyl-7-methylimidazo[4,5-b]pyridine gave a mixture of regioisomers which was separated by flash chromatography, eluting with dichloromethane/methanol (97:3, v/v), to give 2-butyl-7-methyl-3-[(2'-(triphenylmethyl-2H-tetrazol-5-yl)biphenyl-4-yl)methyl]-3H-imidazo[4,5-b]pyridine as a gum; NMR (CDCl₃): 0.9(t,3H), 1.3(m,2H), 17(m,2H), 2.7(s,3H), 2.75(t,2H), 5.4(s,2H), 6.85-6.95(complex m,8H), 7.1(m,3H), 7.15-7.35(complex m,10H), 7.4(m,2H), 79(m,1H), 8.2(d,1H); mass spectrum (-ve FAB, DMSO/glycerol): 422(M-trityl)⁻; the starting material 2-butyl-7-methylimidazo[4,5-b]pyridine was obtained as follows:-
2-amino-4-methyl-3-nitropyridine was catalytically hydrogenated, using an analogous procedure to that described in Example 4, part (ii), and the diamine obtained was cydised directly, using an analogous procedure to that described in Example 6A for the cyclisation of 5-bromo-2,3-diaminopyrimidine, to give 2-butyl-7-methyl imidazo[4,5-b]pyridine as a solid; NMR (CDCl₃): 1.0(t,3H), 1.5(m,2H), 1.9(m,2H), 2.7(s,3H), 3.1(t,2H);

### (Example 12D):

Alkylation of 2-butyl-5-methylimidazo[4,5-b]pyridine gave a mixture of regioisomers which was separated by flash chromatography, eluting first with dichloromethane/methanol (19:1 v/v) and gradually changing to dichloromethane/methanol (9:1 v/v). There was thus obtained 2-butyl-5-methyl-3[(2'-(2-triphenylmethyl-2H-tetrazol-5-yl)biphenyl-4-yl)methyl]-3H-imidazo[4,5-b]pyridine as a solid, m.p. 149-150°C (decompostion); NMR (CDCl₃): 0.9(t,3H), 1.5(m,2H), 1.7(m,2H), 2.6(s,3H), 2.7(t,2H), 5.4(s,2H), 6.89-6.95(complex m,8H), 7.26-7.4(complex m,10H), 7.4(m,2H), 7.9(m,2H); mass spectrum (-ve FAB, DMSO/glycerol): 422(M-trityl)⁻; the starting material 2-butyl-5-methylimidazo[4,5-b]pyridine was obtained as follows:-
2-amino-6-methyl-5-nitropyridine (obtained as described in J.A.C.S., 1952, 74, 3829) was catalytically hydrogenated using an analogous procedure to that described in Example 4, part (ii), and the diamine obtained was cydised directly, using an analogous procedure to that described in Examples 7A and 8A for the cyclisation of 2,3-diaminopyrimidine, to give 2-butyl-5-methylimidazo[4,5-b]pyridine as a solid, m.p. 87-89°C; NMR (d₆-DMSO): 0.9(t,3H), 1.5(m,2H), 1.9(m,2H), 2.7(s,3H), 3.0(t,2H), 7.1(d,1H), 7.9(d,1H); mass spectrum (chemical ionisation, ammonia): 190(M+H)⁺; microanalysis, found: C,69.9; H,8.2; N,22.4%; C₁₁H₁₅N₃ requires: C,69.8; H,7.9; N,22.2%;

### (Examples 13D and 14D):

Alkylation of 2-butyl-5-chloroimidazo[4,5-b]pyridine gave a mixture of regioisomers which was separated by flash chromatography, eluting with ethyl acetate/hexane (1:1 v/v), gradually changing to ethyl acetate/hexane (2:1 v/v). There was thus obtained initially 2-butyl-5-chloro-3-[(2'-(2-triphenylmethyl-2H-tetrazol-5-yl)biphenyl-4-yl)methyl]-3H-imidazo[4,5-b]pyridine **(Example 13D)** as a solid, m.p. 76-81°C; NMR (CDCl₃): 0.9(t,3H), 1.3(m,2H), 1.7(m,2H), 2.7(t,2H), 5.3(s,2H), 6.85-6.95(complex m,8H), 7.1(d,2H), 7.2-7.4(complex m,11 H), 7.45(m,2H), 8.95(m,2H): mass spectrum (-ve FAB, DMSO/glycerol): 422, 444(M-trityl)⁻; microanalysis, found: C,75.0; H,5.3; N,13.9; Cl,5.1%; C₄₃H₃₆N₇Cl requires: C,75.3; H,5.3; N,14.3; Cl,5.2%; and then 2-butyl-5-chloro-1-[(2'-(2-triphenylmethyl-2H-tetrazol-5-yl)biphenyl-4-yl)methyl]-1H-imidazo[4,5-b]pyridine (**Example 14D**) as a solid, m.p. 93-98°C; NMR (CDCl₃): 0.9(t,3H), 1.45(m,2H), 1.9(m,2H), 2.8(t,2H), 5.2(s,2H), 6.8(d,2H), 6.9-7.0(complex m,7H), 7.1-7.4(complex m,13H), 7.5(m,2H), 7.9(m,1H); mass spectrum (-ve FAB, DMSO/glycerol): 442, 444(M-trityl)⁻; microanalysis, found: C,74.5; H,5.4; N,14.1; Cl,5.2%; C₄₃H₃₆N₇Cl,0.5H₂O requires; C,74.3; H,5.4; N,14.1; Cl,5.1%; the starting material 2-butyl-5-chloroimidazo[4,5-b]pyridine was obtained as follows:-
Tin (II) chloride (8.2 g) was added to a cooled solution of 2-amino-6-chloro-3-nitropyridine (obtained as described in British patent 1184848) (2.0 g) in concentrated hydrochloric acid (21 ml) over a period of 10 minutes. The cooled solution was stirred for 30 minutes and then basified to pH 10 with 4M sodium hydroxide solution. The resultant solution was extracted with dichloromethane and the organic phase treated using a similar work up procedure to that described in Example 1, part (iii). The crude diamine thus obtained was cyclised directly, using an analogous procedure to that described in Examples 7A and 8A for the cydisation of 2,3-diaminopyrimidine, to give 2-butyl-5-chloroimidazo[4,5-b]pyridine as a solid, m.p. 141-143°C; NMR (CDCl₃): 0.9(t,3H), 1.5(m,2H), 1.7(m,2H), 3.3(t,2H), 7.2(d,1H), 8.0(d,1H); mass spectrum (chemical ionisation, ammonia): 210, 212(M+H)⁺; microanalysis, found: C,57.4; H,5.9; N,20.1; Cl,17.0%; C₁₀H₁₂N₃Cl requires: C,57.3; H,5.3; N,20.0; Cl,16.9%;

### (Example 15D):

Alkylation of 2-butyl-7-chloroimidazo[4,5-b]pyridine gave a mixture of regioisomers, which was separated by flash chromatography, eluting with dichloromethane/methanol (1:49 v/v), gradually changing to dichloromethane/methanol (1:19 v/v) to give initially the less polar and then the more polar isomer. The less polar isomer was repurified by flash chromatography, eluting first with ethyl acetate/hexane (3:7 v/v) and gradually changing to ethyl acetate/hexane (2:3 v/v), to give 2-butyl-7-chloro-3-[(2'-(2-triphenylmethyl-2H-tetrazol-5-yl)biphenyl-4-yl)methyl]-3H-imidazo[4,5-b]pyridine as a gum; NMR (CDCl₃): 0.9(t,3H), 1.3(m,2H), 1.7(m,2H), 2.8(t,2H), 5.4(s,2H), 6.85-6.95(complex m,8H), 7.1(m,2H), 7.2-7.4(complex m,11H), 7.5(m,2H), 7.9(m,1H), 8.2(d,1H): mass spectrum; (-ve FAB, DMSO/glycerol): 442, 444(M-trityl)⁻; the starting material 2-butyl-7-chloroimidazo[4,5-b]pyridine was obtained as follows:-
(i) A mixture of 2-butylimidazo[4,5-b]pyridine (7.6 g), 32% peracetic acid solution (25 ml) and acetic acid (15 ml) was heated at 80°C for 16 hours. The solvent was removed by evaporation and the residue recrystallised from ethyl acetate/methanol (19:1 v/v) to give 2-butyl-1H-imidazo[4,5-b]pyridine-4-oxide (3.0 g) as a solid, m.p. 176-178°C; NMR (d₆-DMSO): 0.9(t,3H), 1.3(m,2H), 1.7(m,2H), 2.8(t,2H), 7.1(dd,1H), 7.5(d,1H), 8.1(d,1H); mass spectrum (chemical ionisation, ammonia): 192(M+H)⁺; microanalysis, found: C,62.5; H,6.5; N,21.8%; C₁₀H₁₃N₃O requires: C,62.8; H,6.8; N,22.0%.
(ii) A mixture of 2-butyl-1H-imidazo[4,5-b]pyridine-4-oxide (0.38 g) and phosphorus oxychloride (5 ml) was heated at reflux for 30 minutes. The dark solution was evaporated and the residue partitioned between ethyl acetate and sodium carbonate solution. The aqueous phase was separated, extracted with ethyl acetate and the combined organic extracts worked up using a similar procedure to that described in Example 1, part (iii). The product obtained was purified by flash chromatography eluting with ethyl acetate/hexane (3:2 v/v) to give 2-butyl-7-chloroimidazo[4,5-b]pyridine (0.24 g) as a solid; NMR (CDCl₃): 1.0(t,3H), 1.5(m,2H), 1.9(m,2H), 3.1(t,2H), 7.3(d,1H), 8.2(d,1H); mass spectrum (chemical ionisation, ammonia): 210, 212(M+H)⁺;

### (Example 16D):

Alkylation of 2-butyl-5,7-dimethylimidazo[4,5-b]pyridine gave a mixture of regioisomers which was separated by flash chromatography, eluting with ethyl acetate/hexane (1:1 v/v), to give 2-butyl-5,7-dimethyl-3-[(2'-(2-triphenylmethyl-2H-tetrazol-5-yl)biphenyl-4-yl)methyl]-3H-imidazo[4,5-b]pyridine as a solid m.p. 148-150°C(decomposition); NMR (CDCl₃): 0.85(t,3H), 1.35(m,2H), 1.65(m,2H), 2.58(s,3H), 2.65(s,3H), 2.7(t,2H), 5.35(s,2H), 6.8-7.0(complex m,9H), 7.05(d,2H), 7.15-7.4(complex m,10H), 7.45(m,2H), 7.9(m,1H); mass spectrum (-ve FAB, DMSO/glycerol); 436(M-trityl)⁻; the starting material 2-butyl-5,7-dimethylimidazo[4,5-b]pyridine was obtained as follows:-
2-amino-4,6-dimethyl-3-nitropyridine (obtained as in Rocz. Chem., 1969, 43, 481; Chem. Abs., 1969, 71, 3228g) was catalytically hydrogenated using a similar procedure to that described in Example 4, part (ii), and the diamine obtained was cyclised directly, using an analogous procedure to that described in Examples 7A and 8A for the cyclisation of 2,3-diaminopyrimidine, to give 2-butyl-5,7-dimethylimidazo[4,5-b]pyridine as a solid, m.p. 111-111.5°C; NMR (CDCl₃): 0.95(t,3H), 1.45(m,2H), 1.85(m,2H), 2.65(s,3H), 2.7(s,3H), 3.3(t,2H), 6.9(s,1H); mass spectrum (chemical ionisation, ammonia): 204(M+H)⁺.

### Examples 17-20

Using an analogous procedure to that described in Example 4, but starting from the appropriate ester instead of compound E, the following compounds were prepared in yields of 38-79%:-

### (Example 17):

4'-[(2-butyl-7-methyl-3H-imidazo[4,5-b]pyrid-3-yl)methyl]biphenyl-2-carboxylic acid was obtained as a solid, m.p. 224-225°C; NMR (d₆-DMSO): 0.9(t,3H), 1.4(m,2H), 1.7(m,2H), 2.6(s,3H), 2.8(t,H), 5.5(s,2H), 7.1(d,1H), 7.1-7.4(m,5H), 7.5(m,2H), 7.7(dd,1H), 8.2(d,1H), 12.6(bs,1H); mass spectrum (chemical ionisation, ammonia): 400(M+H)⁺; microanalysis, found: C,74.9; H,6.1; N,10.3%; C₂₃H₂₅N₃O₂ requires: C,75.2; H,6.3; N,10.5%;

### (Example 18):

4'-[(2-butyl-5-methoxy-3H-imidazo[4,5-b]pyrid-3-yl)methyl]biphenyl-2-carboxylic acid was obtained as a solid, m.p. 211-213°C; NMR (d₆-DMSO): 0.9(t,3H), 1.4(m,2H), 1.7(m,2H), 2.8(t,2H), 3.9(s,3H), 5.5(s,2H), 6.7(d,1H), 7.2-7.5(complex m,7H), 7.7(dd,1H), 8.0(d,1H), 12.7(bs,1H); mass spectrum (chemical ionisation, ammonia): 416(M+H)⁺; microanalysis, found: C,72.0; H,5.7; N,10.1%; C₂₅H₂₅N₃O₃ requires: C,72.3; H,6.0; N,10.1%.

### (Example 19):

4'-[(2-butyl-3H-imidazo[4,5-b]pyrid3-yl)methyl]biphenyl-2-carboxylic acid was obtained as a solid, m.p. 184-186°C; NMR (d₆-DMSO): 0.85(t,3H), 1.35(m,2H), 1.7(m,2H), 2.85(t,2H), 5.6(s,2H), 7.1-7.65(complex m,8H), 7.65(dd,1H), 8.0(dd,1H), 8.3(dd,1H); mass spectrum (-ve FAB, DMSO/glycerol): 384(M-H)⁻; microanalysis, found: C,74.6; H,6.3; N,10.7%; C₂₄O₂₃N₃O₂ requires: C,74.8; H,6.0; N,10.9%.

### (Example 20):

4'-[(2-butyl-3H-imidazo[4,5-c]pyrid3-yl)methyl]biphenyl-2-carboxylic acid was obtained as a solid, m.p. 203-205°C(phase change at 100°C); NMR (d₆-DMSO): 0.9(t,3H), 1.4(m,2H), 1.75(m,2H), 2.93(t,2H), 5.65(s,2H), 7.15(d,2H), 7.2(d,2H), 7.25-7.65(complex m,6H), 7.7(d,2H), 8.3(d,1H), 8.85(s,1H); mass spectrum (-ve FAB, DMSO/glycerol): 384(M-H)⁻; microanalysis, found: C,71.5; H,6.0; N,10.2%; C₂₄H₂₃N₃O₂, 1H₂O requires: C,71.44; H,6.25; N,10.42%.

The necessary starting materials for Examples 17-20, corresponding to compound E in Example 4, part (iii), were obtained (in yields of 55-82%) by reduction of the appropriate nitroamine corresponding to compound F in Example 4, part (i) using an analogous procedure to that described in Example 4, part (ii), followed by cyclisation using a similar procedure to that described for the cyclisation of 2,3-diaminopyrimidine in Examples 7A and 8A, as follows:-

### (Example 17E):

Reduction and cyclisation of methyl 4'-N-(4-methyl-3-nitropyrid-2-yl)aminomethylbiphenyl-2-carboxylate gave methyl 4'-[(2-butyl-7-methyl-3H-imidazo[4,5-b]pyrid-3-yl)methyl]biphenyl-2-carboxylate as a gum; NMR (CDCl₃): 0.9(t,3H), 1.4(m,2H), 1.7(m,2h), 2.7(s,3H), 2.9(t,2H), 3.6(s,3H), 5.5(s,2H), 7.0(d,1H), 7.1-7.3(m,5H), 7.5(m,2H), 7.8(dd,1H), 8.2(d,1H): mass spectrum (chemical ionisation, ammonia): 414(M+H)⁺;

### (Example 18E):

Reduction and cyclisation of methyl 4'-N-(6-methoxy-3-nitropyrid-2-yl)aminomethylbiphenyl-2-carboxylate gave methyl 4'-[(2-butyl-5-methoxy-3H-imidazo[4,5-b]pyrid-3-yl)methyl]biphenyl-2-carboxylate as a solid, m,p. 90-93°C; NMR (CDCl₃): 0.9(t,3H), 1.4(m,2H), 1.7(m,2H), 2.8(t,2H), 3.6(s,3H), 4.0(s,3H), 5.4(s,2H), 6.7(d,1H), 7.2-7.55(complex m,7H), 7.8(dd,1H), 7.9(d,1H); mass spectrum (chemical ionisation, ammonia): 430(M+H)⁺;

### (Example 19E):

Reduction and cyclisation of methyl 4'-N-(3-nitropyrid-2-yl)aminomethylbiphenyl-2-carboxylate gave methyl 4'-[(2-butyl-3H-imidazo[4,5-b]pyrid-3-yl)methyl]biphenyl-2-carboxylate as a gum; NMR (CDCl₃): 0.95(t,3H), 1.4(m,2H), 1.8(m,2H), 2.85(t,2H), 3.6(s,3H), 5.55(s,2H), 7.1-7.6(complex m,8H), 7.7(dd,1H), 8.05(dd,1H), 8.35(dd,1H); mass spectrum (chemical ionisation, ammonia): 400(M+H)⁺; and

### (Example 20E):

Reduction and cyclisation of methyl-4'-N-(4-nitro-1-oxidopyrid-3-yl)aminomethylbiphenyl-2-carboxylate gave methyl 4'-[(2-butyl-3H-imidazo[4,5-c]pyrid-3-yl)methyl]biphenyl-2-carboxylate as a gum; NMR (CDCl₃): 0.95(t,3H), 1.45(m,2H), 1.90(m,2H), 2.95(t,2H), 3.65(s,3H), 5.45(s,2H), 7.13(d,2H), 7.2-7.6(complex m,5H), 7.65(d,1H), 7.85(dd,1H), 8.43(d,1H), 8.65(s,1H); mass spectrum (chemical ionisation, ammonia): 400(M+H)⁺.

The necessary starting materials for Examples 17E-20E, analogous to compound F in Example 4, part (i), were obtained in yields of 32-98% as follows:-

### (Example 17F):

A mixture of 2-chloro-4-methyl-3-nitropyridine (1.06 g), methyl 4'-(aminomethyl)biphenyl-2-carboxylate (1.44 g) and potassium carbonate (0.86 g) in DMF (14 ml) was heated at 70°C for 3 hours. The mixture was filtered, solvent removed by evaporation and the residue purified by flash chromatography, eluting with ether, to give methyl 4'-N-(4-methyl-3-nitropyrid-2-yl)aminomethylbiphenyl-2-carboxylate as a yellow gum (1.1 g); NMR (CDCl₃): 2.6(s,3H), 3.6(s,3H), 4.8(d,2H), 6.5(d,1H) 7.2-7.6(complex m,7H), 8.2(d,1H); mass spectrum (chemical ionisation, ammonia): 378(M+H)⁺;

### (Example 18F):

2-chloro-6-methoxy-3-nitropyridine (obtained as described in German Patent No. 3308499; Chem Abs., 1984,101,23354) and methyl 4'-(aminomethyl)biphenyl-2-carboxylatewere reacted, using a similar procedure to that described in Example 17F, to give methyl 4'-N-(6-methoxy-3-nitropyrid-2-yl)aminomethylbiphenyl-2-carboxylate as a solid, m.p. 148-149°C; NMR (d₆-DMSO): 3.6(s,3H), 3.8(s,3H), 4.8(d,2H), 6.2(d,1H), 7.2(m,2H), 7.4-7.7(m,6H), 8.3(d,1H), 9.6(bt,1H); mass spectrum (chemical ionisation, ammonia): 394(M+H)⁺.

### (Example 19F):

2-chloro-3-nitropyridine and methyl 4'-(aminomethyl)biphenyl-2-carboxylate were reacted, using a similar procedure to that described in Example 17F, to give methyl 4'-N-(3-nitropyrid-2-yl)aminomethylbiphenyl-2-carboxylate as a yellow solid, m.p. 112-116°C NMR (CDCl₃): 3.65(s,3H), 4.95(d,2H), 6.7(m,1H), 7.2-7.6(complex m,7H), 7.85(dd,1H), 8.45(m,1H), 8.55(bs,1H); mass spectrum (chemical ionisation, ammonia): 363(M+NH₄-H₂O)⁺, 331(M-CH₃OH)⁺; and

### (Example 20F):

3-fluoro-4-nitropyridine-1-oxide (obtained as described in Rocz. Chem., 1964, 38, 777; Chem. Abs. 1964, 61, 10653) and methyl 4'-(aminomethyl)biphenyl-2-carboxylate were reacted together, using a similar procedure to that described in Example 17F, except that the reaction was carried out at ambient temperature. The reaction mixture was then diluted with excess water and filtered to give methyl 4'-N-(4-nitro-1-oxidopyrid-3-yl)aminomethylbiphenyl-2-carboxylate as a yellow solid, m.p. 186-189°C; NMR (d₆-DMSO): 3.55(s,3H), 4.7(d,2H), 7.28(d,2H), 7.35-7.8(complex m,7H), 7.95(d,1H), 8.05(d,1H), 8.79(t,1H); mass spectrum (chemical ionisation, ammonia): 384(M+H)⁺, 364, 330.

Methyl 4'-(aminomethyl)biphenyl-2-carboxylate used in Examples 17F-2°F was prepared as follows:-
A solution of methyl 4'-(bromomethyl)biphenyl-2-carboxylate (0.763 g) in methanol (19 ml) was added to a cooled (ice bath), saturated solution of ammonia in methanol (38 ml) over 15 minutes. The mixture was warmed to ambient temperature and was stirred for a further 30 minutes. Solvent was removed by evaporation and the residue was dissolved in 0.1 M hydrochloric acid (25 ml). The acidic solution was extracted with dichloromethane (4 x 25 ml) and the aqueous phase separated and basified to pH 9 with potassium carbonate. The aqueous phase was extracted with ether and the combined ether extracts dried (Na₂SO₄) and evaporated to give methyl 4'-(aminomethyl)biphenyl-2-carboxylate, as a gum; NMR (CDCl₃): 3.65(s, 3H), 3.95(bs,2H), 7.2-7.45(complex m,6H), 7.5(dt,1H), 7.8(dd,1H); mass spectrum (chemical ionisation, ammonia): 242(M+H)⁺.

### Example 21

Using an analogous procedure to that described in Example 1, methyl 2-bromo-4-[(2-butyl-3H-imidazo[4.5-b]pyrid-3-yl)methyl]benzoate (A) was hydrolysed to give 2-bromo-4-[(2-butyl-3H-imidazo[4,5-b]pyrid-3-yl)methyl]benzoic acid as a solid, m.p. 175-176°C; NMR (d₆-DMSO): 0.86(t,3H), 1.36(m,2H), 1.68(m,2H), 2.84(t,2H), 5.57(s,2H), 7.15(dd,1H), 7.27(dd,1H), 7.55(d,1H), 7.69(d,1H), 8.01(dd,1H), 8.30(dd,1H), 13.0-13.5(bs,1H); mass spectrum (chemical ionisation, ammonia): 388(M+H)⁺, 345, 267, 176; microanalysis found: C,55.4; 0,4.7; N,10.7%; C₁₈H₁₈N₃BrO₂ requires: C,55.7; H,4.6; N,10.8%.

The starting material (A) was obtained, in 10% yield, by an analogous procedure to that described in Example 1, part (iii) but starting from 2-butylimidazo[4,5-b]pyridine and methyl 2-bromo-4-(bromomethyl)benzoate (B), as an oil; NMR (CDCl₃): 0.94(t,3H), 1.44(m,2H), 1.83(m,2H), 2.80(t,3H), 3.91(s,3H), 5.50(s,2H), 7.08(dd,1H), 7.25(dd,1H), 7.48(d,1H), 7.74(d,1H), 8.04(dd,1H), 8.35(dd,1H).

The starting material (B) was obtained, using an analogous procedure to that described in Example 1, part (ii) but starting from methyl 2-bromo-4-methylbenzoate, as an oil; NMR (CDCl₃): 3.94(s,3H), 4.41(s,2H), 7.37(dd,1H), 7.69(d,1H), 7.76(d,1H).

### Example 22

Using an analogous procedure to that described in Example 5,4-[(2-butyl-3H-imidazo[4,5-b]pyrid-3-yl)methyl]benzoic acid and (2-methylbenzene)sulphonamide were reacted to give 4-[(2-butyl-3H-imidazo[4,5-b]pyrid-3-yl)methyl]-N-(2-methylphenylsulphonyl)benzamide as a solid, m.p. 257-259°C; NMR (d₆-DMSO): 0.83(t,3H), 1.33(m,2H), 1.67(m,2H), 2.59(s,3H), 2.80(t,3H), 5.58(s,2H), 7.2-7.5(complex m,5H), 7.56(dt,1H), 7.83(d,2H), 8.0(m,2H), 8.27(dd,1H); mass spectrum (chemical ionisation, ammonia): 309(M+H-C₇H₇SO₂)⁺; microanalysis found: C,65.1; H,5.3; N,12.0%; C₂₅H₂₆N₄O₃S requires: C,64.9; H,5.6; N,12.1%.

### Example 23

Using an analogous procedure to that described in Example 1, methyl 4'-[(2-butyl-1-H-imidazo[4,5-b]pyrazin-1-yl)methyl]biphenyl-2-carboxylate (A) was hydrolysed to give 4'-[(2-butyl-1H-imidazo[4,5-b]pyrazin-1-yl)methyl]biphenyl-2-carboxylic acid as a solid, m.p. 201-202°C; NMR (d₆-DMSO): 0.9(t,3H), 1.4(m,2H), 1.7(m,2H), 2.9(t,2H), 5.6(s,2H), 7.2-7.4(m,5H), 7.5(m,2H), 7.7(dd,1H), 8.3(d,1H), 8.5(d,1H), 12.6(bs,1H); mass spectrum (-ve FAB, DMSO/glycerol): 385(M-H)⁻ microanalysis found: C,71.2; H,5.9; N,14.5%; C₂₃H₂₂N₄O₂ requires: C,71.5; H,5.7; N,14.5%.

The starting material (A) was obtained as follows:-
A solution of methyl 4'-[(2-butyl-5-bromo-1H-imidazo[4,5-b]pyrazin-1-yl)methyl]biphenyl-2-carboxylate (obtained as in Example 6A) (0.330 g) in methanol (20 ml) was catalytically hydrogenated over 5% palladium on charcoal (20 mg), in the presence of sodium methoxide (26 mg), for 16 hours. The catalyst was removed by filtration through diatomaceous earth, and the filtrate evaporated. The residue was triturated with hexane to give methyl 4'-[(2-bytyl-1-H-imidazo[4,5-b]pyrazin-1-yl)methyl]biphenyl-2-carboxylate (A) (74 mg) as a solid, m.p. 74-78°C; NMR (CDCl₃):0.9(t,3H), 1.4(m,2H), 1.9(m,2H), 2.9(t,2H), 3.6(s,38), 5.5(s,2H), 7.1-7.3(m,5H), 7.4(m,2H), 7.8(dd,1H), 7.8(dd,1H), 8.3(d,1H); mass spectrum (chemical ionisation, ammonia): 401(M+H)⁺.

### Example 24

Using an analogous procedure to that described in Example 1, methyl 4'-[(2butyl-1H-imidazo[4,5-d]pyridazin-1-yl)methyl]biphenyl-2-carboxylate (A) was hydrolysed to give 4'-[(2-butyl-1H-imidazo[4,5-d]pyrazin-1-yl]biphenyl-2-carboxylic acid as a solid, m.p. 185-186°C; NMR (d₆-DMSO): 0.9(t,3H), 1.35(m,2H), 1.75(m,2H), 2.95(d,2H), 5.7(s,2H), 7.2(d,2H), 7.3-7.35(m,3H), 7.42(dt,1H), 7.55(dt,1H), 7.7(dd,1H), 9.5(d,1H), 9.6(d,1H); mass spectrum (chemical ionisation, ammonia): 387(M+H)⁺; microanalysis found: C,71.5; B,5.7; N,14.5%; C₂₃H₂₂N₄O₂ requires: C,71.1; H,5.7; N,14.3%.

The starting material (A) was prepared as follows:-
(i) Phosphorus pentasulphide (5.1 g) was added to a solution of methyl 4'-[(2-butyl-4-hydroxy-3H-imidazo[4,5-d]pyridazin-3-yl)methyl] biphenyl-2-carboxylate (obtained as in Example 9A) (1.4 g) in pyridine (75 ml) and the mixture was heated under reflux for 24 hours. The mixture was cooled to ambient temperature and volatile material removed by evaporation. Water (200 ml) was added and the mixture heated at reflux for 1 hour. The solution was extracted with ethyl acetate (2 x 150 ml) and the combined extracts washed successively with 2 M hydrochloric acid (2 x 100 ml), water (2 x 150 ml), saturated brine (150 ml), and then dried (MgSO₄). Volatile material was removed by evaporation and the residue purified by flash chromatography eluting with ethyl acetate to give 4'-[(2-butyl-4-mercapto-3H-imidazo[4,5-d]pyridazin-3-yl)methyl]biphenyl-2-carboxylate (B) (1.1 g) as a solid, m.p. 149-150°C; NMR (d₆-DMSO): 0.8(t,3H), 1.3(m,2H), 1.6(m,2H), 2.8(t,2H), 3.55(s,3H), 6.45(s,2H), 7.12-7.3(q,4H), 7.4(d,1H), 7.5(dt,1H), 7.6(dt,1H), 7.7(dd,1H), 8.8(s,1H), 14.3(s,1H); mass spectrum (chemical ionisation, ammonia): 432(M+H)⁺.
(ii) A solution of the thiol (B) (1.0 g) in ethanol (100 ml) containing Raney nickel (7 g) was heated at reflux for 4 hours. The mixture was filtered through diatomaceous earth, the filtrate evaporated and the residue azeotroped with toluene to remove water. The residue was purified by flash chromatography eluting with methanol/dichloromethane (5:95 v/v) to give methyl 4'-[(2-butyl-1H-imidazo[4,5-d]pyridazin-1-yl]biphenyl-2-carboxylate (A) (0.38 g) as a solid, m.p. 118-120°C; NMR (CDCl₃): 1.0(t,3H), 1.45(m,2H), 1.9(m,2H) 2.95(t,2H), 3.65(s,3H), 5.45(s,2H), 7.1(d,2H), 7.25-7.35(m,3H), 7.5(m,2H), 7.85(dd,1H), 9.2(d,1H), 9.6(d,1H); mass spectrum (chemical ionisation, ammonia): 401(M+H)⁺.

### Example 25

Sodium metal (0.67 9) was added to a stirred suspension of 2-butyl-7-chloro-3-[2'-(2-triphenylmethyl-2H-tetrazol-5-yl)biphenyl-4-yl)methyl]-3H-imidazo[4,5-b]pyridine (obtained as in Example 15D; 1.0g) in methanol (100 ml) under an atmosphere of argon. The mixture was heated at reflux for 16 hours and then cooled to ambient temperature. Further sodium metal (2.6 g) was added and the mixture heated at reflux for a further 72 hours. The mixture was evaporated and the residue partitioned between dilute citric acid solution (final pH = 7) and ethyl acetate. The organic phase was worked up using a similar procedure to that described in Example 1, part (iii). The crude product was dissolved in ethyl acetate and ethereal hydrogen chloride solution added. The precipitated solid was collected by filtration to give 2-butyl-7-methoxy-3-[(2'-(1H-tetrazol-5-yl)biphenyl-4-yl)methyl]-3H-imidazo[4,5-b]pyridine hydrochloride (0.43 g) as a solid, m. p. 130-170°C (decomposition); NMR (d₆-DMSO): 0.8(t,3H), 1.3(m,2H), 1.6(m,2H): 3.0(t,2H), 4.1(s,3H), 5.6(s,2H), 7.0-7.2(m,5H), 7.45-7.7(m,4H), 8.4(d,1 H); mass spectrum (-ve FAB, DMSO/glycerol): 438(M-H)⁻; microanalysis found: C,62.5; H,5.7; N,19.7%; C₂₅H₂₅N₇O,1HCl,0.25CH₃CO₂C₂H₅ requires: C,62.7; H,5.6; N,19.7%.

### Example 26

Sodium hydride (50% dispersion in oil; 0.24g) was added to a cooled, stirred solution of ethanethiol (0.42 ml) in 1,3-dimethyl-3,4,5,6-tetrahydro-2(1H)-pyrimidinone (DMPU) (14 ml) under an argon atmosphere. Methyl 4'-[(2-butyl-5-methoxy-3H-imidazo[4,5-b]pyrid-3-yl)methyl]biphenyl-2-carboxylate (obtained as in Example 18E; 0.25 g) was added and mixture heated at 110°C for 1.5 hours. The mixture was partitioned between dilute acetic acid (final pH = 5) and ethyl acetate. The organic phase was worked up using a similar procedure to that described in Example 1, part (iii) and the crude product partially purified by flash chromatography, eluting with methanol/dichloromethane (5:95 v/v). The resultant product was dissolved in sodium carbonate solution and repeatedly washed with ethyl acetate and then dichloromethane to remove residual DMPU. The aqueous phase was separated, acidified with acetic acid and extracted with ethyl acetate. The organic phase was worked up using a similar procedure to that described in Example 1, part (iii) to give, after trituration with ether, 4'-[(2-butyl-5-hydroxy-3H-imidazo[4,5-b]pyrid-3-yl)methyl]biphenyl-2-carboxylic acid as a solid, m.p. 255-257°C; NMR (d₆-DMSO): 0.8(t,3H), 1.3(m,2H), 1.7(m,2H), 2.7(t,2H), 5.4(s,2H), 6.5(d,1H), 7.2(dd,4H), 7.3-7.6(m,3H), 7.7(d,1H), 7.8(d,1H), 10.7(bs,1H), 12.5(bs,1H); mass spectrum (chemical ionisation, ammonia): 402(M+H)⁺; microanalysis found: C,71.1; H,5.6; N,10.3%; C₂₄O₂₃N₃O₃,0.25H₂O requires: C,71.0; H,5.8; N,10.4%.

### Examples 27-29

Using an analogous procedure to that described in Example 3, but starting from the appropriate compound of formula III wherein L is triphenylmethyl, the following compounds were prepared in yields of 50-92%:-

### (Example 27):

2-butyl-6-(methoxycarbonyl)-3-[(2'-1H-tetrazol-5-yl)biphenyl-4-yl)methyl-3H-imidazo[4,5-b]pyridine was obtained as a solid; NMR (d₆-DMSO): 0.9(t,3H), 1.3-1.5(m,2H), 1.6-1.8(m,2H), 2.9(t,2H), 3.9(s,3H), 5.6(s,2H), 7.0-7.2(m,4H), 6.95-7.2(m,4H), 8.5(d,1H), 8.95(d,1H); mass spectrum (-ve FAB, DMSO/glycerol): 466(M-H)⁻; microanalysis, found: C,62.0; H,5.1; N,19.4%; C₂₆H₂₅N₇O₂,HCl requires: C,62.0; H,5.2; N,19.5%;

### (Example 28):

2-butyl-4-hydroxy-3-[(2'-(1H-tetrazol-5-yl)biphenyl-4-yl)methyl-3H-imidazo[4,5-c]pyridine was obtained as a solid, m.p. 234-237°C; NMR (d₆-DMSO): 0.85(t,3H), 1.2-1.4(m,2H), 1.5-1.68(m,2H), 2.9(t,2H), 5.95(s,2H), 4.0-6.0(bs,2H), 6.65(d,1H), 7.05-7.25(dd,4H), 7.35(t,1H), 7.45-7.75(m,4H), 11.8-11.95(bs,1H); mass spectrum (-ve FAB, DMSO/glycerol): 424(M-H)⁻; microanalysis, found: C,62.4; H,5.2; N,21.2%; C₂₄B₂₃N₇O,HCl requires: C,62.4; H,5.1; N,21.2%; and

### (Example 29):

8-butyl-9-[(2'-(1H-tetrazol-5-yl)biphenyl-4-yl)methyl]-9H-purine was obtained as a solid; NMR (d₆-DMSO): 0.85(t,3H), 1.35(m,2H), 1.7(m,2H), 2.9(t,2H), 5.6(s,1H), 7.05(d,2H), 7.15(d,2H), 7.4-7.8(m,4H), 9.15(s,1H), 9.35(s,1H); mass spectrum (+ve FAB, DMSO/glycerol): 411(M+H)⁺, accurate mass determination: 411.2035; C₂₃H₂₃N₈ requires 411.2046.

The necessary starting materials for examples 27-29, corresponding to starting material D in Example 3, were prepared using an analogous procedure to that described in Example 3, part (i) by alkylation of the appropriate imidazole derivative of formula IV. The compounds were obtained as follows:-

### (Example 27D):

Alkylation of 2-butyl-6-(methoxycarbonyl)imidazo[4,5-b]pyridine gave 2-butyl-6-(methoxycarbonyl)-3[(2'-(triphenylmethyl-2H-tetrazol-5-yl)biphenyl-4-yl)methyl]-3H-imidazo[4,5-b]pyridine as a solid; NMR (CDCl₃): 0.9(t,3H), 1.3-1.5(m,2H), 1.7-1.85(m,2H), 2.7(t,2H), 4.0(s,3H), 5.4(s,2H), 6.9(complex m,8H), 7.1(m,2H), 7.2-7.35(complex m,10H), 7.4-7.5(m,2H), 7.9(m,1H), 8.65(d,1H), 9.05(d,1H); mass spectrum (-ve FAB, DMSO/glycerol): 466(M-trityl)⁻; the starting material 2-butyl-6(methoxycarbonyl)imidazo[4,5-b]pyridine was obtained as follows:-
(i) methyl 2-chloro-3-nitropyridine-5-carboxylate (obtained as described in J.C.S., 1951, 2590) was dissolved in a mixture of concentrated aqueous ammonia solution (5ml) and dioxan (30ml). The mixture was stirred for 2 hours and then volatile material was removed by evaporation. The residue was purified by flash chromatography eluting with dichloromethane/ethyl acetate(4:1 v/v) to give methyl 2-amino-3-nitropyridine-5-carboxylate as a solid, m.p. 195-196°C; NMR(d₆-DMSO): 3.9(s,3H), 8.7(d,1H), 8.8(d,1H); mass spectrum (chemical ionisation, ammonia): 198(M+H)⁺.
(ii) Methyl 2-amino-3-nitropyridine-5-carboxylate was reduced with tin (II) chloride, using an analogous procedure to that described in Examples 13D and 14D. The crude diamine thus obtained was cyclised directly with polyphosphoric acid and valeric acid using a similar procedure to that described in Example 6A to give 2-butyl-6(methoxycarbonyl)imidazo[4,5-b]pyridine as a solid, m.p. 182-184°C; NMR (d₆-DMSO): 0.9(t,3H), 1.3-1.5(m,25), 1.7-1.9(m,2H), 2.9(t,2H), 3.9(s,3H), 8.3(bs,1H), 8.9(bs,1H); mass spectrum (chemical ionisation, ammonia): 234(M+H)⁺.

### (Example 28D):

Alkylation of 2-butyl-4-hydroxyimidazo[4,5-c]pyridine gave 2-butyl-4-hydroxy-3-[(2'-(2-triphenylmethyl-2H-tetrazol-5-yl)biphenyl-4-yl)methyl]-3H-imidazo[4,5-c]pyridine as a solid, m.p. 158-161°C; NMR (d₆-DMSO): 0.75(t,3H), 1.1-1.25(m,2H), 1.45-1.6(m,2H), 2.55(t,2H), 5.75(s,2H), 6.55(d,1H), 6.8-6.9(complex m, 6H), 7.0-7.1(m,4H), 7.10-7.15(m,1H), 7.2-7.4(complex m,11H), 7.4-7.45(d,1H), 7.5-7.6(m,2H), 7.8(d,1H), 11.25(bs,1H); mass spectrum (-ve FAB, DMSO/glycerol): 666(M-H)⁻; the starting material 2-butyl-4-hydroxyimidazo[4,5-c]pyridine was obtained as follows:-
2-chloro-3,4-diaminopyridine (obtained as described in Chem. Pharm. Bull. Japan, 1924, 12(8), 866) was cyclised with valeric anhydride by an analogous procedure to that described in Example 7A and 8A to give crude 2-butyl-4-chloroimidazo[4,5-c]pyridine, which was heated at reflux in formic acid overnight. Volatile material was removed by evaporation and the residue recrystallised from ether/methanol to give 2-butyl-4-hydroxyimidazo[4,5-c]pyridine as a solid, m.p. 275-280°C; NMR (d₆-DMSO): 0.9(t,3H), 1.2-1.4(m,2H), 1.6-1.75(m,2H), 2.7(t,2H), 6.45(d,1H), 7.05(t,1H), 10.9-11.1(bs,1H), 12.3-13.2(bs,1H); mass spectrum (chemical ionisation, ammonia): 192(M+H)⁺; and

### (Example 29D):

Alkylation of 8-butylpurine gave a mixture of regioisomers which was separated by flash chromatography, eluting with ethyl acetate/methanol (9:1 v/v) to give 8-butyl-9-[(2'-(2-triphenylmethyl-2H-tetrazol-5-yl)biphenyl-4-yl)methyl]-9H-purine (the least polar isomer) as a solid, m.p. 142-144°C (decomposition); NMR (CDCl₃): 0.9(t,3H), 1.35(m,2H), 1.7(m,2H), 2.75(t,2H), 5.35(s,2H), 6.7-7.4(complex m, 20H), 7.5(m,2H), 7.95(m,2H), 8.95(s,1H), 9.05(s,1H); mass spectrum (-ve FAB, DMSO/glycerol): 651(M-H)⁻, 409(M-trityl)⁻.

### Example 30

1M Sodium hydroxide solution (0.9 ml) was added to a solution of 2-butyl-6-methoxycarbonyl-3-[(2'-(2-triphenylmethyl-2H-tetrazol-5-yl)biphenyl-4-yl)methyl]3H-imidazo[4,5-b]pyridine (0.6 g) in dioxan/methanol (2:5 v/v; 5 ml) and the mixture was heated at reflux for 4 hours. Volatile material was removed by evaporation and the residue was triturated with dichloromethane and 1M citric acid solution. The residue was purified by flash chromatography, eluting with acetic acid/dichloromethane/methanol(1:20:80 v/v) to give 2-butyl-6-carboxy-3-[(2'-(1H-tetrazol-5-yl)biphenyl-4--yl)methyl-3H-imidazo[4,5-b]pyridine (0.19 g) as a solid, m.p. 230-232°C; NMR (d₆-DMSO): 0.9(t,3H), 1.2-1.45(m,2H), 1.6-1.8(m,2H), 3.85(t,2H), 5.55(s,2H), 7.0-7.15(m,4H), 7.45-7.7(m,4H), 8.4(d,1H), 8.9(d,1H); mass spectrum (-ve FAB, DMSO/glycerol): 452(M-H)⁻; microanalysis, found: c,65.2; H,5.1; N,21.1%; C₂₅H₂₃N₇O₂,0.36H₂O requires: C,65.2; H,5.2; N,21.3%.

### Example 31

Lithium borohydride (0.2 g) was added to an ice-cooled solution of 2-butyl-6-(methoxycarbonyl)3-[(2'-(2-triphenylmethyl-2H-tetrazol-5-yl)biphenyl-4-yl)methyl]-3H-imidazo[4,5-b]pyridine (0.97 g) in THF (50 ml). The cooled mixture was stirred for three hours and then acidified by dropwise addition of 2M hydrochloric acid. Volatile material was removed by evaporation and the residue was partially purified by flash chromatography, eluting with dichloromethane/methanol (9:1 v/v). Purification was completed by flash chromatography eluting with acetic acid/dichloromethane/methanol (0.5:5:95 v/v), followed by recrystallisation from ethyl acetate/methanol to give 2-butyl-6-(hydroxymethyl)-3-[(2'-(1H-tetrazol-5-yl)biphenyl-4-yl)methyl]imidazo[4,5-b]pyridine (0.1 g) as a solid, m.p. 93-105°C; NMR (d₆-DMSO): 0.9(t,3H), 1.25-1.45(m,2H), 1.6-1.8(m,2H), 2.8(t.2H), 4.6(s,2H), 5.7(s,2H), 7.0-7.2(m,4H), 7.5-7.7(m,4H), 7.9(d,1H), 8.3(d,1H); mass spectrum (-ve FAB, DMSO/glycerol): 438(M-H)⁻; microanalysis, found: C,68.2; H,5.5; N, 22.3%; C₂₅H₂₅N₇O requires: C,68.3; H,5.7; N,22.3%.

### Example 32 (Note: all parts by weight)

The compounds of the invention may be administered for therapeutic or prophylactic use to warm-blooded animals such as man in the form of conventional pharmaceutical compositions, typical examples of which include the following:-

| a) **Capsule** (for oral administration) | |
|---|---|
| Active ingredient * | 20 |
| Lactose powder | 578.5 |
| Magnesium stearate | 1.5 |
| | |

| b) **Tablet** (for oral administration) | |
|---|---|
| Active ingredient * | 50 |
| Microcrystalline cellulose | 400 |
| Starch (pregelatinised) | 47.5 |
| Magnesium stearate | 2.5 |
| | |

| c) **Injectable Solution** (for intravenous administration) | |
|---|---|
| Active ingredient * | 0.05 - 1.0 |
| Propylene glycol | 5.0 |
| Polyethylene glycol (300) | 3.0 - 5.0 |
| Purified water | to 100% |
| | |

| d) **Injectable Suspension** (for intramuscular administration) | |
|---|---|
| Active ingredient * | 0.05 - 1.0 |
| Methylcellulose | 0.5 |
| Tween 80 | 0.05 |
| Benzyl alcohol | 0.9 |
| Benzalkonium chloride | 0.1 |
| Purified water | to 100% |

| | |
|---|---|
| Note: the active ingredient ^{*} may typically be an Example described hereinbefore and will conveniently be present as a pharmaceutically acceptable acid-addition salt, such as the hydrochloride salt. Tablets and capsules formulations may be coated in conventional manner in order to modify or sustain dissolution of the active ingredient. Thus, for example, they may be coated with a conventional enterically digestible coating. | |

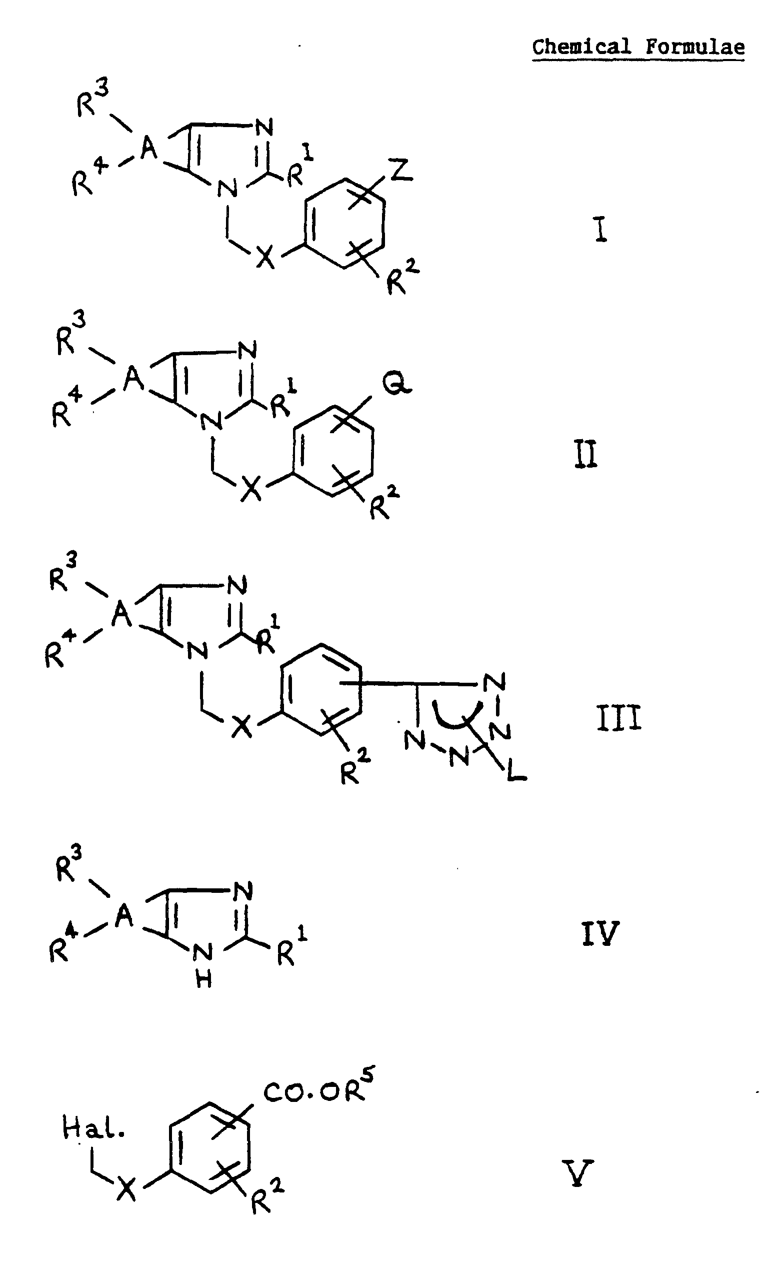

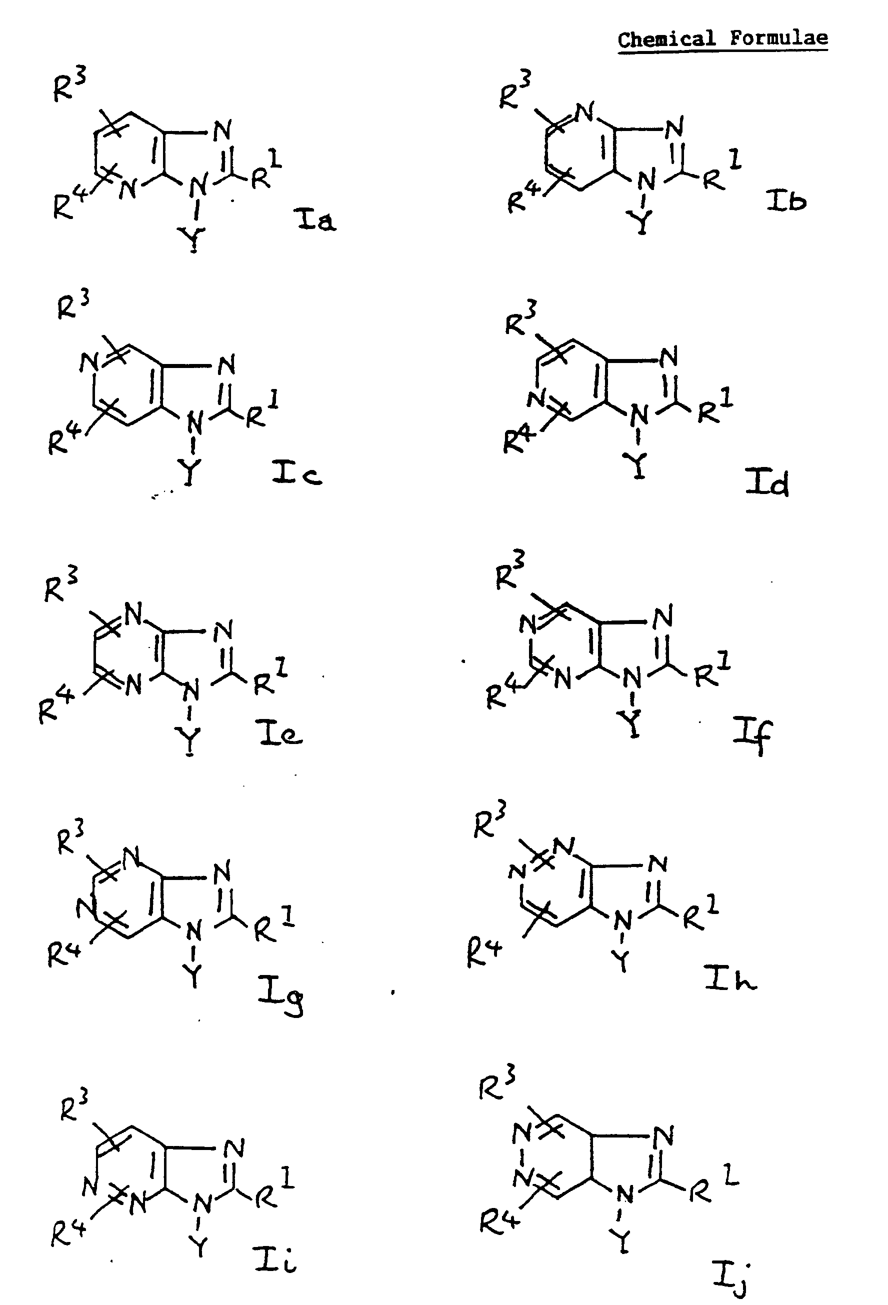

in which Y is

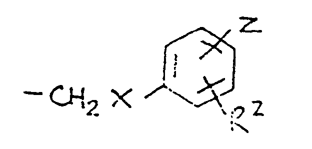

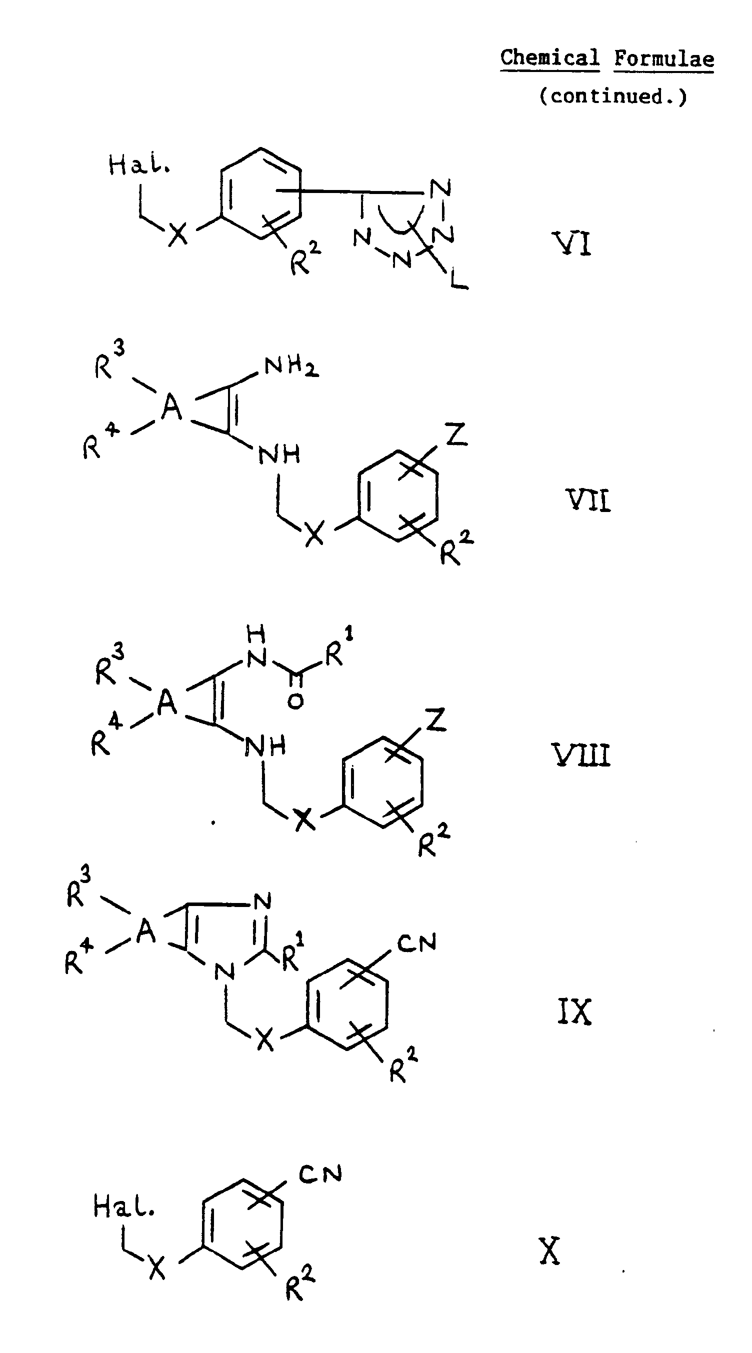

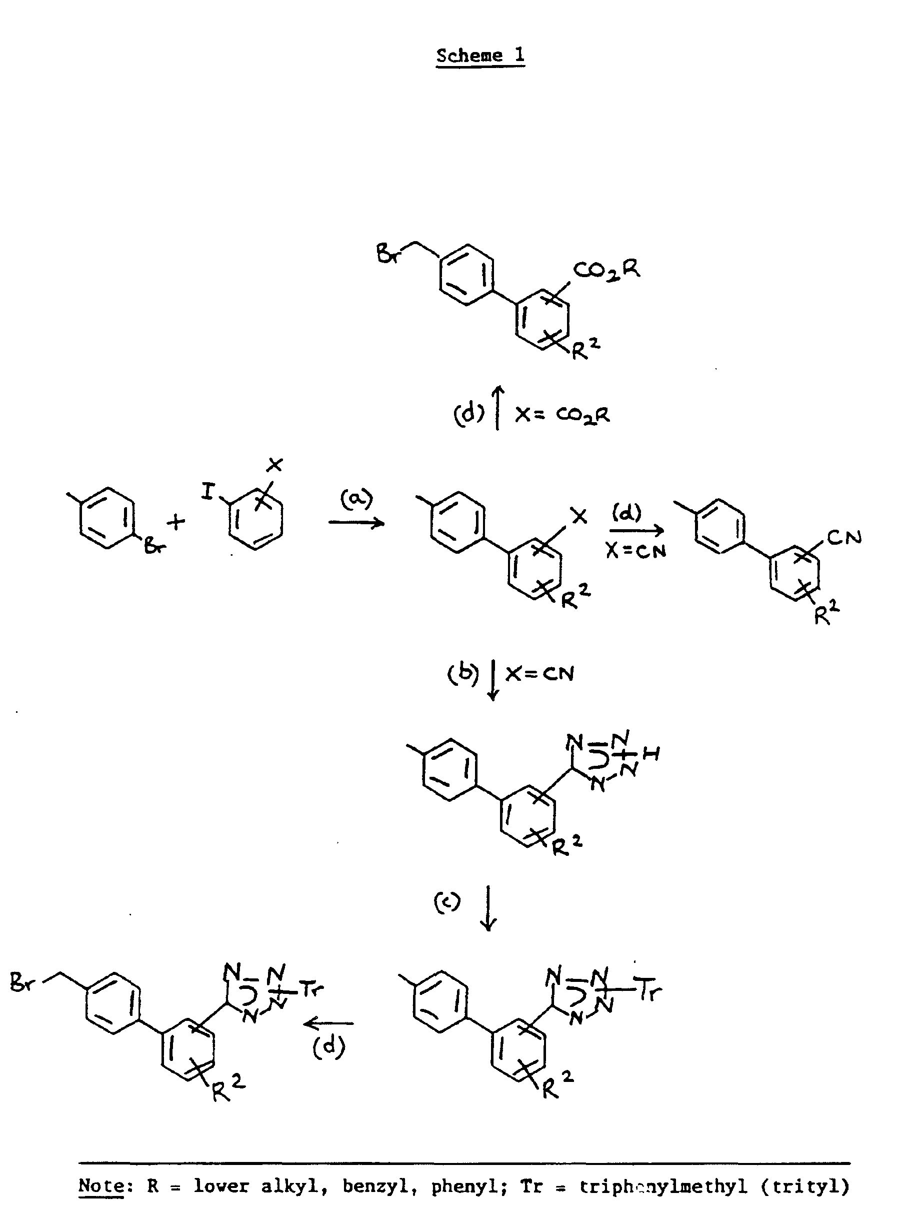

### Reagents:

a) BuLi/THF; ZnCl₂/Et₂O; Pd(Ph₃P)₄
b) Bu₃Sn.N₃/toluene; HCl/toluene
c) Tr.Cl/Et₃N/CH₂Cl₂
d) N-bromosuccinimide/azoisobutyronitrile/CCl₄

## Claims

1. An azaindene derivative of the formula I 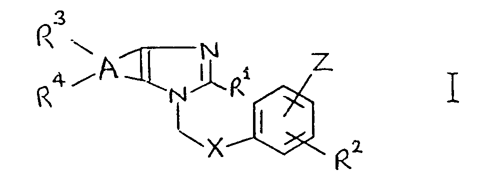 wherein A, together with the adjacent vinylene group of the imidazole moiety completes an azene ring selected from pyridine, pyrimidine, pyridazine or pyrazine ring; R¹ is (1-8C)alkyl, (3-8C)cycloalkyl, (3-8C)cycloalkyl-(1-4C)alkyl, phenyl or phenyl(1-4C)alkyl; R² is hydrogen, (1-4C)alkyl, (1-4C)alkoxy, halogeno, trifluoromethyl, cyano or nitro; R³ and R⁴ are optional substituents on the said azene ring, independently selected from hydrogen, (1-4C)alkyl, (3-8C)cycloalkyl, (1-4C)alkoxy, halogeno, trifluoromethyl, cyano, hydroxy, hydroxymethyl, formyl, and nitro; or when A together with the imidazole moiety to which it is attached is an imidazo[4,5-b]pyridine or imidazo[4,5-c]pyridine group, R³ and R⁴ when they are on adjacent carbon atoms of A form a trimethylene or tetramethylene group, or together with the adjacent vinylene group of A complete a benzene ring, the latter optionally bearing a halogeno, (1-4C)alkyl or (1-4C)alkoxy substituent; or when A together with the imidazole moiety to which it is attached is other than a 1H-imidazo[4,5-c]pyridine ring, one of R³ or R⁴ is a carboxy or (1-6C)alkoxycarbonyl group and the other is as defined above; X is phenylene optionally bearing a substituent selected from (1-4C)alkyl, (1-4C)alkoxy and halogeno, or X is a direct bond between the adjacent phenyl and methylene moieties; and Z is 1H-tetrazol-5-yl or a group of the formula **-CO.OR**^{**5**} or **-CO.NH.SO**_{**2**}**.R**^{**6**} in which R⁵ is hydrogen or a non-toxic, biodegradable residue of a physiologically acceptable alcohol or phenol, and R⁶ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or a physiologically acceptable salt thereof except when R⁵ is other than hydrogen and R³ or R⁴ is other than carboxy.

2. A compound as claimed in claim 1 wherein R¹ is methyl, ethyl, propyl, butyl, isobutyl, sec-butyl, pentyl, hexyl, cyclopropyl, cyclopentyl, cyclohexyl, cyclopropylmethyl, cyclopentylmethyl, cyclohexylmethyl, 2-cyclopentylethyl, phenyl, benzyl, 1-phenylethyl or 2-phenylethyl; R² is hydrogen, methyl, ethyl, methoxy, ethoxy, fluoro, chloro, bromo, iodo, trifluoromethyl, cyano or nitro; R³ and R⁴ are optional substituents on the said azene ring, independently selected from hydrogen, methyl, ethyl, cyclopropyl, cyclopentyl, cyclohexyl, methoxy, ethoxy, fluoro, chloro, bromo, iodo, trifluoromethyl, cyano, hydroxy, hydroxymethyl, formyl, and nitro; or when A together with the imidazole moiety to which it is attached is an imidazo[4,5-b]pyridine or imidazo[4,5-c]pyridine group, R³ and R⁴ when they are on adjacent carbon atoms of A form a trimethylene or tetramethylene group, or together with the adjacent vinylene group of A complete a benzene ring, the latter optionally bearing a fluoro, chloro, bromo, iodo, methyl, ethyl, methoxy or ethoxy substituent; or when A together with the imidazole moiety to which it is attached is other than a 1H-imidazo[4,5-c]pyridine ring, one of R³ or R⁴ is a carboxy, methoxycarbonyl, ethoxycarbonyl or propoxycarbonyl group and the other is as defined above; X is phenylene optionally bearing a substituent selected from methyl, ethyl, methoxy, ethoxy, fluoro, chloro, bromo and iodo, or X is a direct bond between the adjacent phenyl and methylene moieties; R⁵ is hydrogen or a residue derived from a (1-6C)alkanol, phenol or glycerol; and R6 is methyl, ethyl, propyl, isopropyl, butyl, pentyl, cyclobutyl, cyclopentyl, cyclohexyl or phenyl; and wherein any of said phenyl moieties may be unsubstituted or bear one or two substituents independently selected from methyl, ethyl, methoxy, ethoxy, fluoro, chloro, bromo, cyano and trifluoromethyl.

3. A compound of the formula Ia 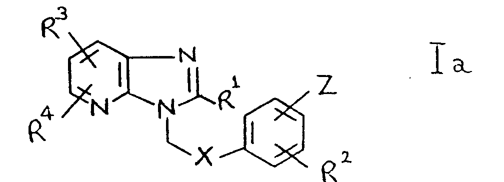 wherein R¹, R², R³, R⁴, X and Z have any of the values as defined in claim 1 or 2; or the physiologically acceptable salts thereof.

4. A compound of the formula I wherein A, R¹, R², X and Z have any of the meanings defined in claim 1; and R³ and R⁴ are optional substituents on A independently selected from hydrogen, (1-4C)alkyl, (1-4C)alkoxy, halogeno, trifluoromethyl, cyano and nitro; or a physiologically acceptable salt thereof except when R⁵ is other than hydrogen.

5. A compound as claimed in any preceding claim wherein R¹ is butyl.

6. A compound of the formula I selected from
2-butyl-3-[(2'-(1H-tetrazol-5-yl)biphenyl-4-yl)methyl]-3H-imidazo-[4,5-b]pyridine;
2-butyl-4-hydroxy-3-[(2'-(1H-tetrazol-5-yl)biphenyl-4-yl)methyl]-3H-imidazo[4,5-d]pyridazine;
2-butyl-5-methyl-3-[(2'-(1H-tetrazol-5-yl)biphenyl-4-yl)methyl]-3H-imidazo[4,5-b]pyridine;
2-butyl-7-chloro-3-[(2'-(1H-tetrazol-5-yl)biphenyl-4-ylmethyl]-3H-imidazo[4,5-b]pyridine; and
2-butyl-4-hydroxy-3-[(2'-(1H-tetrazol-5-yl)biphenyl-4-yl)methyl]-3H-imidazo[4,5-c]pyridine; and
2-butyl-6-(hydroxymethyl)-3-[(2'-(1H-tetrazol-5-yl)biphenyl-4-yl)-methyl]imidazo[4,5-b]pyridine;
and the physiologically acceptable salts thereof.

7. A salt as claimed in any one preceding claim which is selected from alkali metal, alkaline earth metal, aluminium and ammonium salts, and from salts with organic bases affording physiologically acceptable cations, and for those compounds of formula I which are basic, salts with acids affording physiologically acceptable anions.

8. A process for the manufacture of a compound of formula I or a physiologically acceptable salt thereof, as defined in claim 1 which is characterised in that:-
a) For those compounds in which Z is carboxy, a carboxylic acid derivative of the formula II 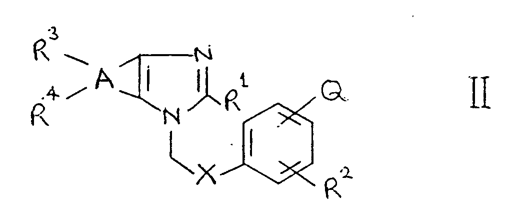 in which Q is a protected carboxy group selected from (1-6C)alkoxycarbonyl, phenoxycarbonyl, benzyloxycarbonyl and carbamoyl, is converted to carboxy;
b) For those compounds of formula I wherein Z is tetrazolyl, a compound of the formula III 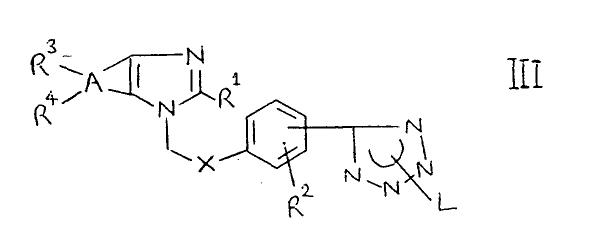 in which L is a suitable protecting group affixed to a nitrogen of the tetrazolyl moiety, is deprotected;
c) For those compounds of formula I wherein Z is a group of the formula **-CO.OR**^{**5**}**,** an imidazole derivative of the formula IV 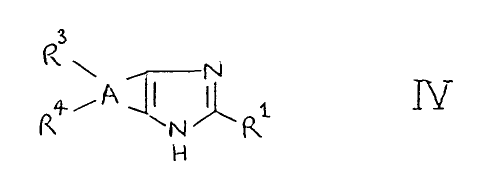 is reacted with a compound of the formula V 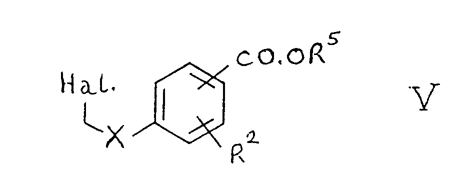 wherein **Hal.** stands for a suitable leaving group; or
d) A diamino derivative of the formula VII 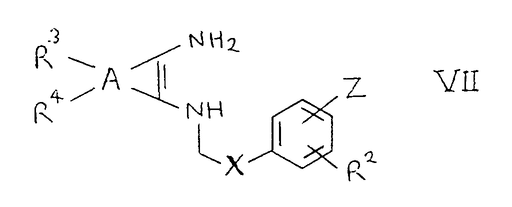 is reacted with a carboxylic acid of the formula **R**^{**1**}**.CO**_{**2**}**H** or a (1-4C)alkyl orthoester thereof;
whereafter, when a compound of the formula I is required wherein Z is a group of the formula **-CO.NH.SO**_{**2**}**R**^{**6**} or a group of the formula **-CO.OR**^{**5**} in which R⁵ is other than hydrogen, a carboxylic acid of the formula I in which Z is carboxy (or a reactive derivative of said acid) is reacted with a sulphonamide of the formula **NH**_{**2**}**.SO**_{**2**}**R**^{**6**} or a hydroxy compound of the formula **HO.R**^{**5**}**,** or with a salt thereof;
whereafter, when a salt of a compound of formula I is required, it is obtained by reaction with the appropriate base or acid affording a physiologically acceptable ion, or by any other conventional salt formation procedure; and when an optically active form of a compound of formula I is required, one of the processes (a)-(d) is carried out using an optically active starting material, or the racemic form of a compound of formula I is resolved by reaction with an optically active form of a suitable organic base or acid, followed by conventional separation of the diastereoisomeric mixture of salts thus obtained, and liberation of the required optically active form of said compound of formula I by conventional treatment with acid or base; and wherein A, R¹, R², R³, R⁴, X and Z have any of the meanings defined in any of claims 1-5 unless otherwise stated.

9. A pharmaceutical composition which comprises a compound of the formula I or Ia, or a physiologically acceptable salt thereof, as claimed in any of claims 1 to 7, together with a pharmaceutically acceptable diluent or carrier.

10. A compound of the formula III wherein A, R¹, R², R³, R⁴ and X have any of the meanings defined in any of claims 1-5, and L is a protecting group.

## Claims (Claims for the following Contracting State(s): ES)

1. A process for the manufacture of an azaindene derivative of the formula I 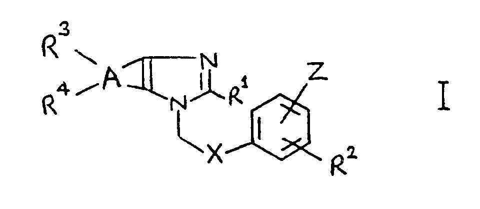 wherein A, together with the adjacent vinylene group of the imidazole moiety completes an azene ring selected from pyridine, pyrimidine, pyridazine or pyrazine ring; R¹ is (1-8C)alkyl, (3-8C)cycloalkyl, (3-8C)cycloalkyl-(1-4C)alkyl, phenyl or phenyl(1-4C)alkyl; R² is hydrogen, (1-4C)alkyl, (1-4C)alkoxy, halogeno, trifluoromethyl, cyano or nitro; R³ and R⁴ are optional substituents on the said azene ring, independently selected from hydrogen, (1-4C)alkyl, (3-8C)cycloalkyl, (1-4C)alkoxy, halogeno, trifluoromethyl, cyano, hydroxy, hydroxymethyl, formyl, and nitro; or when A together with the imidazole moiety to which it is attached is an imidazo[4,5-b]pyridine or imidazo[4,5-c]pyridine group, R³ and R⁴ when they are on adjacent carbon atoms of A form a trimethylene of tetramethylene group, or together with the adjacent vinylene group of A complete a benzene ring, the latter optionally bearing a halogeno, (1-4C)alkyl or (1-4C)alkoxy substituent; or when A together with the imidazole moiety to which it is attached is other than a 1H-imidazo[4,5-c]pyridine ring, one of R³ or R⁴ is a carboxy or (1-6C)alkoxycarbonyl group and the other is as defined above; X is phenylene optionally bearing a substituent selected from (1-4C)alkyl, (1-4C)alkoxy and halogeno, or X is a direct bond between the adjacent phenyl and methylene moieties; and Z is 1H-tetrazol-5-yl or a group of the formula **-CO.OR**^{**5**} or **-CO.NH.SO**_{**2**}**.R**^{**6**} in which R⁵ is hydrogen or a nontoxic, biodegradable residue of a physiologically acceptable alcohol or phenol, and R⁶ is (1-6C)alkyl, (3-8C)cycloalkyl or phenyl; and wherein any of said phenyl moieties may be unsubstituted or bear one of two substituents independently selected from (1-4C)alkyl, (1-4C)alkoxy, halogeno, cyano and trifluoromethyl; or a physiologically acceptable salt thereof except when R⁵ is other than hydrogen and R³ or R⁴ is other than carboxy; which is characterised by:-
a) for those compounds in which Z is carboxy, a carboxylic acid derivative of the formula II 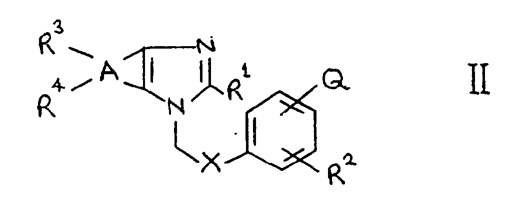 in which Q is a protected carboxy group selected from (1-6C)alkoxycarbonyl, phenoxycarbonyl, benzyloxycarbonyl and carbamoyl, is converted to carboxy;
b) for those compounds of formula I wherein Z is tetrazolyl, a compound of the formula III 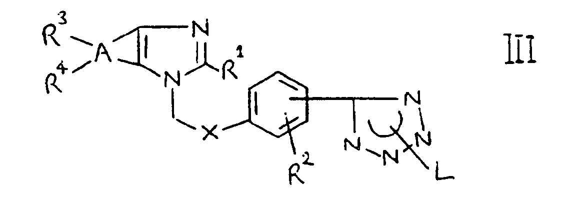 in which L is a suitable protecting group affixed to a nitrogen of the tetrazolyl moiety, is deprotected;
c) for those compounds of formula I wherein Z is a group of the formula **-CO.OR**^{**5**}, an imidazole derivative of the formula IV 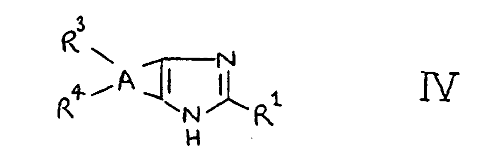 is reacted with a compound of the formula V 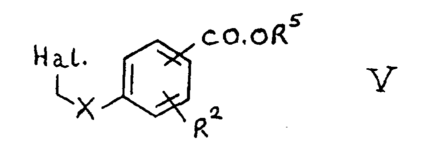 wherein **Hal.** stands for a suitable leaving group; or
d) a diamino derivative of the formula VII 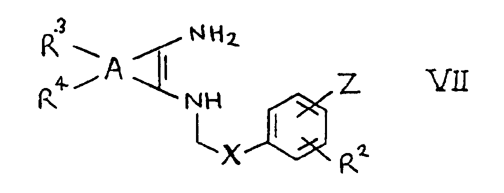 is reacted with a carboxylic acid of the formula **R**^{**1**}**.CO**_{**2**}**H** or a (1-4C)alkyl orthoester thereof;
whereafter, when a compound of the formula I is required wherein Z is a group of the formula-**CO.NH.SO**_{**2**}**R**^{**6**} or a group of the of the formula **-CO.OR**^{**5**} in which R⁵ is other than hydrogen, a carboxylic acid of the formula I in which Z is carboxy (or a reactive derivative is a said acid) is reacted with a sulphonamide of the formula **NH**_{**2**}**.SO**_{**2**}**R**^{**6**} or a hydroxy compound of the formula **HO.R**^{**5**}**,** or with a salt thereof;
whereafter, when a salt of a compound of formula I is required, it is obtained by reaction with the appropriate base or acid affording a physiologically acceptable ion, or by any other conventional salt formation procedure; and when an optically active form of a compound of formula I is required, one of the processes (a)-(d) is carried out using an optically active starting material, or the racemic form of a compound of formula I is resolved by reaction with an optically active form of a suitable organic base or acid, followed by conventional separation of the diastereoisomeric mixture of salts thus obtained, and liberation of the required optically active form of said compound of formula I by conventional treatment with acid or base; and wherein A, R¹, R², R³, R⁴, X and Z have any of the meanings defined above unless otherwise stated.

2. A process according to claim 1 wherein in the starting materials R¹ is methyl, ethyl, propyl, butyl, isobutyl, sec-butyl pentyl, hexyl, cyclopropyl, cyclopentyl, cyclohexyl, cyclopropylmethyl, cyclopentylmethyl, cyclohexylmethyl, 2-cyclopentylethyl, phenyl, benzyl, 1-phenylethyl or 2-phenylethyl; R² is hydrogen, methyl, ethyl, methoxy, ethoxy, fluoro, chloro, bromo, iodo, trifluoromethyl, cyano or nitro; R³ and R⁴ are optional substituents on the said azene ring, independently selected from hydrogen, methyl, ethyl, cyclopropyl, cyclopentyl, cyclohexyl, methoxy, ethoxy, fluoro, chloro, bromo, iodo trifluoromethyl, cyano, hydroxy, hydroxymethyl, formyl, and nitro; or when A together with the imidazole moiety to which it is attached is an imidazo[4,5-b]pyridine or imidazo[4,5-c]pyridine group, R³ and R⁴ when they are on adjacent carbon atoms of A form a trimethylene or tetramethylene group, or together with the adjacent vinylene group of A complete a benzene ring, the latter optionally bearing a fluoro, chloro, bromo, iodo, methyl, ethyl, methoxy or ethoxy substituent; or when Atogether with the imidazole moiety to which it is attached is other than a 1H-imidazo[4,5-c]pyridine ring, one of R³ or R⁴ is a carboxy, methoxycarbonyl, ethoxycarbonyl or propxycarbonyl group and the other is as defined above; X is phenylene optionally bearing a substituent selected from methyl, ethyl, methoxy, ethoxy, fluoro, chloro, bromo and iodo, orX is a direct bond between the adjacent phenyl and methylene moieties; R⁵ is hydrogen or a residue derived from a (1-6C)alkanol, phenol or glycerol; and R6 is methyl, ethyl, propyl, isopropyl, butyl, pentyl, cyclobutyl, cyclopentyl, cyclohexyl or phenyl; and wherein any of said phenyl moieties may be unsubstituted or bear one or two substituents independently selected from methyl, ethyl, methoxy, ethoxy, fluoro, chloro, bromo, cyano and trifluoromethyl.

3. The use of the compound of formula I as defined in claim 1 in the manufacture of a novel medicament.

## Claims (Claims for the following Contracting State(s): GR)

1. A process for the manufacture of an azaindene derivative of the formula I 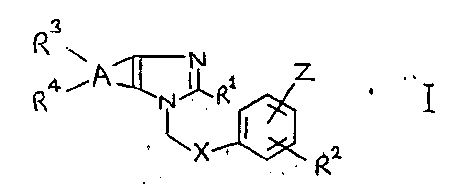 wherein A, together with the adjacent vinylene group of the imidazole moiety completes an azene ring selected from pyridine, pyrimidine, pyridazine or pyrazine ring; R¹ is (1-8C)alkyl, (3-8C)cycloalkyl, (3-8C)cycloalkyl-(1-4C)alkyl, phenyl or phenyl(1-4C)alkyl; R² is hydrogen, (1-4C)alkyl, (1-4C)alkoxy, halogeno, trifluoromethyl, cyano or nitro; R³ and R⁴ are optional substituents on the said azene ring, independently selected from hydrogen, (1-4C)alkyl, (3-8C)cycloalkyl, (1-4C)alkoxy, halogeno, trifluoromethyl, cyano, hydroxy, hydroxymethyl, formyl, and nitro; or when A together with the imidazole moiety to which it is attached is an imidazo[4,5-b]pyridine or imidazo[4,5-c]pyridine group, R³ and R⁴ when they are on adjacent carbon atoms of A form a trimethylene or tetramethylene group, or together with the adjacent vinylene group of A complete a benzene ring, the latter optionally bearing a halogeno, (1-4C)alkyl or (1-4C)alkoxy substituent; or when A together with the imidazole moiety to which it is attached is other than a 1 H-imidazo[4,5-c]pyridine ring, one of R³ or R⁴ is a carboxy or (1-6C)alkoxycarbonyl group and the other is as defined above; X is phenylene optionally bearing a substituent selected from (1-4C)alkyl, (1-4C)alkoxy and halogeno, or X is a direct bond between the adjacent phenyl and methylene moieties; and 2 is 1H-tetrazol-5-yl or a group of the formula **-CO.OR**^{**5**} or **-CO.NH.SO**_{**2**}**.R**^{**6**} in which R⁵ is hydrogen or a nontoxic, biodegradable residue of a physiologically acceptable alcohol or phenol, and R⁶ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or a physiologically acceptable salt thereof except when R⁵ is other than hydrogen and R³ or R⁴ is other than carboxy; which is characterised by:-
a) for those compounds in which Z is carboxy, a carboxylic acid derivative of the formula II 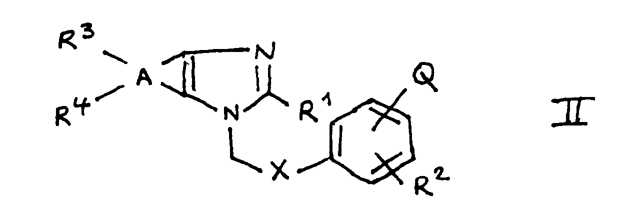 in which Q is a protected carboxy group selected from (1-6C)alkoxycarbonyl, phenoxycarbonyl, benzyloxycarbonyl and carbamoyl, is converted to carboxy;
b) for those compounds of formula I wherein Z is tetrazolyl, a compound of the formula III 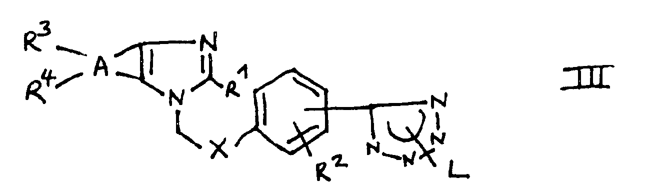 in which L is a suitable protecting group affixed to a nitrogen of the tetrazolyl moiety, is deprotected;
c) for those compounds of formula I wherein Z is a group of the formula **-CO.OR**^{**5**}**,** an imidazole derivative of the formula IV 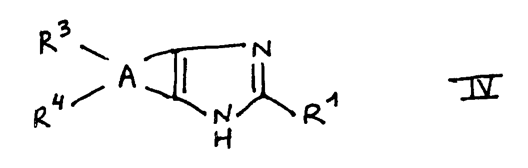 is reacted with a compound of the formula V 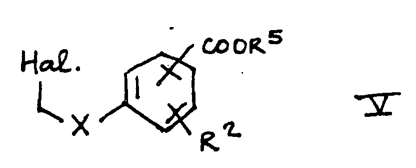 wherein **Hal.** stands for a suitable leaving group; or
d) a diamino derivative of the fomula VII 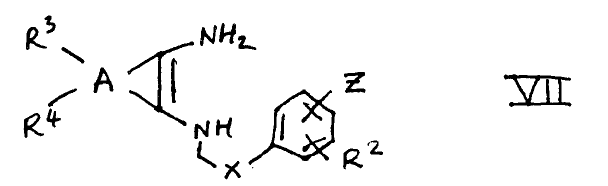 is reacted with a carboxylic acid of the formula **R**^{**1**}**.CO**_{**2**}**H** or a (1-4C)alkyl orthoester thereof;
whereafter, when a compound of the formula I is required wherein Z is a group of the formula-**CO.NH.SO**_{**2**}**R**^{**6**} or a group of the formula**-CO.OR**^{**5**} in which R⁵ is other than hydrogen, a carboxylic acid of the formula I in which Z is carboxy (or a reactive derivative of said acid) is reacted with a sulphonamide of the formula **NH**_{**2**}**.SO**_{**2**}**R**^{**6**} or a hydroxy compound of the formula **HO.R**^{**5**}**,** or with a salt thereof;
whereafter, when a salt of a compound of formula I is required, it is obtained by reaction with the appropriate base or acid affording a physiologically acceptable ion, or by any other conventional salt formation procedure; and when an optically active form of a compound of formula I is required, one of the processes (a)-(d) is carried out using an optically active starting material, or the racemic form of a compound of formula I is resolved by reaction with an optically active form of a suitable organic base or acid, followed by conventional separation of the diastereoisomeric mixture of salts thus obtained, and liberation of the required optically active form of said compound of formula I by conventional treatment with acid or base; and wherein A, R¹, R², R³, R⁴, X and Z have any of the meanings defined above unless otherwise stated.

2. A process according to claim 1 wherein in the starting materials R¹ is methyl, ethyl, propyl, butyl, isobutyl, sec-butyl, pentyl, hexyl, cyclopropyl, cyclopentyl, cyclohexyl, cyclopropylmethyl, cyclopentylmethyl, cyclohexylmethyl, 2-cyclopentylethyl, phenyl, benzyl, 1-phenylethyl or2-phenylethyl; R² is hydrogen, methyl, ethyl, methoxy, ethoxy, fluoro, chloro, bromo, iodo, trifluoromethyl, cyano or nitro; R³ and R⁴ are optional substituents on the said azene ring, independently selected from hydrogen, methyl, ethyl, cyclopropyl, cyclopentyl, cyclohexyl, methoxy, ethoxy, fluoro, chloro, bromo, iodo, trifluoromethyl, cyano, hydroxy, hydroxymethyl, formyl, and nitro; or when A together with the imidazole moiety to which it is attached is an imidazo[4,5-b]pyridene or imidazo[4,5-c]pyridene group, R³ and R⁴ when they are on adjacent carbon atom of A form a trimethylene or tetramethylene group, or together with the adjacent vinylene group of A complete a benzene ring, the latter optionally bearing a fluoro, chloro, bromo, iodo, methyl, ethyl, methoxy or ethoxy substituent; or when A together with the imidazole moiety to which it is attached is other than a 1H-imidazo[4,5-c]pyridine ring, one of R³ or R⁴ is a carboxy, methoxycarbonyl, ethoxycarbonyl or propoxycarbonyl group and the other is as defined above; X is phenylene optionally bearing a substituent selected from methyl, ethyl, methoxy, ethoxy, fluoro, chloro, bromo and iodo, or X is a direct bond between the adjacent phenyl and methylene moieties; R⁵ is hydrogen or a residue derived from a (1-6C)alkanol, phenol or glycerol; and R6 is methyl, ethyl, propyl, isopropyl, butyl, pentyl, cyclobutyl, cyclopentyl, cyclohexyl or phenyl; and wherein any of said phenyl moieties may be unsubstituted or bear one or two substituents independently selected from methyl, ethyl, methoxy, ethoxy, fluoro, chloro, bromo, cyano and trifluoromethyl.

3. A novel compound of the formula III wherein A, R¹, R², R³, R⁴, and X have any of the meanings defined in claim 1 or 2, and L is a protecting group.

4. The use of the compound of formula I as defined in claim 1 in the manufacture of a novel medicament.

## Patentansprüche

1. Azainden-Derivate der Formel I, 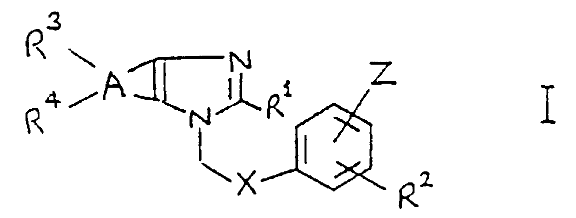 worin A zusammen mit der benachbarten Vinylen-Gruppe der Imidazol-Gruppierung einen Azen-Ring vervollständigt, der ausgewählt ist aus einem Pyridin-, Pyrimidin-, Pyridazin- oder Pyrazin-Ring; R¹ für (1-8C)Alkyl, (3-8C)Cycloalkyl, (3-8C)Cycloalkyl-(1-4C)alkyl, Phenyl oder Phenyl-(1-4C)alkyl steht; R² für Wasserstoff, (1-4C)Alkyl, (1-4C)Alkoxy, Halogeno, Trifluormethyl, Cyano oder Nitro steht; R³ und R⁴ für fakultative Substituenten am genannten Azen-Ring stehen, die unabhängig ausgewählt sind aus Wasserstoff, (1-4C)Alkyl, (3-8C)Cycloalkyl, (1-4C)Alkoxy, Halogeno, Trifluormethyl, Cyano, Hydroxy, Hydroxymethyl, Formyl und Nitro; oder wenn A zusammen mit der Imidazol-Gruppierung, an welche es gebunden ist, eine Imidazo[4,5-b]pyridin- oder Imidazo[4,5-c]pyridin-Gruppe darstellt, R³ und R⁴, sofern sie an benachbarte Kohlenstoffatome von A gebunden sind, eine Trimethylen- oder Tetramethylen-Gruppe bilden oder zusammen mit der benachbarten Vinylen-Gruppe von A einen Benzol-Ring vervollständigen, wobei letzterer gegebenenfalls einen Halogeno-, (1-4C)Alkyl- oder (1-4C)Alkoxy-Substituenten trägt; oder, wenn A zusammen mit der Imidazol-Gruppierung, an welche es gebunden ist, etwas anderes als einen 1H-Imidazo[4,5-c]pyridin-Ring darstellt, eines der Symbole R³ oder R⁴ für eine Carboxy- oder (1-6C)Alkoxycarbonyl-Gruppe steht und das andere die oben angegebene Definition besitzt; X für Phenylen steht, das gegebenenfalls einen Substituenten trägt, der ausgewählt ist aus (1-4C)Alkyl, (1-4C)Alkoxy und Halogeno oder X für eine direkte Bindung zwischen den benachbarten Phenyl- und Methylen-Gruppierungen steht; und Z für 1H-Tetrazol-5-yl oder eine Gruppe der Formel -CO.OR⁵ oder -CO.NH.SO₂.R⁶ steht, worin R⁵ für Wasserstoff oder einen nichttoxischen, biologisch abbaubaren Rest eines physiologisch unbedenklichen Alkohols oder Phenols steht und R⁶ für (1-6C)Alkyl, (3-8C)Cycloalkyl oder Phenyl steht; wobei jede der genannten Phenyl-Gruppierungen unsubstituiert sein oder einen oder zwei Substituenten tragen kann, die unabhängig ausgewählt sind aus (1-4C)Alkyl, (1-4C)Alkoxy, Halogeno, Cyano und Trifluormethyl; sowie die physiologisch unbedenklichen Salze davon, außer wenn R⁵ für etwas anderes als Wasserstoff steht und R³ oder R⁴ für etwas anderes als Carboxyl steht.

2. Verbindungen nach Anspruch 1, worin R¹ für Methyl, Ethyl, Propyl, Butyl, Isobutyl, sec-Butyl, Pentyl, Hexyl, Cyclopropyl, Cyclopentyl, Cyclohexyl, Cyclopropylmethyl, Cyclopentylmethyl, Cyclohexylmethyl, 2-Cyclopentylethyl, Phenyl, Benzyl, 1-Phenylethyl oder 2-Phenylethyl steht; R² für Wasserstoff, Methyl, Ethyl, Methoxy, Ethoxy, Fluor, Chlor, Brom, Iod, Trifluormethyl, Cyano oder Nitro steht; R³ und R⁴ für fakultative Substituenten am genannten Azen-Ring stehen, die unabhängig ausgewählt sind aus Wasserstoff, Methyl, Ethyl, Cyclopropyl, Cyclopentyl, Cyclohexyl, Methoxy, Ethoxy, Fluor, Chlor, Brom, Iod, Trifluormethyl, Cyano, Hydroxy, Hydroxymethyl, Formyl und Nitro; oder, wenn A zusammen mit der Imidazol-Gruppierung, an welche es gebunden ist, eine Imidazo[4,5-b]pyridin- oder Imidazo[4,5-c]pyridin-Gruppe darstellt, R³ und R⁴, sofern sie an benachbarte Kohlenstoffatome von A gebunden sind, eine Trimethylen- oder Tetramethylen-Gruppe bilden oder zusammen mit der benachbarten Vinylen-Gruppe von A einen Benzol-Ring vervollständigen, wobei letzterer gegebenenfalls einen Fluor-, Chlor-, Brom-, Iod-, Methyl-, Ethyl-, Methoxy- oder Ethoxy-Substituenten trägt; oder, wenn A zusammen mit der Imidazol-Gruppierung, an welche es gebunden ist, etwas anderes als einen 1H-Imidazo[4,5-c]pyridin-Ring darstellt, eines der Symbole R³ oder R⁴ für eine Carboxy-, Methoxycarbonyl-, Ethoxycarbonyl- oder Propoxycarbonyl-Gruppe steht und das andere die obige Definition besitzt; X für Phenylen steht, das gegebenenfalls einen Substituenten trägt, der ausgewählt ist aus Methyl, Ethyl, Methoxy, Ethoxy, Fluor, Chlor, Brom und Iod, oder X für eine direkte Bindung zwischen den benachbarten Phenyl- und Methylen-Gruppierungen steht; R⁵ für Wasserstoff oder einen Rest steht, der sich von einem (1-6C)Alkanol, Phenol oder Glycerin ableitet; und R⁶ für Methyl, Ethyl, Propyl, Isopropyl, Butyl, Pentyl, Cyclobutyl, Cyclopentyl, Cyclohexyl oder Phenyl steht; wobei jede der genannten Phenyl-Gruppierungen unsubstituiert sein oder einen oder zwei Substituenten tragen kann, die unabhängig ausgewählt sind aus Methyl, Ethyl, Methoxy, Ethoxy, Fluor, Chlor, Brom, Cyano und Trifluormethyl.

3. Verbindungen der Formel Ia 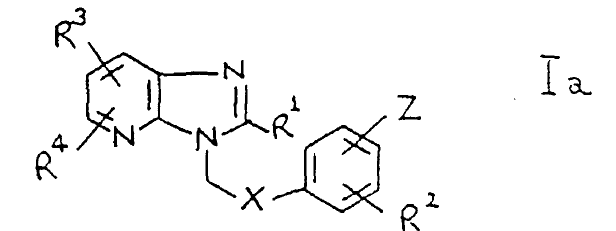 worin R¹, R², R³, R⁴, X und Z die in Anspruch 1 oder 2 angegebenen Bedeutungen besitzen; sowie die physiologisch unbedenklichen Salze davon.

4. Verbindungen der Formel I, worin A, R¹, R², X und Z die in Anspruch 1 angegebenen Bedeutungen besitzen; und R³ und R⁴ für fakultative Substituenten an A stehen, die unabhängig ausgewählt sind aus Wasserstoff, (1-4C)Alkyl, (1-4C)Alkoxy, Halogeno, Trifluormethyl, Cyano und Nitro; sowie die physiologisch unbedenklichen Salze davon, außer wenn R⁵ für etwas anderes steht als Wasserstoff.

5. Verbindungen nach einem der vorhergehenden Ansprüche, worin R¹ für Butyl steht.

6. Verbindungen der Formel I, welche ausgewählt sind aus
2-Butyl-3-[(2'-(1H-tetrazol-5-yl)biphenyl-4-yl)methyl]-3H-imidazo[4,5-b]pyridin;
2-Butyl-4-hydroxy-3-[(2'-(1H-tetrazol-5-yl)biphenyl-4-yl)methyl]-3H-imidazo[4,5-d]pyridazin;
2-Butyl-5-methyl-3-[(2'-(1H-tetrazol-5-yl)biphenyl-4-yl)methyl]-3H-imidazo[4,5-b]pyridin;
2-Butyl-7-chlor-3-[(2'-(1H-tetrazol-5-yl)biphenyl-4-yl)methyl]-3H-imidazo[4,5-b]pyridin; und
2-Butyl-4-hydroxy-3-[(2'-(1H-tetrazol-5-yl)biphenyl-4-yl)methyl]-3H-imidazo[4,5-c]pyridin; und
2-Butyl-6-(hydroxymethyl)-3-[(2'-(1H-tetrazol-5-yl)biphenyl-4-yl)methyl]imidazo[4,5-b]pyridin;
und die physiologisch unbedenklichen Salze davon.

7. Salz nach einem der vorhergehenden Ansprüche, welches ausgewählt ist aus Alkali-, Erdalkali-, Aluminium- und Ammonium-Salzen und aus Salzen mit organischen Basen, die physiologisch unbedenkliche Kationen liefern, und, für solche Verbindungen der Formel I, die basisch sind, aus Salzen mit Säuren, die physiologisch unbedenkliche Anionen liefern.

8. Verfahren zur Herstellung einer Verbindung der Formel I oder eines physiologisch unbedenklichen Salzes davon nach Anspruch, dadurch gekennzeichnet, daß
a) für solche Verbindungen, worin Z für Carboxy steht, ein Carbonsäure-Derivat der Formel II 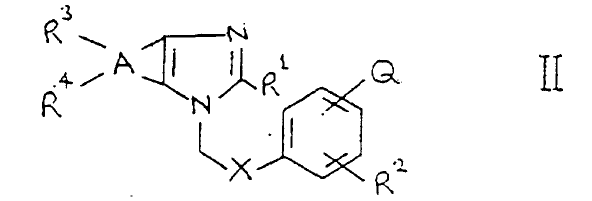 worin Q für eine geschützte Carboxy-Gruppe steht, die ausgewählt ist aus (1-6C)Alkoxycarbonyl, Phenoxycarbonyl, Benzyloxycarbonyl und Carbamoyl, in das Carboxy-Derivat überführt wird;
b) für solche Verbindungen der Formel I, worin Z für Tetrazolyl steht, eine Verbindung der Formel III 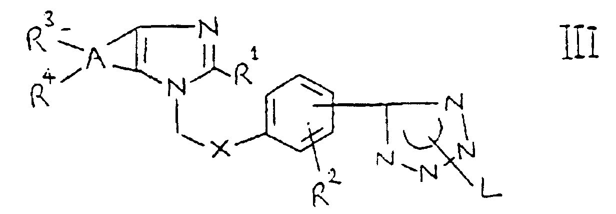 worin L für eine geeignete Schutzgruppe steht, die an einen Stickstoff der Tetrazolyl-Gruppierung gebunden ist, entschützt wird;
c) für solche Verbindungen der Formel I, worin Z für eine Gruppe der Formel -CO.OR⁵ steht, ein Imidazol-Derivat der Formel IV 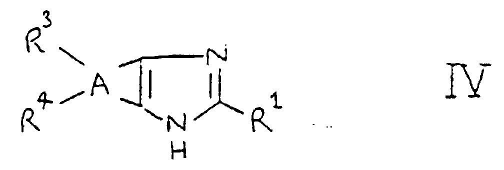 mit einer Verbindung der Formel V 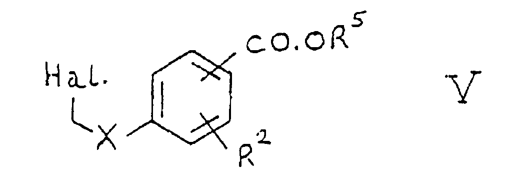 worin Hal. für eine geeignete Abgangsgruppe steht, umgesetzt wird; oder
d) ein Diamino-Derivat der Formel VII 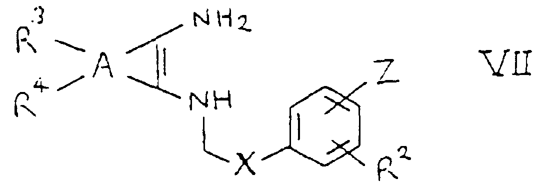 mit einer Carbonsäure der Formel R¹.CO₂H oder einem (1-4C)Alkyl-Orthoester davon umgesetzt wird;
worauf, wenn eine Verbindung der Formel I gewünscht wird, worin Z für eine Gruppe der Formel -CO.NH.SO₂R⁶ oder eine Gruppe der Formel -CO.OR⁵ steht, worin R⁵ für etwas anderes als Wasserstoff steht, eine Carbonsäure der Formel I, worin Z für Carboxy (oder ein reaktives Derivat dieser Säure) steht, mit einem Sulfonamid der Formel NH₂.SO₂R⁶ oder einer Hydroxy-Verbindung der Formel HO.R⁵ oder mit einem Salz davon umgesetzt wird;
worauf, wenn ein Salz einer Verbindung der Formel I gewünscht wird, dieses durch Umsetzung mit der entsprechenden Base oder Säure, die ein physiologisch unbedenkliches Ion liefert, oder durch ein beliebiges anderes übliches Salzbildungsverfahren erhalten wird; und, wenn eine optisch aktive Form einer Verbindung der Formel I gewünscht wird, eines der Verfahren (a)-(d) unter Verwendung eines optisch aktiven Ausgangsmaterials ausgeführt wird oder die razemische Form einer Verbindung der Formel I razematgespalten wird, indem man sie mit einer optisch aktiven Form einer geeigneten organischen Base oder Säure umsetzt, hierauf das so erhaltene diastereoisomere Salzgemisch in üblicher Weise trennt und die gewünschte optisch aktive Form der Verbindung der Formel I durch übliche Behandlung mit einer Säure oder Base freisetzt; wobei A, R¹, R², R³, R⁴, X und Z die in den Ansprüchen 1 bis 5 angegebenen Bedeutungen besitzen, sofern nichts anderes angegeben ist.

9. Pharmazeutische Zusammensetzung, welche eine Verbindung der Formel I oder Ia oder ein physiologisch unbedenkliches Salz davon nach einem der Ansprüche 1 bis 7 zusammen mit einem pharmazeutisch unbedenklichen Verdünnungsmittel oder Trägerstoff enthält.

10. Verbindungen der Formel III, worin A, R¹, R², R³, R⁴ und X die in den Ansprüchen 1-5 angegebenen Bedeutungen besitzen und L für eine Schutzgruppe steht.

## Patentansprüche (Patentansprüche für folgende(n) Vertragsstaat(en): ES)

1. Verfahren zur Herstellung eines Azainden-Derivats der Formel I 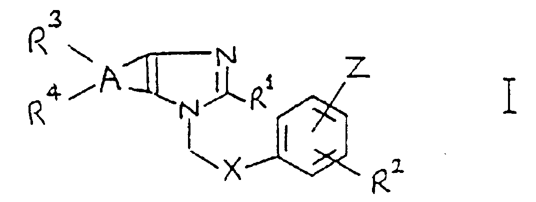 worin A zusammen mit der benachbarten Vinylen-Gruppe der Imidazol-Gruppierung einen Azen-Ring vervollständigt, der ausgewählt ist aus einem Pyridin-, Pyrimidin-, Pyridazin- oder Pyrazin-Ring; R¹ für (1-8C)Alkyl, (3-8C)Cycloalkyl, (3-8C)Cycloalkyl -(1-4-C)alkyl, Phenyl oder Phenyl-(1-4C)alkyl steht; R² für Wasserstoff, (1-4C)Alkyl, (1-4C)Alkoxy, Halogeno, Trifluormethyl, Cyano oder Nitro steht; R³ und R⁴ für fakultative Substituenten am genannten Azen-Ring stehen, die unabhängig ausgewählt sind aus Wasserstoff, (1-4C)Alkyl, (3-8C)Cycloalkyl, (1-4C)Alkoxy, Halogeno, Trifluormethyl, Cyano, Hydroxy, Hydroxymethyl, Formyl und Nitro; oder wenn A zusammen mit der Imidazol-Gruppierung, an welche es gebunden ist, eine Imidazo[4,5-b]pyridin- oder Imidazo[4,5-c]pyridin-Gruppe darstellt, R³ und R⁴, sofern sie an benachbarte Kohlenstoffatome von A gebunden sind, eine Trimethylen- oder Tetramethylen-Gruppe bilden oder zusammen mit der benachbarten Vinylen-Gruppe von A einen Benzol-Ring vervollständigen, wobei letzterer gegebenenfalls einen Halogeno-, (1-4C)Alkyl- oder (1-4C)Alkoxy-Substituenten trägt; oder, wenn A zusammen mit der Imidazol-Gruppierung, an welche es gebunden ist, etwas anderes als einen 1H-Imidazo[4,5-c]pyridin-Ring darstellt, eines der Symbole R³ oder R⁴ für eine Carboxy- oder (1-6C)Alkoxycarbonyl-Gruppe steht und das andere die oben angegebene Definition besitzt; X für Phenylen steht, das gegebenenfalls einen Substituenten trägt, der ausgewählt ist aus (1-4C)Alkyl, (1-4C)Alkoxy und Halogeno oder X für eine direkte Bindung zwischen den benachbarten Phenyl- und Methylen-Gruppierungen steht; und Z für 1H-Tetrazol-5-yl oder eine Gruppe der Formel -CO.OR⁵ oder -CO.NH.SO₂.R⁶ steht, worin R⁵ für Wasserstoff oder einen nichttoxischen, biologisch abbaubaren Rest eines physiologisch unbedenklichen Alkohols oder Phenols steht und R⁶ für (1-6C)Alkyl, (3-8C)Cycloalkyl oder Phenyl steht; wobei jede der genannten Phenyl-Gruppierungen unsubstituiert sein oder einen oder zwei Substituenten tragen kann, die unabhängig ausgewählt sind aus (1-4C)Alkyl, (1-4C)Alkoxy, Halogeno, Cyano und Trifluormethyl; sowie die physiologisch unbedenklischen Salze davon, außer wenn R⁵ für etwas anderes steht als Wasserstoff und R³ oder R⁴ für etwas anderes steht als Carboxyl; dadurch gekennzeichnet, daß
a) für solche Verbindungen, worin Z für Carboxy steht, ein Carbonsäure-Derivat der Formel II 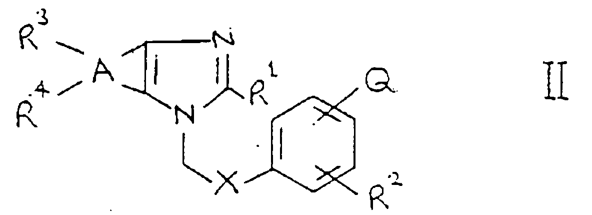 worin Q für eine geschützte Carboxy-Gruppe steht, die ausgewählt ist aus (1-6C)Alkoxycarbonyl, Phenoxycarbonyl, Benzyloxycarbonyl und Carbamoyl, in das Carboxy-Derivat überführt wird;
b) für solche Verbindungen der Formel I, worin Z für Tetrazolyl steht, eine Verbindung der Formel III 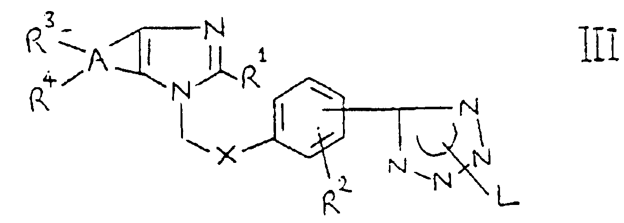 worin L für eine geeignete Schutzgruppe steht, die an einen Stickstoff der Tetrazolyl-Gruppierung gebunden ist, entschützt wird;
c) für solche Verbindungen der Formel I, worin Z für eine Gruppe der Formel -CO.OR⁵ steht, ein Imidazol-Derivat der Formel IV 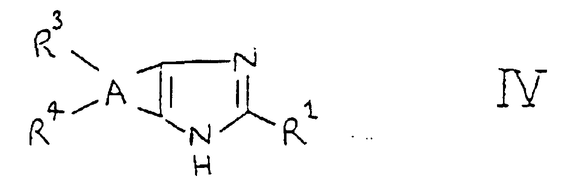 mit einer Verbindung der Formel V 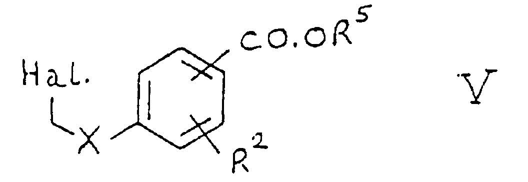 worin Hal. für eine geeignete Abgangsgruppe steht, umgesetzt wird; oder
d) ein Diamino-Derivat der Formel VII 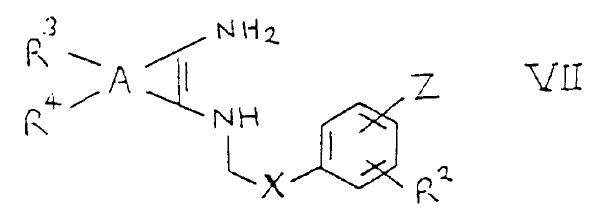 mit einer Carbonsäure der Formel R¹.CO₂H oder einem (1-4C)Alkyl-Orthoester davon umgesetzt wird;
worauf, wenn eine Verbindung der Formel I gewünscht wird, worin Z für eine Gruppe der Formel -CO.NH.SO₂R⁶ oder eine Gruppe der Formel -CO.OR⁵ steht, worin R⁵ für etwas anderes als Wasserstoff steht, eine Carbonsäure der Formel I, worin Z für Carboxy (oder ein reaktives Derivat dieser Säure) steht, mit einem Sulfonamid der Formel NH₂.SO₂R⁶ oder einer HydroxyVerbindung der Formel HO.R⁵ oder mit einem Salz davon umgesetzt wird;
worauf, wenn ein Salz einer Verbindung der Formel I gewünscht wird, dieses durch Umsetzung mit der entsprechenden Base oder Säure, die ein physiologisch unbedenkliches Ion liefert, oder durch ein beliebiges anderes übliches Salzbildungsverfahren erhalten wird; und, wenn eine optisch aktive Form einer Verbindung der Formel I gewünscht wird, eines der Verfahren (a)-(d) unter Verwendung eines optisch aktiven Ausgangsmaterials ausgeführt wird oder die razemische Form einer Verbindung der Formel I razematgespalten wird, indem man sie mit einer optisch aktiven Form einer geeigneten organischen Base oder Säure umsetzt, hierauf das so erhaltene diastereoisomere Salzgemisch in üblicher Weise trennt und die gewünschte optisch aktive Form der Verbindung der Formel I durch übliche Behandlung mit einer Säure oder Base freisetzt; wobei A, R¹, R², R³, R⁴, X und Z die oben angegebenen Bedeutungen besitzen, sofern nichts anderes angegeben ist.

2. Verfahren nach Anspruch 1, bei welchem in den Ausgangsmaterialien R¹ für Methyl, Ethyl, Propyl, Butyl, Isobutyl, sec-Butyl, Pentyl, Hexyl, Cyclopropyl, Cyclopentyl, Cyclohexyl, Cyclopropylmethyl, Cyclopentylmethyl, Cyclohexylmethyl, 2-Cyclopentylethyl, Phenyl, Benzyl, 1-Phenylethyl oder 2-Phenylethyl steht; R² für Wasserstoff, Methyl, Ethyl, Methoxy, Ethoxy, Fluor, Chlor, Brom, Iod, Trifluormethyl, Cyano oder Nitro steht; R³ und R⁴ für fakultative Substituenten am genannten Azen-Ring stehen, die unabhängig ausgewählt sind aus Wasserstoff, Methyl, Ethyl, Cyclopropyl, Cyclopentyl, Cyclohexyl, Methoxy, Ethoxy, Fluor, Chlor, Brom, Iod, Trifluormethyl, Cyano, Hydroxy, Hydroxymethyl, Formyl und Nitro; oder, wenn A zusammen mit der Imidazol-Gruppierung, an welche es gebunden ist, eine Imidazo[4,5-b]pyridin- oder Imidazo[4,5-c]pyridin-Gruppe darstellt, R³ und R⁴, sofern sie an benachbarte Kohlenstoffatome von A gebunden sind, eine Trimethylen- oder Tetramethylen-Gruppe bilden oder zusammen mit der benachbarten Vinylen-Gruppe von A einen Benzol-Ring vervollständigen, wobei letzterer gegebenenfalls einen Fluor-, Chlor-, Brom-, Iod-, Methyl-, Ethyl-, Methoxy- oder Ethoxy-Substituenten trägt; oder, wenn A zusammen mit der Imidazol-Gruppierung, an welche es gebunden ist, etwas anderes als einen 1H-Imidazo[4,5-c]pyridin-Ring darstellt, eines der Symbole R³ oder R⁴ für eine Carboxy-, Methoxycarbonyl-, Ethoxycarbonyl- oder Propoxycarbonyl-Gruppe steht und das andere die obige Definition besitzt; X für Phenylen steht, das gegebenenfalls einen Substituenten trägt, der ausgewählt ist aus Methyl, Ethyl, Methoxy, Ethoxy, Fluor, Chlor, Brom und Iod, oder X für eine direkte Bindung zwischen den benachbarten Phenyl- und Methylen-Gruppierungen steht; R⁵ für Wasserstoff oder einen Rest steht, der sich von einem (1-6C)Alkanol, Phenol oder Glycerin ableitet; und R⁶ für Methyl, Ethyl, Propyl, Isopropyl, Butyl, Pentyl, Cyclobutyl, Cyclopentyl, Cyclohexyl oder Phenyl steht; wobei jede der genannten Phenyl-Gruppierungen unsubstituiert sein oder einen oder zwei Substituenten tragen kann, die unabhängig ausgewählt sind aus Methyl, Ethyl, Methoxy, Ethoxy, Fluor, Chlor, Brom, Cyano und Trifluormethyl.

3. Verwendung einer Verbindung der Formel I nach Anspruch 1 bei der Herstellung eines neuen Medikaments.

## Patentansprüche (Patentansprüche für folgende(n) Vertragsstaat(en): GR)

1. Verfahren zur Herstellung eines Azainden-Derivats der Formel I 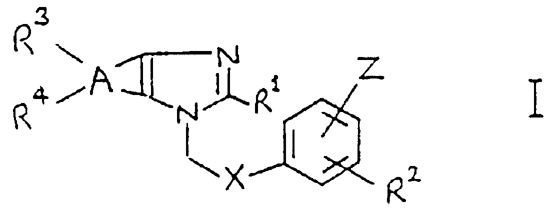 worin A zusammen mit der benachbarten Vinylen-Gruppe der Imidazol-Gruppierung einen Azen-Ring vervollständigt, der ausgewählt ist aus einem Pyridin-, Pyrimidin-, Pyridazin- oder Pyrazin-Ring; R¹ für (1-8C)Alkyl, (3-8C)Cycloalkyl-, (3-8C)Cycloalkyl-(1-4C)alkyl, Phenyl oder Phenyl-(1-4C)alkyl steht; R² für Wasserstoff, (1-4C)Alkyl, (1-4C)Alkoxy, Halogeno, Trifluormethyl, Cyano oder Nitro steht; R³ und R⁴ für fakultative Substituenten am genannten Azen-Ring stehen, die unabhängig ausgewählt sind aus Wasserstoff, (1-4C)Alkyl, (3-8C)Cycloalkyl, (1-4C)Alkoxy, Halogeno, Trifluormethyl, Cyano, Hydroxy, Hydroxymethyl, Formyl und Nitro; oder wenn A zusammen mit der Imidazol-Gruppierung, an welche es gebunden ist, eine Imidazo[4,5-b]pyridin- oder Imidazo[4,5-c]pyridin-Gruppe darstellt, R³ und R⁴, sofern sie an benachbarte Kohlenstoffatome von A gebunden sind, eine Trimethylen- oder Tetramethylen-Gruppe bilden oder zusammen mit der benachbarten Vinylen-Gruppe von A einen Benzol-Ring vervollständigen, wobei letzterer gegebenenfalls einen Halogeno-, (1-4C)Alkyl- oder (1-4C)Alkoxy-Substituenten trägt; oder, wenn A zusammen mit der Imidazol-Gruppierung, an welche es gebunden ist, etwas anderes als einen 1H-Imidazo[4,5-c]pyridin-Ring darstellt, eines der Symbole R³ oder R⁴ für eine Carboxy- oder (1-6C)Alkoxycarbonyl-Gruppe steht und das andere die oben angegebene Definition besitzt; X für Phenylen steht, das gegebenenfalls einen Substituenten trägt, der ausgewählt ist aus (1-4C)Alkyl, (1-4C)Alkoxy und Halogeno oder X für eine direkte Bindung zwischen den benachbarten Phenyl- und Methylen-Gruppierungen steht; und Z für 1H-Tetrazol-5-yl oder eine Gruppe der Formel -CO.OR⁵ oder -CO.NH.SO₂.R⁶ steht, worin R⁵ für Wasserstoff oder einen nichttoxischen, biologisch abbaubaren Rest eines physiologisch unbedenklichen Alkohols oder Phenols steht und R⁶ für (1-6C)Alkyl, (3-8C)Cycloalkyl oder Phenyl steht; wobei jede der genannten Phenyl-Gruppierungen unsubstituiert sein oder einen oder zwei Substituenten tragen kann, die unabhängig ausgewählt sind aus (1-4C)Alkyl, (1-4C)Alkoxy, Halogeno, Cyano und Trifluormethyl; sowie die physiologisch unbedenklischen Salze davon, außer wenn R⁵ für etwas anderes steht als Wasserstoff und R³ oder R⁴ für etwas anderes steht als Carboxyl; dadurch gekennzeichnet, daß
a) für solche Verbindungen, worin Z für Carboxy steht, ein Carbonsäure-Derivat der Formel II 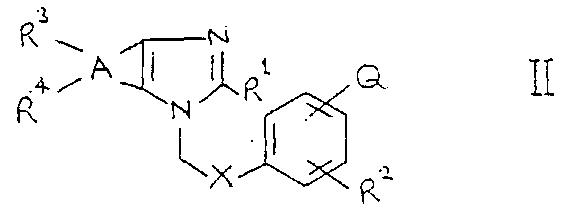 worin Q für eine geschützte Carboxy-Gruppe steht, die ausgewählt ist aus (1-6C)Alkoxycarbonyl, Phenoxycarbonyl, Benzyloxycarbonyl und Carbamoyl, in das Carboxy-Derivat überführt wird;
b) für solche Verbindungen der Formel I, worin Z für Tetrazolyl steht, eine Verbindung der Formel III 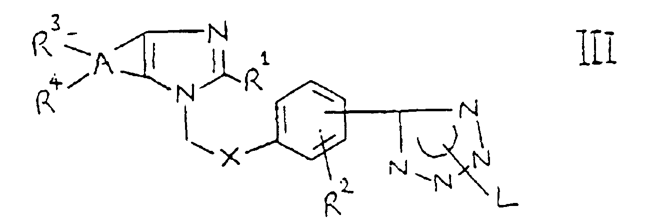 worin L für eine geeignete Schutzgruppe steht, die an einen Stickstoff der Tetrazolyl-Gruppierung gebunden ist, entschützt wird;
c) für solche Verbindungen der Formel I, worin Z für eine Gruppe der Formel -CO.OR⁵ steht, ein Imidazol-Derivat der Formel IV 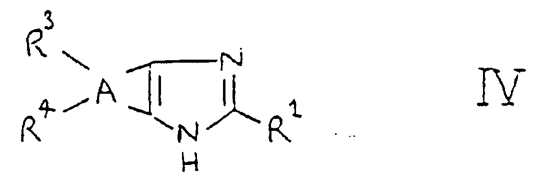 mit einer Verbindung der Formel V 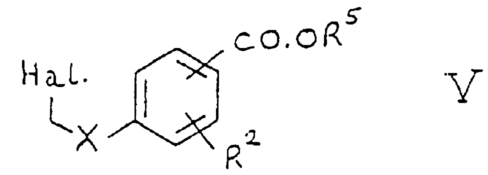 worin Hal. für eine geeignete Abgangsgruppe steht, umgesetzt wird; oder
d) ein Diamino-Derivat der Formel VII 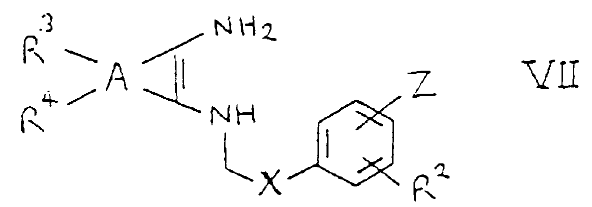 mit einer Carbonsäure der Formel R¹.CO₂H oder einem (1-4C)Alkyl-Orthoester davon umgesetzt wird;
worauf, wenn eine Verbindung der Formel I gewünscht wird, worin Z für eine Gruppe der Formel -CO.NH.SO₂R⁶ oder eine Gruppe der Formel -CO.OR⁵ steht, worin R⁵ für etwas anderes als Wasserstoff steht, eine Carbonsäure der Formel I, worin Z für Carboxy (oder ein reaktives Derivat dieser Säure) steht, mit einem Sulfonamid der Formel NH₂.SO₂R⁶ oder einer Hydroxy-Verbindung der Formel HO.R⁵ oder mit einem Salz davon umgesetzt wird;
worauf, wenn ein Salz einer Verbindung der Formel I gewünscht wird, dieses durch Umsetzung mit der entsprechenden Base oder Säure, die ein physiologisch unbedenkliches Ion liefert, oder durch ein beliebiges anderes übliches Salzbildungsverfahren erhalten wird; und, wenn eine optisch aktive Form einer Verbindung der Formel I gewünscht wird, eines der Verfahren (a)-(d) unter Verwendung eines optisch aktiven Ausgangsmaterials ausgeführt wird oder die razemische Form einer Verbindung der Formel I razematgespalten wird, indem man sie mit einer optisch aktiven Form einer geeigneten organischen Base oder Säure umsetzt, hierauf das so erhaltene diastereoisomere Salzgemisch in üblicher Weise trennt und die gewünschte optisch aktive Form der Verbindung der Formel I durch übliche Behandlung mit einer Säure oder Base freisetzt; wobei A, R¹, R², R³, R⁴, X und Z die oben angegebenen Bedeutungen besitzen, sofern nichts anderes angegeben ist.

2. Verfahren nach Anspruch 1, bei welchem in den Ausgangsmaterialien R¹ für Methyl, Ethyl, Propyl, Butyl, Isobutyl, sec-Butyl, Pentyl, Hexyl, Cyclopropyl, Cyclopentyl, Cyclohexyl, Cyclopropylmethyl, Cyclopentylmethyl, Cyclohexylmethyl, 2-Cyclopentylethyl, Phenyl, Benzyl, 1-Phenylethyl oder 2-Phenylethyl steht; R² für Wasserstoff, Methyl, Ethyl, Methoxy, Ethoxy, Fluor, Chlor, Brom, Iod, Trifluormethyl, Cyano oder Nitro steht; R³ und R⁴ für fakultative Substituenten am genannten Azen-Ring stehen, die unabhängig ausgewählt sind aus Wasserstoff, Methyl, Ethyl, Cyclopropyl, Cyclopentyl, Cyclohexyl, Methoxy, Ethoxy, Fluor, Chlor, Brom, Iod, Trifluormethyl, Cyano, Hydroxy, Hydroxymethyl, Formyl und Nitro; oder, wenn A zusammen mit der Imidazol-Gruppierung, an welche es gebunden ist, eine Imidazo[4,5-b]pyridin- oder Imidazo[4,5-c]pyridin-Gruppe darstellt, R³ und R⁴, sofern sie an benachbarte Kohlenstoffatome von A gebunden sind, eine Trimethylen- oder Tetramethylen-Gruppe bilden oder zusammen mit der benachbarten Vinylen-Gruppe von A einen Benzol-Ring vervollständigen, wobei letzterer gegebenenfalls einen Fluor-, Chlor-, Brom-, Iod-, Methyl-, Ethyl-, Methoxy- oder Ethoxy-Substituenten trägt; oder, wenn A zusammen mit der Imidazol-Gruppierung, an welche es gebunden ist, etwas anderes als einen 1H-Imidazo[4,5-c]pyridin-Ring darstellt, eines der Symbole R³ oder R⁴ für eine Carboxy-, Methoxycarbonyl-, Ethoxycarbonyl- oder Propoxycarbonyl-Gruppe steht und das andere die obige Definition besitzt; X für Phenylen steht, das gegebenenfalls einen Substituenten trägt, der ausgewählt ist aus Methyl, Ethyl, Methoxy, Ethoxy, Fluor, Chlor, Brom und Iod, oder X für eine direkte Bindung zwischen den benachbarten Phenyl- und Methylen-Gruppierungen steht; R⁵ für Wasserstoff oder einen Rest steht, der sich von einem (1-6C)Alkanol, Phenol oder Glycerin ableitet; und R⁶ für Methyl, Ethyl, Propyl, Isopropyl, Butyl, Pentyl, Cyclobutyl, Cyclopentyl, Cyclohexyl oder Phenyl steht; wobei jede der genannten Phenyl-Gruppierungen unsubstituiert sein oder einen oder zwei Substituenten tragen kann, die unabhängig ausgewählt sind aus Methyl, Ethyl, Methoxy, Ethoxy, Fluor, Chlor, Brom, Cyano und Trifluormethyl.

3. Neue Verbindungen der Formel III, worin A, R¹, R², R³, R⁴ und X die in Anspruch 1 oder 2 angegebenen Bedeutungen besitzen und L für eine Schutzgruppe steht.

4. Verwendung einer Verbindung der Formel I nach Anspruch 1 bei der Herstellung eines neuen Medikaments.

## Revendications

1. Dérivés d'azaindène de formule I 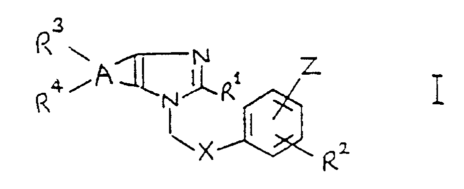 dans laquelle A, conjointement avec le groupement vinylène adjacent de la moitié imidazole, complète un noyau azène choisi parmi le noyau pyridine, pyrimidine, pyridazine ou pyrazine ; R¹ est alkyle(1-8C), cycloalkyle(3-8C), cycloalkyl(3-8C)-alkyle(1-4C), phényle ou phénylalkyle(1-4C) ; R² est hydrogène, alkyle(1-4C), alcoxy(1-4C), halogéno, trifluorométhyle, cyano ou nitro ; R³ et R⁴ sont des substituants éventuels sur ledit noyau azène, choisis indépendamment parmi hydrogène, alkyle(1-4C), cycloalkyle(3-8C), alcoxy(1-4C), halogéno, trifluorométhyle, cyano, hydroxy, hydrométhyle, formyle et nitro ; ou bien lorsque A, conjointement avec la moitié imidazole à laquelle il est lié, est un groupement imidazo[4,5-b]pyridine ou imidazo[4,5-c]pyridine, R³ et R⁴, lorsqu'ils sont sur des atomes de carbone adjacents de A, forment un groupement triméthylène ou tétraméthylène, ou, conjointement avec le groupement vinylène adjacent de A, complètent un noyau benzène, celui-ci portant éventuellement un substituant halogéno, alkyle(1-4C) ou alcoxy(1-4C) ; ou bien lorsque A, conjointement avec la moitié imidazole à laquelle il est lié, est autre qu'un noyau 1H-imidazo[4,5-c]pyridine, l'un de R³ ou R⁴ est un groupement carboxy ou alcoxy(1-6C)carbonyle et l'autre est tel que défini ci-dessus ; X est phénylène portant éventuellement un substituant choisi parmi alkyle(1-4C), alcoxy(1-4C) et halogéno, ou bien X est une liaison directe entre les moitiés phénylène et méthylène adjacentes ; et Z est 1H-tétrazol-5-yle ou un groupement de formule -CO.OR⁵ ou -CO.NH.SO₂.R⁶ dans laquelle R⁵ est hydrogène ou un résidu biodégradable non toxique d'un phénol ou d'un alcool physiologiquement acceptable, et R⁶ est alkyle(1-6C), cycloalkyle(3-8C) ou phényle ; et où l'une quelconque desdites moitiés phényle peut être non substituée ou peut porter un ou deux substituants choisis indépendamment parmi alkyle(1-4C), alcoxy(1-4C), halogéno, cyano et trifluorométhyle ; ou un sel physiologiquement acceptable de celui-ci, sauf lorsque R⁵ est autre que hydrogène et R³ ou R⁴ est autre que carboxy.

2. Composé selon la revendication 1, dans lequel R¹ est méthyle, propyle, butyle, isobutyle, sec-butyle, pentyle, hexyle, cyclopropyle, cyclopentyle, cyclohexyle, cyclopropylméthyle, cyclopentylméthyle, cyclohexylméthyle, 2-cyclopentyléthyle, phényle, benzyle, 1-phényléthyle ou 2-phényléthyle ; R² est hydrogène, méthyle, éthyle, méthoxy, éthoxy, fluoro, chloro, bromo, iodo, trifluorométhyle, cyano ou nitro ; R³ et R⁴ sont des substituants éventuels sur ledit noyau azène, choisi indépendamment parmi hydrogène, méthyle, éthyle, cyclopropyle, cyclopentyle, cyclohexyle, méthoxy, éthoxy, fluoro, chloro, bromo, iodo, trifluorométhyle, cyano, hydroxy, hydroxyméthyle, formyle et nitro ; ou bien lorsque A, conjointement avec la moitié imidazole à laquelle il est lié, est un groupement imidazo[4,5-b]pyridine ou imidazo[4,5-c]pyridine, et R³ et R⁴, lorsqu'ils sont sur des atomes de carbone adjacents de A, forment un groupement triméthylène ou tétraméthylène, ou, conjointement avec le groupement vinylène adjacent de A, complètent un noyau benzène, celui-ci portant éventuellement un substituant fluoro, chloro, bromo, iodo, méthyle, éthyle, méthoxy ou éthoxy ; ou bien lorsque A, conjointement avec la moitié imidazole à laquelle il est lié, est autre qu'un noyau 1H-imidazo[4,5-c]pyridine, l'un de R³ ou R⁴ est un groupement carboxy, méthoxycarbonyle, éthoxycarbonyle ou propoxycarbonyle et l'autre est tel que défini ci-dessus ; X est phénylène portant éventuellement un substituant choisi parmi méthyle, éthyle, méthoxy, éthoxy, fluoro, chloro, bromo et iodo, ou X est une liaison directe entre les moitiés phényle et méthylène adjacentes ; R⁵ est hydrogène ou un résidu dérivé d'un alcanol(1-6C), de phénol ou de glycérol ; et R⁶ est méthyle, éthyle, propyle, isopropyle, butyle, pentyle, cyclobutyle, cyclopentyle, cyclohexyle ou phényle ; et où l'une quelconque desdites moitiés phényle peut être non substituée ou peut porter un ou deux substituants choisis indépendamment parmi méthyle, éthyle, méthoxy, éthoxy, fluoro, chloro, bromo, cyano et trifluorométhyle.

3. Composé de formule Ia 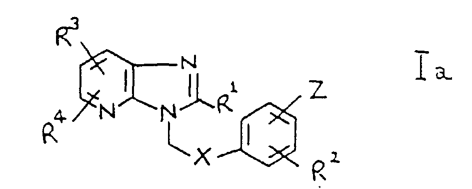 dans laquelle R¹, R², R³, R⁴, X et Z ont l'une quelconque des valeurs telles que définies à la revendication 1 ou 2, ou les sels physiologiquement acceptables de celui-ci.

4. Composé de formule I, dans laquelle A, R¹ et R², X et Z ont l'une quelconque des significations définies à la revendication 1 ; et R³ et R⁴ sont des substituants éventuels sur A, choisis indépendamment parmi hydrogène, alkyle(1-4C), alcoxy(1-4C), halogéno, trifluorométhyle, cyano et nitro ; ou un sel physiologiquement acceptable de celui-ci sauf lorsque R⁵ est autre qu'hydrogène.

5. Composé selon l'une quelconque des revendications précédentes, dans lequel R¹ est butyle.

6. Composé de formule I, choisi parmi
la 2-butyl-3-[(2'-(1H-tétrazol-5-yl)biphényl-4-yl)méthyl]-3H-imidazo-[4,5-b]pyridine ;
la 2-butyl-4-hydroxy-3-[(2'-(1H-tétrazol-5-yl)biphényl-4-yl)méthyl]-3H-imidazo-[4, 5-d]pyridazine ;
la 2-butyl-5-méthyl-3-[(2'-(1H-tétrazol-5-yl)biphényl-4-yl)méthyl]-3H-imidazo-[4, 5-b]pyridine ;
la 2-butyl-7-chloro-3-[(2'-(1H-tétrazol-5-yl)biphényl-4-yl)méthyl]-3H-imidazo-[4,5-b]pyridine ; et
la 2-butyl-4-hydroxy-3-[(2'-(1H-tétrazol-5-yl)biphényl-4-yl)méthyl]-3H-imidazo-[4,5-c]pyridine ; et
la 2-butyl-6-(hydroxyméthyl)-3-[(2'-(1H-tétrazol-5-yl)biphényl-4-yl)méthyl]imidazo-[4,5-b]pyridine ;
et les sels physiologiquement acceptables de celui-ci.

7. Sel selon l'une quelconque des revendications précédentes, choisi parmi les sels de métaux alcalins, de métaux alcalino-terreux, d'aluminium et d'ammonium et parmi les sels avec les bases organiques donnant des cations physiologiquement acceptables, et pour ceux des composés de formule I qui sont basiques, des sels avec les acides donnant des anions physiologiquement acceptables.

8. Procédé de production d'un composé de formule I ou d'un sel physiologiquement acceptable de celui-ci, tel que défini à la revendication 1, caractérisé en ce que :
a) pour ceux des composés dans lesquels Z est carboxy, un dérivé d'acide carboxylique de formule II 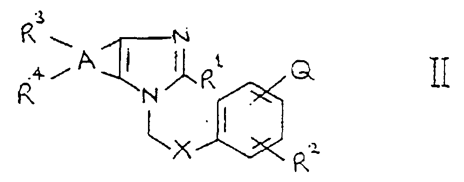 dans laquelle Q est un groupement carboxy protégé choisi parmi alcoxy(1-6C)carbonyle, phénoxycarbonyle, benzyloxycarbonyle et carbamoyle, est transformé en carboxy ;
b) pour ceux des composés de formule I dans lesquels Z est tétrazolyle, un composé de formule III 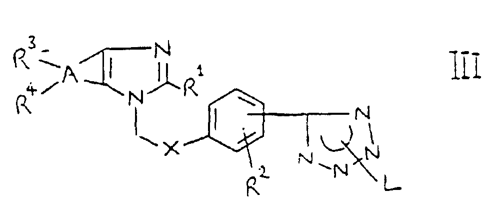 dans laquelle L est un groupement protecteur convenable fixé sur un azote de la moitié tétrazolyle, est déprotégé ;
c) pour ceux des composés de formule I dans laquelle Z est un groupement de formule -CO.OR⁵, on fait réagir un dérivé d'imidazole de formule IV 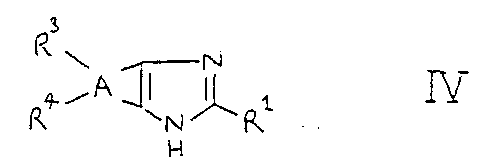 avec un composé de formule V 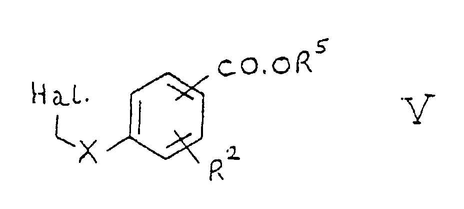 dans laquelle Hal. représente un groupement partant convenable ; ou
d) on fait réagir un dérivé diamino de formule VII 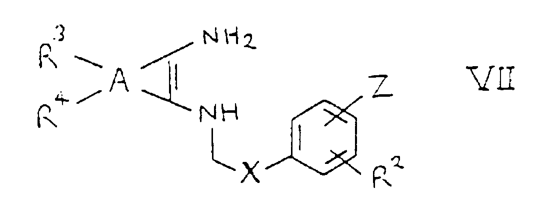 avec un acide carboxylique de formule R¹.CO₂H ou un orthoester d'alkyle(1-4C) de celui-ci ;
après quoi, lorsqu'on souhaite un composé de formule I dans laquelle Z est un groupement de formule -CO.NH.SO₂R⁶ ou un groupement de formule -CO.OR⁵ dans laquelle R⁵ est autre qu'hydrogène, on fait réagir un acide carboxylique de formule I dans laquelle Z est carboxy (ou un dérivé réactif dudit acide) avec un sulfonamide de formule NH₂.SO₂R⁶ ou un composé hydroxy de formule HO.R⁵, ou avec un sel de celui-ci ;
après quoi, lorsqu'on souhaite un sel d'un composé de formule I, on l'obtient par réaction avec la base ou l'acide approprié donnant un ion physiologiquement acceptable, ou par tout autre protocole classique de formation de sel ; et lorsqu'on souhaite une forme optiquement active d'un composé de formule I, on met en oeuvre l'un des procédés (a)-(d) en utilisant un produit de départ optiquement actif, ou la forme racémique d'un composé de formule I est dédoubleé par réaction avec une forme optiquement active d'une base ou d'un acide organique convenable, suivie de la séparation classique du mélange diastéréoisomère des sels ainsi obtenus, et la libération de la forme optiquement active souhaitée dudit composé de formule I par traitement classique par un acide ou une base; et où A, R¹, R², R³, R⁴, X et Z ont l'une quelconque des significations définies dans l'une quelconque des revendications 1-5, sauf indication contraire.

9. Composition pharmaceutique comprenant un composé de formule I ou Ia, ou un sel physiologiquement acceptable de celui-ci, selon l'une quelconque des revendications 1 à 7, conjointement avec un diluant ou un support pharmaceutiquement acceptable.

10. Composé de formule III, dans laquelle A, R¹, R², R³, R⁴ et X ont l'une quelconque des significations définies dans l'une quelconque des revendications 1-5, et L est un groupement protecteur.

## Revendications (Revendications pour l'(les) Etat(s) contractant(s) suivant(s): ES)

1. Procédé de production d'un dérivé d'azaindène de formule I 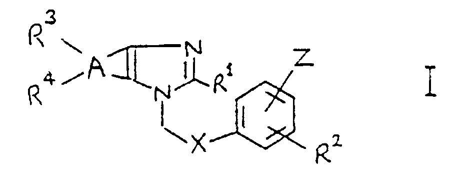 dans laquelle A, conjointement avec le groupement vinylène adjacent de la moitié imidazole, complète un noyau azène choisi parmi le noyau pyridine, pyrimidine, pyridazine ou pyrazine ; R¹ est alkyle(1-8C), cycloalkyle(3-8C), cycloalkyl(3-8C)-alkyle(1-4C), phényle ou phénylalkyle(1-4C) ; R² est hydrogène, alkyle(1-4C), alcoxy(1-4C), halogéno, trifluorométhyle, cyano ou nitro ; R³ et R⁴ sont des substituants éventuels sur ledit noyau azène, choisis indépendamment parmi hydrogène, alkyle(1-4C), cycloalkyle(3-8C), alcoxy(1-4C), halogéno, trifluorométhyle, cyano, hydroxy, hydrométhyle, formyle et nitro ; ou bien lorsque A, conjointement avec la moitié imidazole à laquelle il est lié, est un groupement imidazo[4,5-b]pyridine ou imidazo[4,5-c]pyridine, R³ et R⁴, lorsqu'ils sont sur des atomes de carbone adjacents de A, forment un groupement triméthylène ou tétraméthylène, ou, conjointement avec le groupement vinylène adjacent de A, complètent un noyau benzène, celui-ci portant éventuellement un substituant halogéno, alkyle(1-4C) ou alcoxy(1-4C) ; ou bien lorsque A, conjointement avec la moitié imidazole à laquelle il est lié, est autre qu'un noyau 1H-imidazo[4,5-c]pyridine, l'un de R³ ou R⁴ est un groupement carboxy ou alcoxy(1-6C)carbonyle et l'autre est tel que défini ci-dessus ; X est phénylène portant éventuellement un substituant choisi parmi alkyle(1-4C), alcoxy(1-4C) et halogéno, ou bien X est une liaison directe entre les moitiés phénylène et méthylène adjacentes ; et Z est 1H-tétrazol-5-yle ou un groupement de formule -CO.OR⁵ ou -CO.NH.SO₂.R⁶ dans laquelle R⁵ est hydrogène ou un résidu biodégradable non toxique d'un phénol ou d'un alcool physiologiquement acceptable, et R⁶ est alkyle(1-6C), cycloalkyle(3-8C) ou phényle ; et où l'une quelconque desdites moitiés phényle peut être non substituée ou peut porter un ou deux substituants choisis indépendamment parmi alkyle(1-4C), alcoxy(1-4C), halogéno, cyano et trifluorométhyle ; ou un sel physiologiquement acceptable de celui-ci, sauf lorsque R⁵ est autre que hydrogène et R³ ou R⁴ est autre que carboxy ; caractérisé en ce que :
a) pour ceux des composés dans lesquels Z est carboxy, un dérivé d'acide carboxylique de formule II 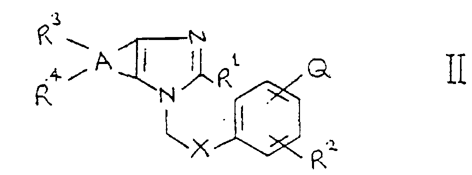 dans laquelle Q est un groupement carboxy protégé choisi parmi alcoxy(1-6C)carbonyle, phénoxycarbonyle, benzyloxycarbonyle et carbamoyle, est transformé en carboxy ;
b) pour ceux des composés de formule I dans lesquels Z est tétrazolyle, un composé de formule III 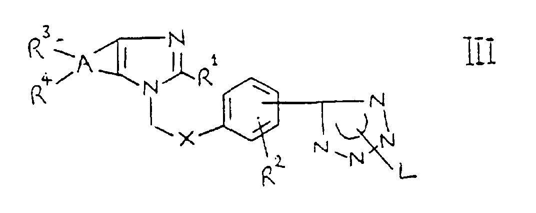 dans laquelle L est un groupement protecteur convenable fixé sur un azote de la moitié tétrazolyle, est déprotégé ;
c) pour ceux des composés de formule I dans laquelle Z est un groupement de formule -CO.OR⁵, on fait réagir un dérivé d'imidazole de formule IV 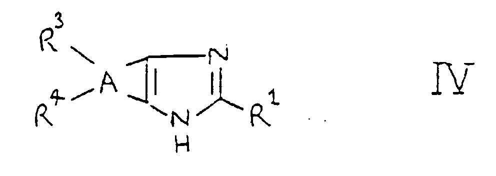 avec un composé de formule V 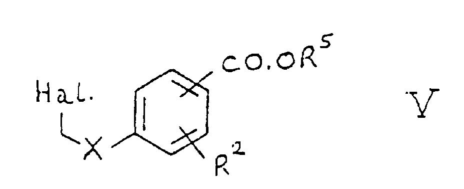 dans laquelle Hal. représente un groupement partant convenable ; ou
d) on fait réagir un dérivé diamino de formule VII 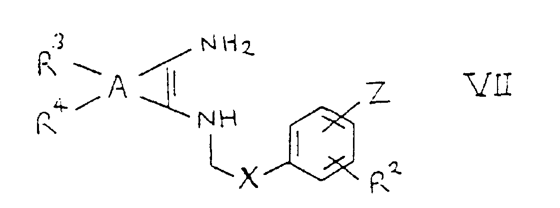 avec un acide carboxylique de formule R¹.CO₂H ou un orthoester d'alkyle(1-4C) de celui-ci ;
après quoi, lorsqu'on souhaite un composé de formule I dans laquelle Z est un groupement de formule -CO.NH.SO₂R⁶ ou un groupement de formule -CO.OR⁵ dans laquelle R⁵ est autre qu'hydrogène, on fait réagir un acide carboxylique de formule I dans laquelle Z est carboxy (ou un dérivé réactif dudit acide) avec un sulfonamide de formule NH₂.SO₂R⁶ ou un composé hydroxy de formule HO.R⁵, ou avec un sel de celui-ci ;
après quoi, lorsqu'on souhaite un sel d'un composé de formule I, on l'obtient par réaction avec la base ou l'acide approprié donnant un ion physiologiquement acceptable, ou par tout autre protocole classique de formation de sel ; et lorsqu'on souhaite une forme optiquement active d'un composé de formule I, on met en oeuvre l'un des procédés (a)-(d) en utilisant un produit de départ optiquement actif, ou la forme racémique d'un composé de formule I est dédoublée par réaction avec une forme optiquement active d'une base ou d'un acide organique convenable, suivie de la séparation classique du mélange diastéréoisomère des sels ainsi obtenus, et la libération de la forme optiquement active souhaitée dudit composé de formule I par traitement classique par un acide ou une base; et où A, R¹, R², R³, R⁴, X et Z ont l'une quelconque des significations définies ci-dessus, sauf indication contraire.

2. Procédé selon la revendication 1, caractérisé en ce que dans les produits de départ, R¹ est méthyle, propyle, butyle, isobutyle, sec-butyle, pentyle, hexyle, cyclopropyle, cyclopentyle, cyclohexyle, cyclopropylméthyle, cyclopentylméthyle, cyclohexylméthyle, 2-cyclopentyléthyle, phényle, benzyle, 1-phényléthyle ou 2-phényléthyle ; R² est hydrogène, méthyle, éthyle, méthoxy, éthoxy, fluoro, chloro, bromo, iodo, trifluorométhyle, cyano ou nitro ; R³ et R⁴ sont des substituants éventuels sur ledit noyau azène, choisis indépendamment parmi hydrogène, méthyle, éthyle, cyclopropyle, cyclopentyle, cyclohexyle, méthoxy, éthoxy, fluoro, chloro, bromo, iodo, trifluorométhyle, cyano,, hydroxy, hydroxyméthyle, formyle et nitro ; ou bien lorsque A, conjointement avec la moitié imidazole à laquelle il est lié, est un groupement imidazo[4,5-b]pyridine ou imidazo[4,5-c]pyridine, et R³ et R⁴, lorsqu'ils sont sur des atomes de carbone adjacents de A, forment un groupement triméthylène ou tétraméthylène, ou, conjointement avec le groupement vinylène adjacent de A, complètent un noyau benzène, celui-ci portant éventuellement un substituant fluoro, chloro, bromo, iodo, méthyle, éthyle, méthoxy ou éthoxy ; ou bien lorsque A, conjointement avec la moitié imidazole à laquelle il est lié, est autre qu'un noyau 1H-imidazo[4,5-c]pyridine, l'un de R³ ou R⁴ est un groupement carboxy, méthoxycarbonyle, éthoxycarbonyle ou propoxycarbonyle et l'autre est tel que défini ci-dessus ; X est phénylène portant éventuellement un substituant choisi parmi méthyle, éthyle, méthoxy, éthoxy, fluoro, chloro, bromo et iodo, ou X est une liaison directe entre les moitiés phényle et méthylène adjacentes ; R⁵ est hydrogène ou un résidu dérivé d'un alcanol(1-6C), de phénol ou de glycérol ; et R⁶ est méthyle, éthyle, propyle, isopropyle, butyle, pentyle, cyclobutyle, cyclopentyle, cyclohexyle ou phényle ; et où l'une quelconque desdites moitiés phényle peut être non substituée ou peut porter un ou deux substituants choisis indépendamment parmi méthyle, éthyle, méthoxy, éthoxy, fluoro, chloro, bromo, cyano et trifluorométhyle.

3. Utilisation du composé de formule I telle que définie à la revendication 1 dans la fabrication d'un nouveau médicament.

## Revendications (Revendications pour l'(les) Etat(s) contractant(s) suivant(s): GR)

1. Procédé de production d'un dérivé d'azaindène de formule I 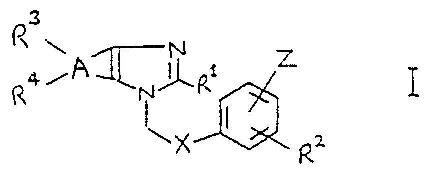 dans laquelle A, conjointement avec le groupement vinylène adjacent de la moitié imidazole, complète un noyau azène choisi parmi le noyau pyridine, pyrimidine, pyridazine ou pyrazine ; R¹ est alkyle(1-8C), cycloalkyle(3-8C), cycloalkyl(3-8C)-alkyle(1-4C), phényle ou phénylalkyle(1-4C) ; R² est hydrogène, alkyle(1-4C), alcoxy(1-4C), halogéno, trifluorométhyle, cyano ou nitro ; R³ et R⁴ sont des substituants éventuels sur ledit noyau azène, choisis indépendamment parmi hydrogène, alkyle(1-4C), cycloalkyle(3-8C), alcoxy(1-4C), halogéno, trifluorométhyle, cyano, hydroxy, hydrométhyle, formyle et nitro ; ou bien lorsque A, conjointement avec la moitié imidazole à laquelle il est lié, est un groupement imidazo[4,5-b]pyridine ou imidazo[4,5-c]pyridine, R³ et R⁴, lorsqu'ils sont sur des atomes de carbone adjacents de A, forment un groupement triméthylène ou tétraméthylène, ou, conjointement avec le groupement vinylène adjacent de A, complètent un noyau benzène, celui-ci portant éventuellement un substituant halogéno, alkyle(1-4C) ou alcoxy(1-4C) ; ou bien lorsque A, conjointement avec la moitié imidazole à laquelle il est lié, est autre qu'un noyau 1H-imidazo[4,5-c]pyridine, l'un de R³ ou R⁴ est un groupement carboxy ou alcoxy(1-6C)carbonyle et l'autre est tel que défini ci-dessus ; X est phénylène portant éventuellement un substituant choisi parmi alkyle(1-4C), alcoxy(1-4C) et halogéno, ou bien X est une liaison directe entre les moitiés phénylène et méthylène adjacentes ; et Z est 1H-tétrazol-5-yl ou un groupement de formule -CO-OR.⁵ ou -CO.NH.SO₂.R⁶ dans laquelle R⁵ est hydrogène ou un résidu d'un phénol ou d'un alcool physiologiquement acceptable, et R⁶ est alkyle(1-6C), cycloalkyle(3-8C) ou phényle ; et où l'une quelconque desdites moitiés phényle peut être non substituée ou peut porter un ou deux substituants choisis indépendamment parmi alkyle(1-4C), alcoxy(1-4C), halogéno, cyano et trifluorométhyle ; ou un sel physiologiquement acceptable de celui-ci, sauf lorsque R⁵ est autre que hydrogène et R³ ou R⁴ est autre que carboxy ; caractérisé en ce que :
a) pour ceux des composés dans lesquels Z est carboxy, un dérivé d'acide carboxylique de formule II 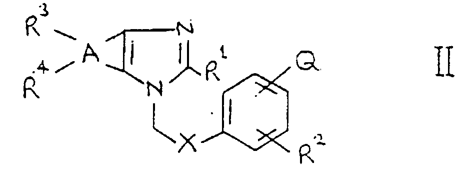 dans laquelle Q est un groupement carboxy protégé choisi parmi alcoxy (1-6C)carbonyle, phénoxycarbonyle, benzyloxycarbonyle et carbamoyle, est transformé en carboxy ;
b) pour ceux des composés de formule I dans lesquels Z est tétrazolyle, un composé de formule III 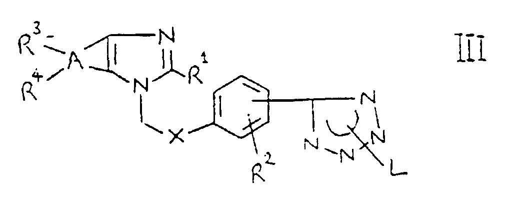 dans laquelle L est un groupement protecteur convenable fixé sur un azote de la moitié tétrazolyle, est déprotégé ;
c) pour ceux des composés de formule I dans laquelle Z est un groupement de formule -CO.OR⁵, on fait réagir un dérivé d'imidazole de formule IV 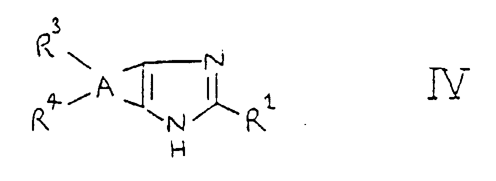 avec un composé de formule V 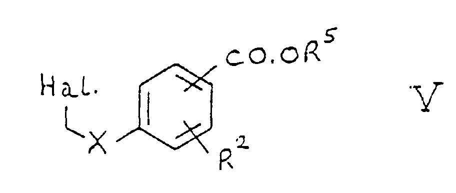 dans laquelle Hal. représente un groupement partant convenable ; ou
d) on fait réagir un dérivé diamino de formule VII 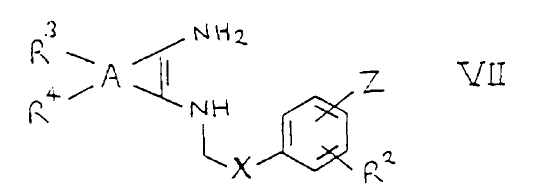 avec un acide carboxylique de formule R¹.CO₂H ou un orthoester d'alkyle(1-4C) de celui-ci ;
après quoi, lorsqu'on souhaite un composé de formule I dans laquelle Z est un groupement de formule -CO.NH.SO₂R⁶ ou un groupement de formule -CO.OR⁵ dans laquelle R⁵ est autre qu'hydrogène, on fait réagir un acide carboxylique de formule I dans laquelle Z est carboxy (ou un dérivé réactif dudit acide) avec un sulfonamide de formule NH₂.SO₂R⁶ ou un composé hydroxy de formule HO.R⁵, ou avec un sel de celui-ci ;
après quoi, lorsqu'on souhaite un sel d'un composé de formule I, on l'obtient par réaction avec la base ou l'acide approprié donnant un ion physiologiquement acceptable, ou par tout autre protocole classique de formation de sel ; et lorsqu'on souhaite une forme optiquement active d'un composé de formule I, on met en oeuvre l'un des procédés (a)-(d) en utilisant un produit de départ optiquement actif, ou la forme racémique d'un composé de formule I est dédoublée par réaction avec une forme optiquement active d'une base ou d'un acide organique convenable, suivie de la séparation classique du mélange diastéréoisomère des sels ainsi obtenus, et la libération de la forme optiquement active souhaitée dudit composé de formule I par traitement classique par un acide ou une base; et où A, R¹, R², R³, R⁴, X et Z ont l'une quelconque des significations définies ci-dessus, sauf indication contraire.

2. Procédé selon la revendication 1, caractérisé en ce que dans les produits de départ, R¹ est méthyle, propyle, butyle, isobutyle, sec-butyle, pentyle, hexyle, cyclopropyle, cyclopentyle, cyclohexyle, cyclopropylméthyle, cyclopentylméthyle, cyclohexylméthyle, 2-cyclopentyléthyle, phényle, benzyle, 1-phényléthyle ou 2-phényléthyle ; R² est hydrogène, méthyle, éthyle, méthoxy, éthoxy, fluoro, chloro, bromo, iodo, trifluorométhyle, cyano ou nitro ; R³ et R⁴ sont des substituants éventuels sur ledit noyau azène, choisis indépendamment parmi hydrogène, méthyle, éthyle, cyclopropyle, cyclopentyle, cyclohexyle, méthoxy, éthoxy, fluoro, chloro, bromo, iodo, trifluorométhyle, cyano,, hydroxy, hydroxyméthyle, formyle et nitro ; ou bien lorsque A, conjointement avec la moitié imidazole à laquelle il est lié, est un groupement imidazo[4,5-b]pyridine ou imidazo[4,5-c]pyridine, et R³ et R⁴, lorsqu'ils sont sur des atomes de carbone adjacents de A, forment un groupement triméthylène ou tétraméthylène, ou, conjointement avec le groupement vinylène adjacent de A, complètent un noyau benzène, celui-ci portant éventuellement un substituant fluoro, chloro, bromo, iodo, méthyle, éthyle, méthoxy ou éthoxy ; ou bien lorsque A, conjointement avec la moitié imidazole à laquelle il est lié, est autre qu'un noyau 1H-imidazo[4,5-c]pyridine, l'un de R³ ou R⁴ est un groupement carboxy, méthoxycarbonyle, éthoxycarbonyle ou propoxycarbonyle et l'autre est tel que défini ci-dessus ; X est phénylène portant éventuellement un substituant choisi parmi méthyle, éthyle, méthoxy, éthoxy, fluoro, chloro, bromo et iodo, ou X est une liaison directe entre les moitiés phényle et méthylène adjacentes ; R⁵ est hydrogène ou un résidu dérivé d'un alcanol(1-6C), de phénol ou de glycérol ; et R⁶ est méthyle, éthyle, propyle, isopropyle, butyle, pentyle, cyclobutyle, cyclopentyle, cyclohexyle ou phényle ; et où l'une quelconque desdites moitiés phényle peut être non substituée ou peut porter un ou deux substituants choisis indépendamment parmi méthyle, éthyle, méthoxy, éthoxy, fluoro, chloro, bromo, cyano et trifluorométhyle.

3. Nouveau composé de formule III, dans laquelle A, R¹, R², R³, R⁴ et X ont l'une quelconque des significations définies à la revendication 1 ou 2, et L est un groupement protecteur.

4. Utilisation du composé de formule I telle que définie à la revendication 1 1 dans la fabrication d'un nouveau médicament.
